(19) Europäisches Patentamt
European Patent Office
Office européen des brevets

(11) EP 4 483 866 A1

(12) EUROPEAN PATENT APPLICATION

(43) Date of publication:
**01.01.2025 Bulletin 2025/01**

(21) Application number: **23182027.5**

(22) Date of filing: **28.06.2023**

(51) International Patent Classification (IPC):
***A61K 31/045*** (2006.01) ***A61K 31/05*** (2006.01)
***A61K 31/12*** (2006.01) ***A61K 31/352*** (2006.01)
***A61K 31/404*** (2006.01) ***A61K 31/4412*** (2006.01)
***A61K 45/06*** (2006.01) ***A61P 35/00*** (2006.01)
***A61K 31/09*** (2006.01) ***A61K 31/167*** (2006.01)
***A61K 31/194*** (2006.01) ***A61K 31/215*** (2006.01)
***A61K 31/216*** (2006.01) ***A61K 31/235*** (2006.01)
***A61K 31/351*** (2006.01) ***A61K 31/4025*** (2006.01)
***A61K 31/4045*** (2006.01) ***A61K 31/405*** (2006.01)
***A61K 31/415*** (2006.01) ***A61K 31/4152*** (2006.01)
***A61K 31/4184*** (2006.01)

(52) Cooperative Patent Classification (CPC):
**A61K 31/045; A61K 31/05; A61K 31/09; A61K 31/12; A61K 31/167; A61K 31/194; A61K 31/215; A61K 31/216; A61K 31/235; A61K 31/351; A61K 31/352; A61K 31/4025; A61K 31/404; A61K 31/4045; A61K 31/405;**
(Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Makrolife Biotech GmbH**
**76646 Bruchsal (DE)**

(72) Inventor: **AHMAD, Fähzan**
**76698 Ubstadt Weiher (DE)**

(74) Representative: **Patentanwälte Dr. Keller, Schwertfeger Partnerschaft mbB**
**Westring 17**
**76829 Landau (DE)**

# (54) PHARMACEUTICAL COMPOSITIONS FOR THE TREATMENT OR PREVENTION OF CANCER

(57) The invention relates to pharmaceutical compositions comprising at least one active compound having the property of polarization or differentiation of monocytes, and repolarization of macrophages into a phenotype with anti-tumor properties, the active compound being selected from the group consisting of apigenin derivative, indol-3-carbinol derivative, lupeol derivative, piperlongumin derivative, quercetin derivative, curcumin derivative and/or resveratrol derivative, or a mixture thereof, and a suitable pharmaceutical carrier. The pharmaceutical compositions are suitable for the treatment or prevention of different types of cancer. The invention furthermore relates to an in-vitro method for polarization or differentiation of monocytes or peripheral blood mononuclear cells (PBMCs) into macrophages with a M1-like phenotype with anti-tumor properties. The invention further relates to a method of preparation of repolarized macrophages with M1-like phenotype with anti-tumor properties.

EP 4 483 866 A1

(52) Cooperative Patent Classification (CPC): (Cont.)
**A61K 31/415; A61K 31/4152; A61K 31/4184;
A61K 31/4412; A61K 31/4433; A61K 31/453;
A61K 31/496; A61K 31/505; A61K 31/513;
A61K 45/06; A61P 35/00**

## Description

### Field of the Invention

The invention relates to pharmaceutical compositions comprising at least one active compound having the property of polarization or differentiation of monocytes, and repolarization of macrophages into a phenotype with anti-tumor properties. The active compound is selected from the group consisting of apigenin derivative, indol-3-carbinol derivative, lupeol derivative, piperlongumin derivative, quercetin derivative, curcumin derivative and/or resveratrol derivative, or a mixture thereof, and a suitable pharmaceutical carrier. The pharmaceutical compositions of the present invention are suitable for the treatment or prevention of different types of cancer. The invention furthermore relates to an in-vitro method for polarization or differentiation of monocytes or peripheral blood mononuclear cells (PBMCs) into macrophages with a M1-like phenotype with anti-tumor properties. The invention further relates to a method of preparation of repolarized macrophages with M1-like phenotype with anti-tumor properties.

### Background of the Invention

Macrophages are specialized cells involved in the detection, phagocytosis and destruction of bacteria, viruses and other harmful organisms. The key functions of macrophages in innate and adaptive immunity are the phagocytosis and subsequent degradation of senescent or apoptotic cells, microbes and neoplastic cells, the secretion of cytokines, chemokines and other soluble mediators, and the presentation of foreign antigens (peptides) on their surface to T lymphocytes. In particular, they have the ability to present antigens to T cells and initiate inflammation by releasing cytokines in order to activate other cells. Macrophages originate from blood monocytes that leave the circulation to differentiate in different tissues. There is a substantial heterogeneity among each macrophage population, which most probably reflects the required level of specialization within the environment of any given tissue. This heterogeneity is reflected in their morphology, the type of pathogens they can recognize, as well as the levels of inflammatory cytokines they produce. The heterogeneous nature of these cells may not solely be the result of their differentiation process, but it is likely to be inherited from their monocyte precursors. Monocytes and macrophages are members of the mononuclear phagocyte system, a component of innate immunity. Monocytes are bone marrow derived leukocytes that circulate in the blood and spleen. Macrophages play an important role in the innate immune system as they are responsible for destroying invading viruses, bacteria, and other pathogens. The most critical tasks of macrophages are the detection and elimination of pathogens which is mediated by phagocytosis, as well as antigen presentation, whereby they can activate the adaptive immune response (Martinez and Gordon, 2014). Three main macrophage subpopulations coexist in human tissues: native macrophages also called M0, pro-inflammatory macrophages referred as M1 macrophages, and anti-inflammatory macrophages also known as M2 macrophages. The activation by different stimuli generates macrophages of different polarization with a wide spectrum of diverse purposes and functions.

The process of reprogramming a macrophage toward a specific function is called macrophage repolarization. Macrophage polarization is a process, whereby macrophages phenotypically mount a specific phenotype and a functional response to the micro-environmental stimuli and signals that encounter in each specific tissue (Sica et al. 2012). There are two subgroups of macrophage polarization, known as the M1 and M2 polarization state. M1 macrophages are also called classically activated macrophages and feature pro-inflammatory effects, whereas M2 polarization leads to the alternatively activated anti-inflammatory state. The M1 polarization can be caused by lipopolysaccharides (LPS), resulting in the production of pro-inflammatory cytokines (Sica et al. 2012). The activation to M1 polarization state is triggered by cytokines such as *TNFa, IL1, IL6, IL12, IL23* and the chemokines *IL9, IL10* and *IFN*$\gamma$ (Cassol et al. 2009). M1 macrophages promote and activate the Th1 response and thereby induce the production of toxic molecules, such as ROS (Loegl et al. 2019).

M1 macrophages operate against injuries and bacterial infections, and also exert antitumor functions, including direct-mediated cytotoxicity and antibody-dependent cell-mediated cytotoxicity (ADCC), which results in killing of tumor cells. M2 macrophages induce anti-inflammatory cytokines to suppress tissue-damaging defense mechanisms and initiate tissue repair, remodeling, wound healing and neovascularization. For correct homeostasis, both polarization states are mandatory (Sica and Mantovani 2012). M2 subtype macrophages can promote tumor cell appearance and metastasis, inhibit the T-cell-mediated antitumor immune response, and promote tumor angiogenesis and ultimately tumor progression. Such an immunosuppressive tumor microenvironment (TME) with best conditions for tumor growth is created by M2 macrophages producing cytokines, chemokines, and growth factors and triggering the release of inhibitory immune checkpoint proteins in T cells. Both M1 and M2 macrophages have a high degree of plasticity and can therefore convert to the other phenotype upon changes in the TME or therapeutic intervention. Within a TME, monocytes differentiate to a large extent into tumor-associated macrophages (TAMs). TAMs are key cells of the cancer immunological microenvironment with multiple tumor-supportive effects on growth, progression, and metastasis (Solinas et al. 2009). TAMs regulate tumor-promoting factors including increased oxygen delivery via the HIF1 alpha gene, upregulated matrix metalloproteases, and EGFR genes for increased cell differentiation in the context of targeted tumor growth. In addition to intrinsic changes in

tumor cells themselves, implicated interactions between cancer cells and components of their altered TME are thus among the main drivers of tumor metastasis.

TAMs arise in the tumor environment and often cause tumor-promoting conditions (Lewis & Pollard 2006). TAM-like macrophages secrete cytokines (tumor necrosis factor $\alpha$, *IL8, IL10*), growth factors (*VEGF, PDGF*) and other mediators that promote the formation of new blood vessels. Matrix metalloproteases are also increasingly needed for tumor formation (Lewis & Pollard, 2006). The TAM-like macrophages phenotype is mostly activated by the tumor environment and is similar to M2 macrophages based on their elevated expression of *CCL1, IL12,* and *IL10* (Cook and Hagemann, 2013). In addition to angiogenesis, TAM-like macrophages promote tumor invasion and metastasis progression (Solinas et al. 2009) by the release of chemokines, MMPs, and other cytokines (*IL1, IL6, TNF*) (Biswas et al. 2013, Sica et al. 2010). In healthy tissues, macrophages present tumor-associated antigens, triggering an immune response against tumor cells, whereas TAM-like macrophages ability to present antigens is decreased. M1 cells inhibit tumor progression by secreting pro-inflammatory tumor-suppressing molecules. The microenvironment of cancer cells converts M1 polarized macrophages into M2, e.g., by *IL10* or M-CSF release. M2-like anti-inflammatory tumor-associated macrophages (TAM) promote cancer cell proliferation (Wynn et al. 2013).

WO2017/019848A1 describes genetically engineered monocytes/macrophages expressing chimeric antigen receptors of the CAR gene to generate an anti-tumor immune response. However, this is a complex genetic process, and TAMs cannot be directly influenced using these methods.

US11103507B2 describes pharmaceutical compositions, comprising orally administering to the patient a therapeutically effective amount of the compound 2-(isopropylamino)-3-methyl-5-(6-methyl-5-((2-(1-methyl-1H-pyrazol-4-yl)pyridin-4-yl)oxy)pyridin-2-yl)pyrimidin-4(3H)-one or a pharmaceutically acceptable salt thereof.

Most known therapeutic approaches that relate to specifically killing of tumor cells by re-programmed macrophages are complex and not suitable for clinical use. Thus, there is a great need in the art for highly effective pharmaceutical formulations for the repolarization of macrophages, which can be individually adapted to the conditions of the respective patient and at the same time produced at low cost.

In order to generate macrophages with anti-tumor properties it is therefore necessary to repolarize them in M1-macrophages with anti-tumor properties.

**Description of the Invention**

Against this background, it is the object of the present invention to provide novel, potent anti-tumor compounds which have the ability to specifically target tumor cells.

This object is solved by the subject-matter of claim 1. Preferred embodiments are claimed in the sub-claims.

The present invention is based on the surprising discovery that derivatives of the compounds apigenin, indol-3-carbinol, lupeol, piperlongumin, quercetin, curcumin and/or resveratrol, or a mixture thereof have anti-tumoral properties, i.e., they have the ability to reduce the number or growth of tumor cells. The derivatives of the compounds apigenin, indol-3-carbinol, lupeol, piperlongumin, quercetin, curcumin and/or resveratrol as described in the present invention have the property to be highly efficient in repolarization of macrophages, and polarization or differentiation of monocytes into a strong tumor suppressive M1 state. More specifically, all active compounds of the invention are able to bind to specific targets on macrophages and thus manipulate these cells in such a way that they are programmed towards an anti-tumor M1 type.

A "monocyte" as used in the context of the present invention is a mononuclear phagocyte of the peripheral blood. Monocytes can vary considerably, ranging in size from 10 to 30 pm in diameter.

A "macrophage" as used in the context of the present invention is a cell exhibiting properties of phagocytosis.

The derivatives of the present invention are derived from the natural compounds apigenin, indol-3-carbinol, lupeol, piperlongumin, quercetin, curcumin or resveratrol and have in common that they have the property of repolarization of macrophages, and polarization or differentiation of monocytes into a strong tumor suppressive M1 state. They furthermore have in common that they have the ability to lead to a gene expression profile with anti-tumor properties.

In a first aspect of the invention, the derivatives of the invention have anti-tumoral properties and can be used without macrophages or monocytes for cancer treatment.

In another aspect of the invention, anti-tumor treatment is enhanced by polarization or differentiation of monocytes and repolarization of macrophages.

The term "tumor" as used in the context of the present invention refers to a volume increase of a tissue. In the narrower sense, a tumor describes the body's cells that multiply independently, continuously and uncontrolled.

A "reduction of tumor growth" as described herein usually refers to a reduction of the size of tumor tissue, a reduction of the number of tumor cells or a reduction of the cell division rate of a tumor cell.

"Anti-tumor properties" or "anti-tumor activity" as used in the context of the present invention refers to an inhibition of tumor cell growth or to the property of being able to be used as an anticancer agent.

In a first aspect, the present invention relates to pharmaceutical compositions comprising at least one active

compound having the capability of polarization or differentiation of monocytes and repolarization of macrophages. According to the invention the active compound is being selected from the group consisting of apigenin derivative, indol-3-carbinol derivative, lupeol derivative, piperlongumin derivative, quercetin derivative, curcumin derivative and/or resveratrol derivative, or a mixture thereof, and a suitable pharmaceutical carrier.

In some embodiments, the active compound is an apigenin derivative, optionally combined with one or more other compounds described herein.

In some embodiments, the active compound is an indol-3-carbinol derivative, optionally combined with one or more other compounds described herein.

In some embodiments, the active compound is a lupeol derivative optionally combined with one or more other compounds described herein.

In some embodiments, the active compound is a quercetin derivate, optionally combined with one or more other compounds described herein.

In some embodiments, the active compound is a curcumin derivative, optionally combined with one or more other compounds described herein.

In some embodiments, the active compound is a piperlongumin derivative, optionally combined with one or more other compounds described herein.

In some embodiments, the active compound is a resveratrol derivative, optionally combined with one or more other compounds described herein.

The term "derivative" as used in the context of the present invention relates to chemical compounds which basic chemical structure is based on the underlying natural compound described herein but includes one or more chemical modifications or chemical moieties in said structure.

The active compounds described by the present invention, namely apigenin derivative, indol-3-carbinol derivative, lupeol derivative, piperlongumin derivative, quercetin derivative, curcumin derivative and/or resveratrol derivative and combinations thereof are preferably introduced directly into a subject to be treated by any mode of administration, preferably by injection or infusion. In other embodiments, the active compounds are administered orally, e.g., in the form of tablets, capsules or liquid as drops or solutions or mouthwashes and dentifrices.

In some embodiments, the active compounds of the invention are brought into contact with monocytes and macrophages that were sampled from blood of a human or animal subject. Such an *ex-vivo* approach comprises the provision of a blood sample from a subject to be treated, an incubation with one or more active compounds according to the present invention in order to repolarize macrophages and polarize monocytes into a M1-like phenotype, and the subsequent administration of the re-programmed immune cells into the subject to be treated. The invention also comprises a combination of an *ex-vivo* approach, where macrophages and monocytes are re-programmed and subsequently administered to the subject to be treated, followed by one or more subsequent administrations of one or more derivatives of the present invention.

The term "subject" as used herein includes, but is not limited to, an organism or animal, including, but not limited to a mammal, such as a human, non-human primate (e.g., monkey), mouse, pig, cow, goat, rabbit, rat, guinea pig, hamster, horse, monkey, sheep, or other non-human mammals.

As shown herein, the inventive active compounds have properties that are superior to the properties of the underlying natural compounds or other known derivatives described in the art. The derivatives of the invention are rather small molecules which travel, when introduced directly into the body of a patient to be treated, through the bloodstream and encounter the target immune cells, i.e., monocytes and macrophages. The circulating active compounds recognize and bind to pro-tumor macrophages, i.e., M0-like macrophages and monocytes, and initiate repolarization of macrophages and polarization or differentiation of monocytes the M1 phenotype so that they exhibit a gene expression profile with anti-tumor properties. That said, the active compounds of the present invention have in common that they are able to repolarize macrophages and polarize monocytes so that they change their phenotype and initiate immune responses with the tumor-killing properties. The inventive compounds are thus suitable for tumor therapy approaches in order to specifically kill tumor cells by immune cells with an altered phenotype. For example, the repolarized macrophages recognize tumor cells and induce cytotoxic secretion, especially by releasing TNF-$\alpha$ and IFN-$\gamma$, triggering a pro-inflammatory tumor microenvironment. The anti-tumor response is long-lasting with a strong immune response which causes tumor suppression and elimination for a long-term anti-tumor immunity.

The anti-tumor response initiated by the active compounds of the present invention includes, but is not limited to an activation of natural killer cells, an activation of T-cells by CD47-SIRPalpha axis blockage, a reactivation of CD4+ T-cells, which mediate anti-tumor immunity by providing help for CD8+ CTL and antibody responses, as well as via secretion of effector cytokines such as interferon-$\gamma$ (IFN$\gamma$) and tumor necrosis factor-$\alpha$ (TNF$\alpha$), and, under specific contexts, via direct cytotoxicity against tumor cells, activation of cytotoxic CD8+ T cells which are major killers of cancer cells, an inhibition of tumor growth by inhibiting the CCR2-CCL2 axis, an inhibition of other tumor-promoting macrophages (TAM) by blocking CSF-1 and CSF-1R, a regulation of angiogenesis for a stable metabolism through HIF1$\alpha$ control, an inhibition of the checkpoint inhibitor PD-1 (R) for increased T-cell activity, a dissolution of the LILRB1-MHC I complex for direct

cytotoxic elimination, a release of cytotoxic signals, especially TNFα and IFNγ for the killing of tumor cells and a phagocytosis of tumor cells by inhibition of the SIRPalpha-CD47 axis.

In some embodiments, the active compound for polarization or differentiation of monocytes and repolarization of macrophages into a phenotype with anti-tumor properties is a derivative of apigenin.

Apigenin is known for its anti-inflammatory and antioxidant effect. Amino-alkylation at the 7-O position results in significant biological activities. Synthesis of an apigenin derivative as provided by the present invention comprises a linkage of the apigenin molecule to another ring system with a nitrogen molecule separated by a 2-carbon spacer at the C-7 position to enhance lipophilicity and the nitrogen group as an electron-withdrawing element to enhance activity. It was found that heterocyclic nitrogen moieties at the C-7 position of apigenin have much higher activity than compounds with aliphatic chains. Furthermore, increasing the lipophilicity of this compound increases its penetration through transmembrane diffusion.

In some embodiments, a 3-carbon spacer is used instead of a 2-carbon spacer to trigger a stronger anti-tumor activity due to the increased lipophilicity and the associated permeability of the cell membrane. The attachment of the alkyl group to this hydroxyl group has the highest affinity compared to the other potential groups.

A preferred apigenin derivative of the invention comprises the following general structure:

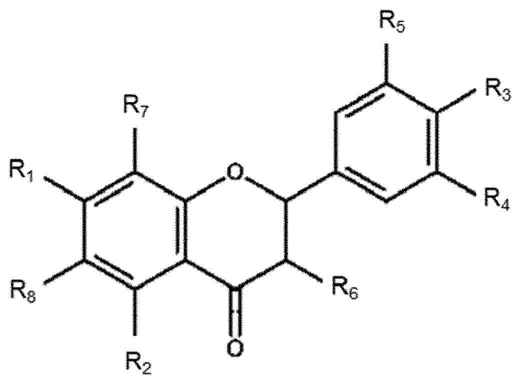

wherein:

$R_1$ = - H, - OH, - O - Me,

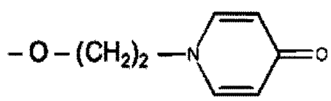

$R_2$ = - H, - OH, - O - Me, - O - CO - $CH_2$ - OH,
$R_3$ = - H, - OH, -O - Me,

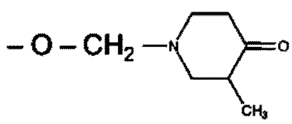 , 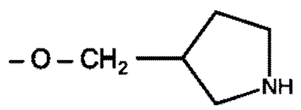 ,

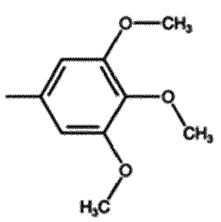 , 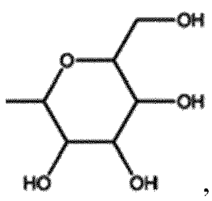 , 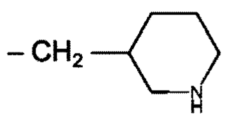

$R_4$= -H, -O-Me
$R_5$= -H, -O-Me
$R_6$= -H, -OH

$R_7$= -H
$R_8$= - H.

In preferred embodiments of the invention, the apigenin derivative is selected from the group consisting of:

1.

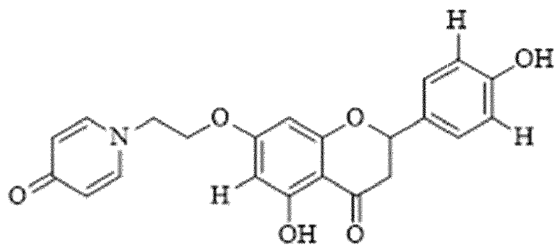

1-(2-((5-hydroxy-2-(4-hydroxyphenyl)-4-oxochroman-7-yl)oxy)ethyl)pyridin-4(1H)-one,

2.

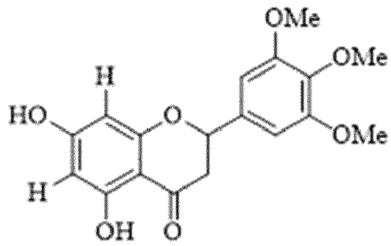

5,7-dihydroxy-2-(3,4,5-trimethoxyphenyl)chroman-4-one,

3.

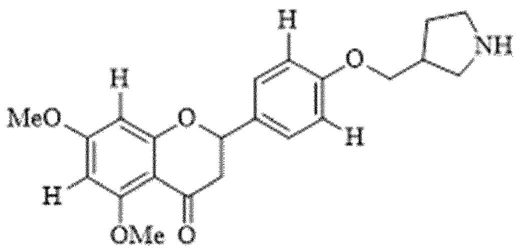

5,7-dimethoxy-2-(4-(pyrrolidin-3-ylmethoxy)phenyl)chroman-4-one,

4.

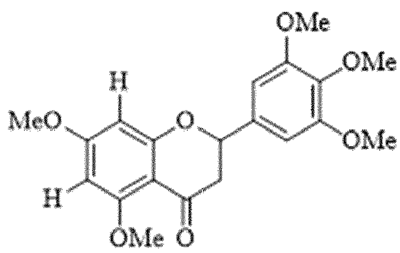

5,7-dimethoxy-2-(3,4,5-trimethoxyphenyl)chroman-4-one,

5.

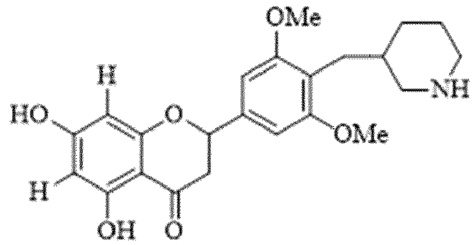

2-(3,5-dimethoxy-4-(piperidin-3-ylmethyl)phenyl)-5,7-dihydroxychroman-4-one,

6.

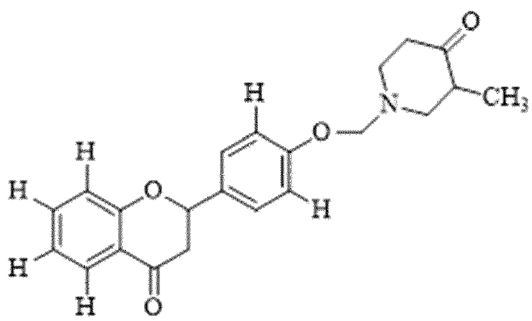

3-methyl-1-((4-(4-oxochroman-2-yl)phenoxy)methyl)piperidin-4-one,

7.

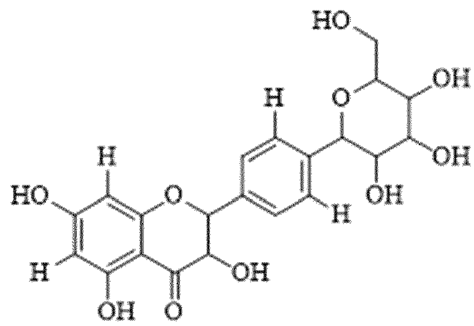

3,5,7-trihydroxy-2-(4-(3,4,5-trihydroxy-6-(hydroxymethyl)tetrahydro-2H-pyran-2-yl)phenyl)chroman-4-one,

8.

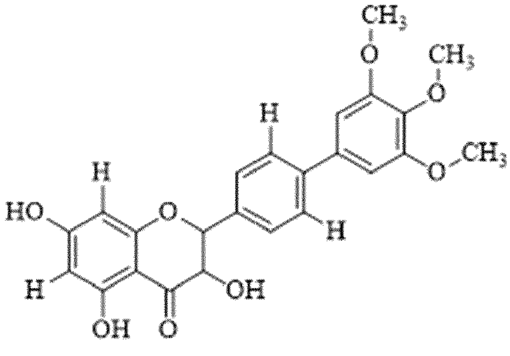

3,5,7-trihydroxy-2-(3',4',5'-trimethoxy-[1,1'-biphenyl]-4-yl)chroman-4-one,

9.

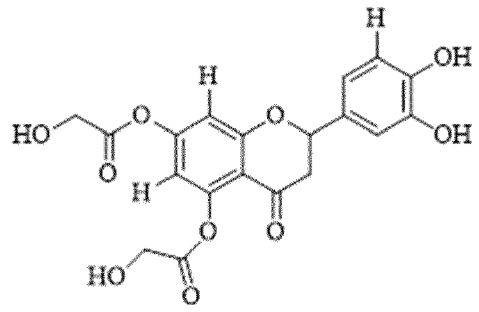

2-(3,4-dihydroxyphenyl)-4-oxochroman-5,7-diyl bis(2-hydroxyacetate),

10.

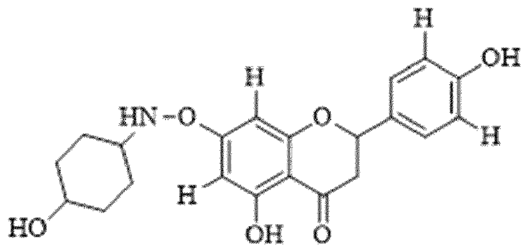

5-hydroxy-7-(((4-hydroxycyclohexyl)amino)oxy)-2-(4-hydroxyphenyl)chroman-4-one,

11.

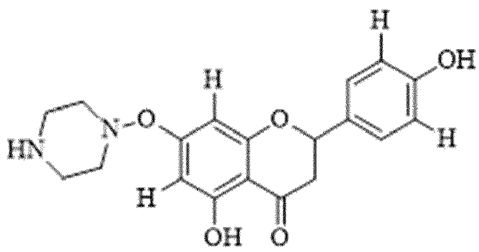

5-hydroxy-2-(4-hydroxyphenyl)-7-(piperazin-1-yloxy)chroman-4-one.

In a preferred embodiment, the active compound for polarization or differentiation of monocytes and repolarization of macrophages into a phenotype with anti-tumor properties is a derivative of quercetin.

Quercetin (3,3',4',5,7-pentahydroxyflavone) is a flavonol belonging to a class of plant secondary metabolites known as flavonoids. It is commonly found in glycosylated form in plants such as Ginkgo biloba, Aesculus hippocastanum, and Hypericum perforatum, and in foods and beverages such as onions, apples, broccoli, berries, green tea, and red wine.

An analysis of the quercetin derivative of the invention revealed that substitution of the hydroxyl groups of the natural compound in various combinations by acetyl esters, ethyl or benzyl ethers, or diphenyl ketals of the catechol system could suppress the proliferating effect of tumor cells and lead to improved anticancer activity compared to natural quercetin. The inventors synthesized several 0-substituted quercetin analogs and investigated their efficacy as inhibitors. Among the various naturally occurring flavonoid scaffolds, a flavonol structure was selected characterized by the planarity of its heterocyclic ring and the presence of a hydroxyl group at the 3-position. The goal was first to investigate the influence of lipophilic or hydrophilic variations in the five substituents around the quercetin moiety and then to investigate the significance of these variations in the 3- and 5-positions of quercetin. The changes in these two positions near the carbonyl group of the pyrone ring most likely lead to the most important changes in the overall electronic and lipophilic properties of

the whole molecule. This strategy resulted in the unexpected discovery of the quercetin derivative of the invention and their use as new potent and highly selective anticancer drugs.

As shown by the present invention, an alkylation of the hydroxyl groups at C-3, C-4', and C-7 in quercetin with short, linear alkyl groups (such as methyl groups in 3 and ethyl groups in 6) increases its antiproliferative activity. The incorporation of ethyl groups into the 3,3',4'-hydroxyl groups of quercetin increase in its inhibitory activity. A structural analysis indicated that the simultaneous introduction of three bulky or very long alkyl groups into the C-3, C-4', and C-7 hydroxyl groups of quercetin could result in a significant loss of antiproliferative activity and that an incorporation of two methyl or ethyl groups into the C-3 and C-7 hydroxyl groups could result in much more potent derivatives.

A preferred quercetin derivative of the invention comprises the following general structure:

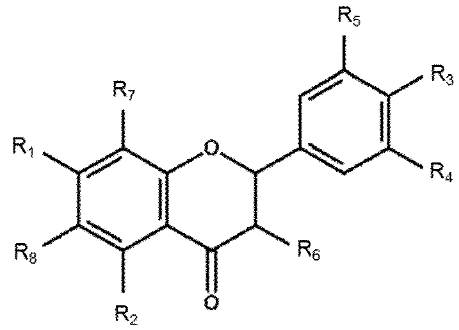

wherein:

$R_1$= $-O-CH_2-OH$, $-OH$, $-O-CO-CH_2-CO-O-CH_3$
$R_2$ = $-O-CH_2-OH$, $-OH$, $-O-CO-CH_2-CO-O-CH_3$
$R_3$ = $-O-CH_2-OH$, $-H$, $-O-CH_2-O-CO-(CH_2)_2-OH$, $-OH$,
$-O-CO-CH_2-CO-O-CH_3$,
$R_4$ = $-O-CH_2-OH$, $-O-CH_3$, $-O-CH_2-O-CO-(CH_2)_2-OH$, $-OH$,

$-O-CH_2-O-CO-O-CH-(CH_3)_2$,
$-O-CO-CH_2-CO-O-CH_3$

$R_5$ = $-OH$, $-O-CH_2-OH$, $-O-CH_3$, $-H$, $-O-CO-CH_2-CO-O-CH_3$
$R_6$ = $-H$, $-OH$,
$R_7$ = $-H$, $-OH$,
$R_8$ = $-H$, $-O-CH_3$, $-O-CH_2-O-CO-(CH_2)_2-OH$, $-OH$,
$-O-CO-CH_2-CO-O-CH_3$

In preferred embodiments of the invention, the quercetin derivative is selected from the group consisting of:

1.

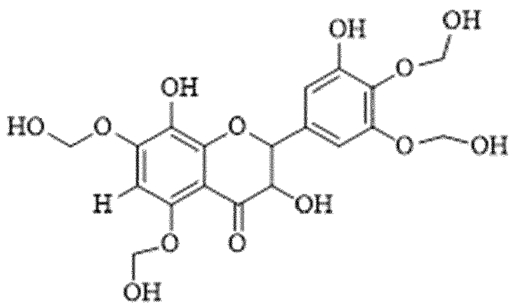

3,8-dihydroxy-2-(3-hydroxy-4,5-bis(hydroxymethoxy)phenyl)-5,7-bis(hydroxymethoxy)chroman-4-one,

2.

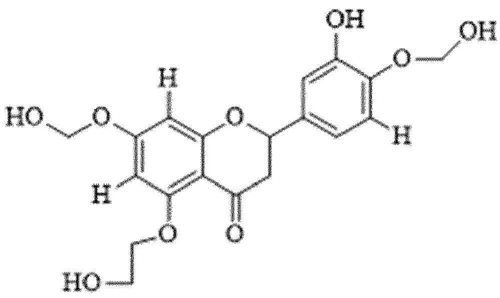

2-(3-hydroxy-4-(hydroxymethoxy)phenyl)-5-(2-hydroxyethoxy)-7-(hydroxymethoxy)chroman-4-one,

3.

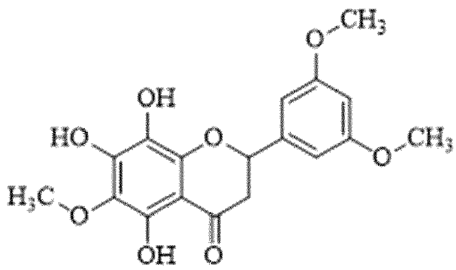

2-(3,5-dimethoxyphenyl)-5,7,8-trihydroxy-6-methoxychroman-4-one,

4.

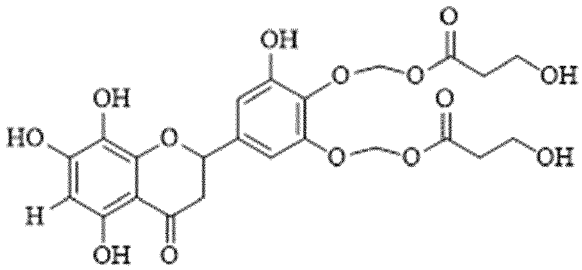

((3-hydroxy-5-(5,7,8-trihydroxy-4-oxochroman-2-yl)-1,2-phenylene)bis(oxy))bis(methylene) bis(3-hydroxypropanoate),

5.

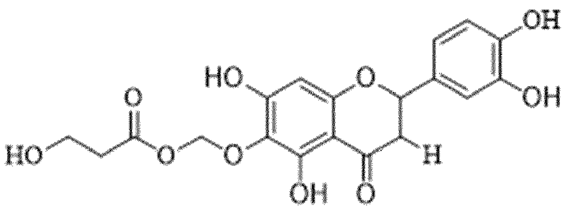

((2-(3,4-dihydroxyphenyl)-5,7-dihydroxy-4-oxochroman-6-yl)oxy)methyl 3-hydroxypropanoate,

6.

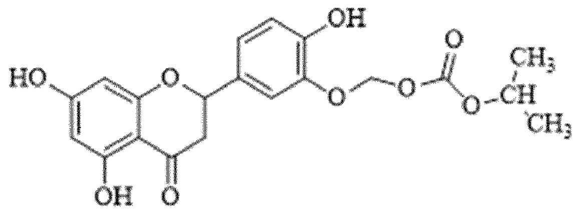

(5-(5,7-dihydroxy-4-oxochroman-2-yl)-2-hydroxyphenoxy)methyl isopropyl carbonate,

7.

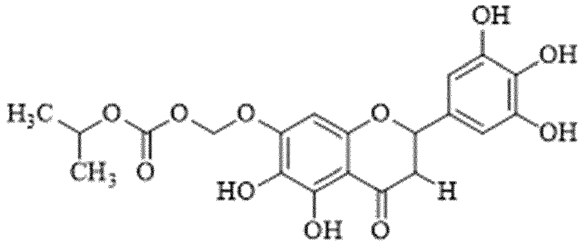

((5,6-dihydroxy-4-oxo-2-(3,4,5-trihydroxyphenyl)chroman-7-yl)oxy)methyl isopropyl carbonate,

8.

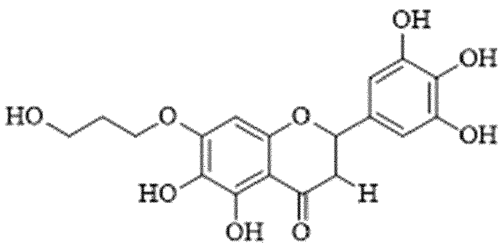

5,6-dihydroxy-7-(3-hydroxypropoxy)-2-(3,4,5-trihydroxyphenyl)chroman-4-one,

9.

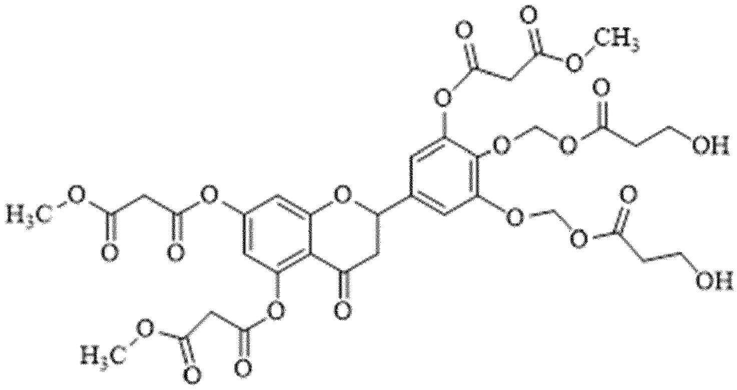

O,O'-(2-(3,4-bis(((3-hydroxypropanoyl)oxy)methoxy)-5-((3-methoxy-3-oxopropanoyl)oxy)phenyl)-4-oxochroman-5,7-diyl) dimethyl dimalonate,

10.

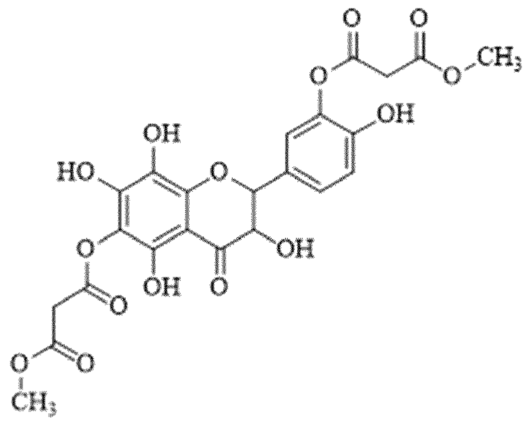

2-hydroxy-5-(3,5,7,8-tetrahydroxy-6-((3-methoxy-3-oxopropanoyl)oxy)-4-oxochroman-2-yl)phenyl methyl malonate.

In a preferred embodiment, the active compound for polarization or differentiation of monocytes and repolarization of macrophages into a phenotype with anti-tumor properties is a derivative of curcumin.

Curcuma longa, curcumin [(1E,6E)-1,7-bis(4- hydroxy-3-methoxyphenyl)hepta-1,6-diene-3,5-dione], is used in as a spice, as a cosmetic, and in the ancient Ayurvedic system of medicine. Curcumin has a strong anti-invasive effect. Several curcumin derivatives were prepared to enhance steroid center inhibition, DAPK1 and NFKB inhibition via STAT3 inhibition and associated caspase pathway activation. In the course of the present invention, amide-containing curcumin derivatives were synthesized, which exhibited improved water solubility and a lipophilicity that showed a better tendency to cross the blood-brain barrier. Another advantage of the nitrogen atoms in the molecule is their electron-rich character.

A preferred curcumin derivative of the invention comprises the following general structure:

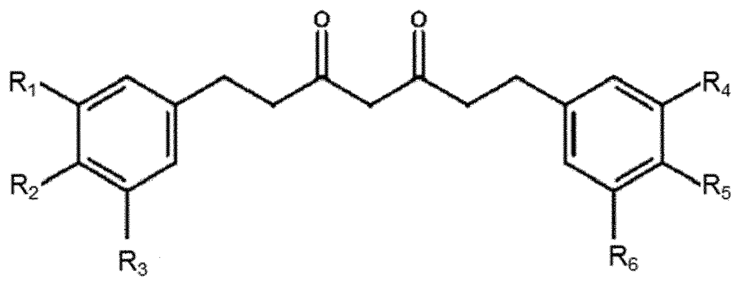

wherein:

$R_1$ = - H, -OH, -O-Me,
$R_2$ = $-CH_2$-OH, -O, -O-CH=CH-CO-O-$CH_2$-Me,
-O-$CH_2$-CH2-CO-OH, -O-$CH_2$-CH=$CH_2$,

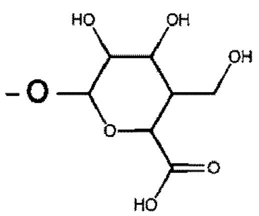

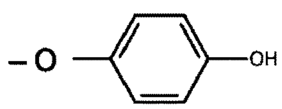

,

$R_3$ = -H, -OH
$R_4$ = -H, -OH, -O-Me
$R_5$ = -H, $-CH_2$-OH, -O,-O-CH=CH-CO-O-$CH_2$-Me,
-O-$CH_2$-$CH_2$-CO-OH, -O-$CH_2$-CH=$CH_2$,

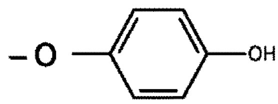

,

$R_6$ = - H.

In preferred embodiments, the curcumin derivative is selected from the group consisting of:

1.

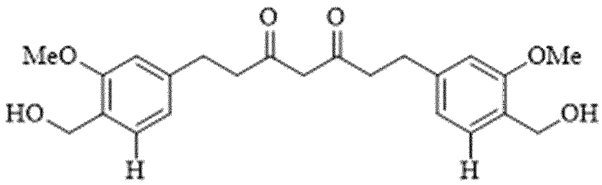

1,7-bis(4-(hydroxymethyl)-3-methoxyphenyl)heptane-3,5-dione,

2.

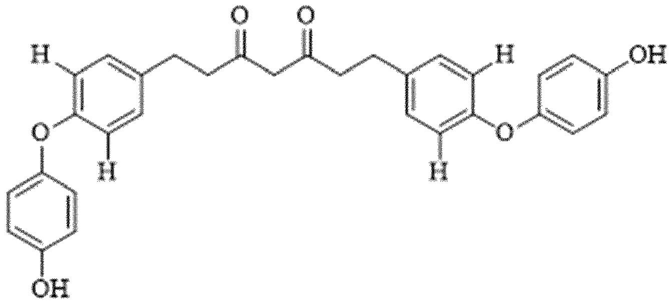

1,7-bis(4-(4-hydroxyphenoxy)phenyl)heptane-3,5-dione,

3.

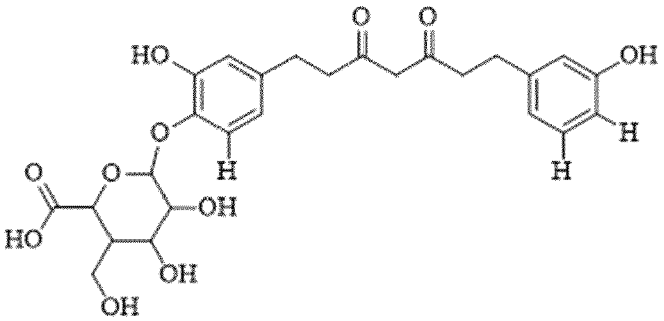

4,5-dihydroxy-6-(2-hydroxy-4-(7-(3-hydroxyphenyl)-3,5-dioxoheptyl)phenoxy)-3-(hydroxymethyl)tetrahydro-2H-pyran-2-carboxylic acid,

4.

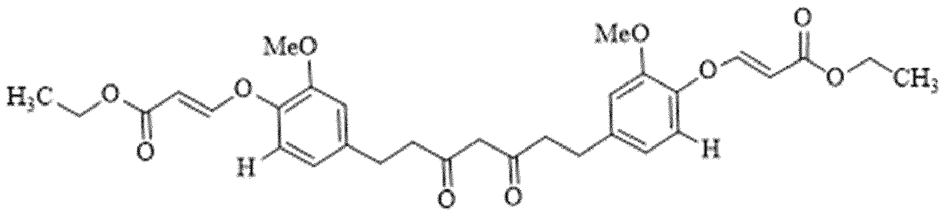

(2E,2'E)-diethyl 3,3'-(((3,5-dioxoheptane-1,7-diyl)bis(2-methoxy-4,1-phenylene))bis(oxy))diacrylate,

5.

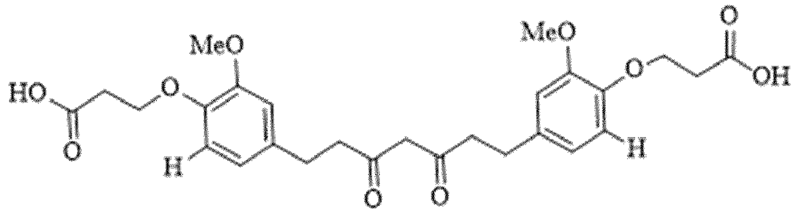

3,3'-(((3,5-dioxoheptane-1,7-diyl)bis(2-methoxy-4,1-phenylene))bis(oxy))dipropanoic acid,

6.

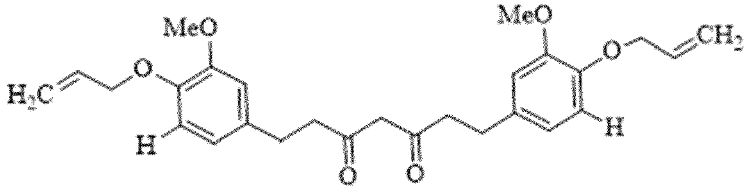

1,7-bis(4-(allyloxy)-3-methoxyphenyl)heptane-3,5-dione,

7.

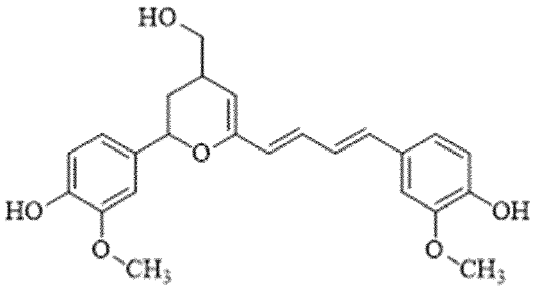

4-((1E,3E)-4-(2-(4-hydroxy-3-methoxyphenyl)-4-(hydroxymethyl)-3,4-dihydro-2H-pyran-6-yl)buta-1,3-dien-1-yl)-2-methoxyphenol.

In a preferred embodiment, the active compound for polarization or differentiation of monocytes and repolarization of macrophages into a phenotype with anti-tumor properties is a derivative of resveratrol.

Resveratrol, (RES; 3,4',5-trihydroxy-stilbene) is a natural stilbene found in various foods and beverages, and has attracted increasing attention over the past decade due to its multiple beneficial properties, including chemopreventive and antitumor effects. There are several other natural derivatives of resveratrol that are structurally similar to resveratrol and are also found in foods. Resveratrol exerts various biological activities beneficial to human health, including chemopreventive, anti-inflammatory, antioxidant, anti proliferative, proapoptotic, cardioprotective, and anticancer properties. Resveratrol interferes with multiple signaling pathways, including inflammatory mediators (e.g., COX-1/2, iNOS, TNF), transcription factors (e.g., NF-κB, β-catenin, STAT3, PPAR-γ), cell cycle regulatory genes (e.g., cycling, p53, CDKs), angiogenesis genes (e.g., VEGF, MMPs, ICAM-1), apoptotic genes (e.g., Survivin, Bcl-2, Bcl-XL), antioxidant enzymes (e.g., SOD, CAT, HO-1), protein kinases (e.g., AKT, PI3K, JNK), and many others.

A preferred resveratrol derivative of the invention comprises the following general structure:

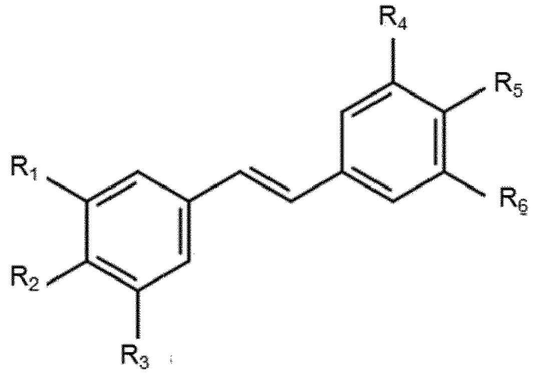

wherein:

$R_1$ = -H, -OH, -O-Me, -O-$CH_2$-CO-O-CH=$CH_2$, -O-CO-Me,

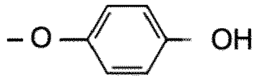

$R_2$ = -H, -OH, -O-Me,

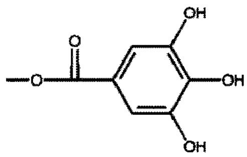

$R_3$ = -H, -OH, -O-Me, -O-CO-Me, -O-$CH_2$-CO-O-CH=$CH_2$,

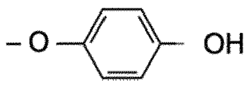
,
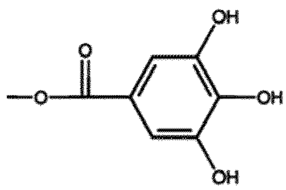
,

$R_4$ = -H, -OH, -O-Me, -O-CO-Me,

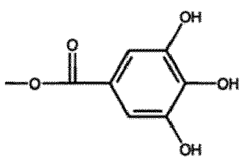
,

$R_5$ = -H, -OH, -O-Me, -O-CO-Me, -O-$CH_2$-CO-O-CH=$CH_2$

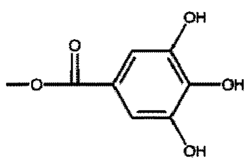

$R_6$ = -H, -OH, -O-Me, -O-CO-Me,

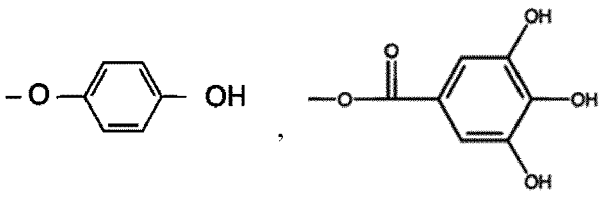

In preferred embodiments, the resveratrol derivative is selected from the group consisting of:

1.

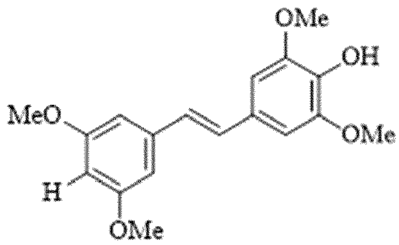

4-(3,5-dimethoxystyryl)-2,6-dimethoxyphenol,

2.

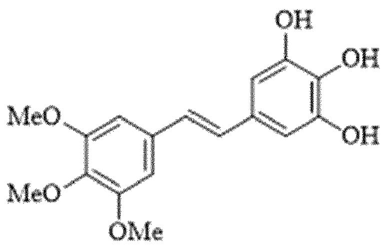

5-(3,4,5-trimethoxystyryl)benzene-1,2,3-triol,

3.

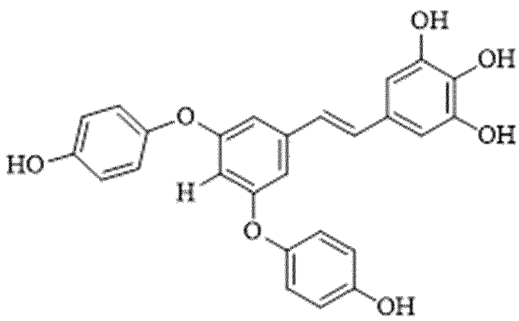

5-(3,5-bis(4-hydroxyphenoxy)styryl)benzene-1,2,3-triol,

4.

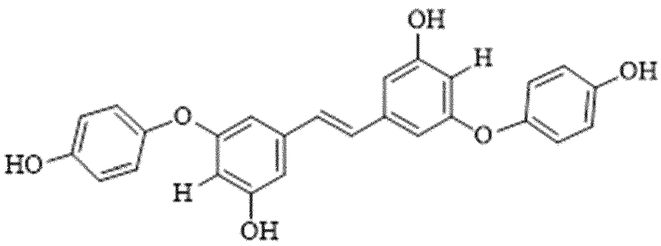

(5,5'-(ethene-1,2-diyl)bis(3-(4-hydroxyphenoxy)phenol),

5.

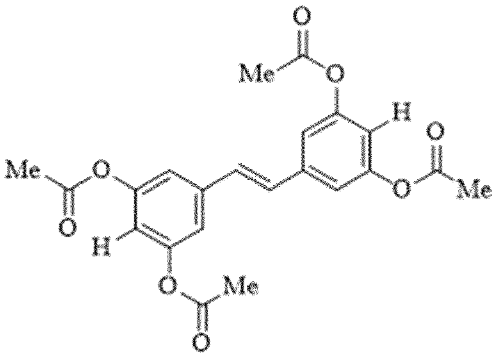

ethene-1,2-diylbis(benzene-5,3,1-triyl) tetraacetate,

6.

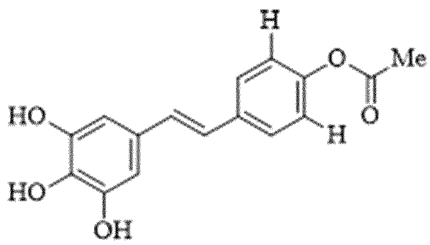

4-(3,4,5-trihydroxystyryl)phenyl acetate,

7.

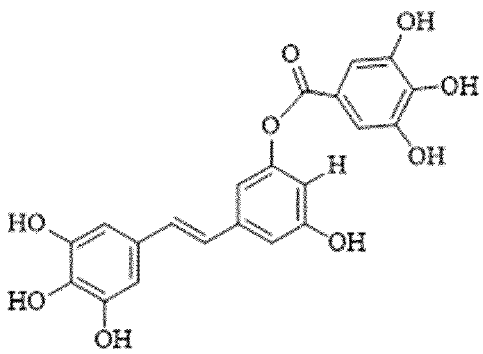

3-hydroxy-5-(3,4,5-trihydroxystyryl)phenyl 3,4,5-trihydroxybenzoate,

8.

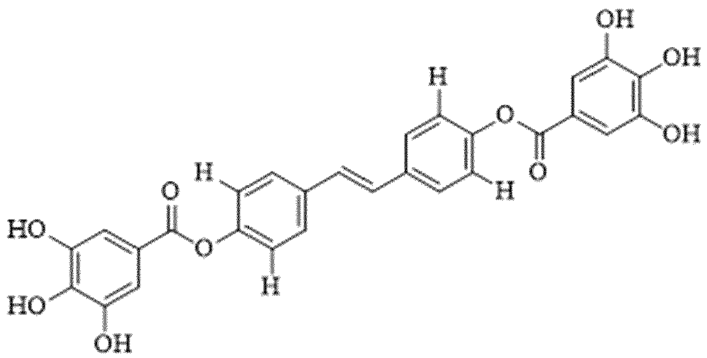

ethene-1,2-diylbis(4,1-phenylene) bis(3,4,5-trihydroxybenzoate),

9.

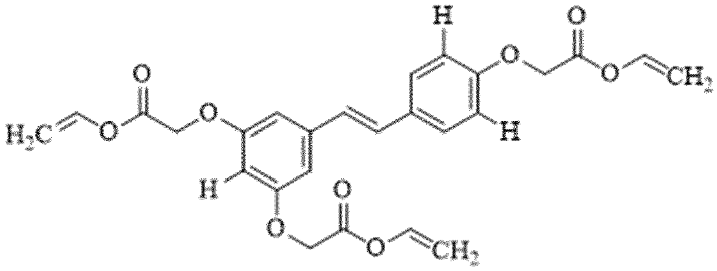

divinyl 2,2'-((5-(4-(2-oxo-2-(vinyloxy)ethoxy)styryl)-1,3-phenylene)bis(oxy))diacetate.

In a preferred embodiment, the active compound for polarization or differentiation of monocytes and repolarization of macrophages into a phenotype with anti-tumor properties is a derivative of lupeol.

Lupeol is a pentacyclic triterpenoid widely distributed in the plants and found in edible fruits and vegetables. It is a naturally occurring triterpene used to reduce inflammatory responses and also has immunomodulatory properties. Lupeol has a great potential to act as anti-inflammatory, antimicrobial, anti-proliferative, anti-invasive, anti-angiogenic, anti-protozoal, and cholesterol-lowering agents.

Derivatization of lupeol is expected not only to enhance existing tumor-killing mechanisms, but also to amplify other molecular intrinsic pathways. A treatment of a subject with a lupeol derivative according to the present invention could increase ROS (reactive oxygen species) levels, increase Bax/Bcl-2 ratio, and then enhance PARP (poly (ADP-ribose) polymerase) cleavage, activating caspases and initiating the execution phase of apoptosis. In addition, apoptosis via the p53 pathway is expected to be promoted. Furthermore, an anti-tumor response triggered by the lupeol derivative of the invention could induce apoptosis of cancer cells by inhibiting the Akt/PKB pathway. Furthermore, the lupeol derivative of the invention could reduce the activity of AKT1 by inhibiting its phosphorylation. This effect may inhibit the phosphorylation of the Bcl-2-associated death promoter (BAD) protein, which triggers the tumor cell killing function of BAD.

A further mechanism that could be induced by a lupeol derivative of the invention is apoptosis of cancer cells by suppressing EGFR/STAT3 activities. EFGR (epidermal growth factor receptor) is a transmembrane protein that plays an important role in non-small cell lung cancer (NSCLC). Another response that may be triggered by the lupeol derivative of the invention is the inhibition of cell proliferation via the PI3K/Akt and Wnt signaling pathways. Mutations in the Wnt signaling pathway are often associated with tumor progression.

Natural lupeol can be extracted from the leaves of *Aegle marmelos* and its ester derivatives possess C-3 anti hyperglycemic activity. Lupeol has a nonpolar molecular skeleton, so it has low bioavailability and water solubility. To overcome these problems, the inventors synthesized lupeol derivatives as prodrugs with increased bioavailability. Although it has a large number of stereogenic centers, the molecule can be functionalized at two positions, C-3 and C-30.

Accordingly, chemical modifications to the structure of lupeol were required to improve its biological activities while exhibiting lower toxicity. Indole groups containing only one nitrogen atom or cyclic di-nitrogen groups have a noticeable effect on the activity and also exhibit strong inhibitory effects. In addition, substituted electron-donating groups on the phenyl ring of the indole moiety enhance the activity.

A preferred lupeol derivative of the invention comprises the following general structure:

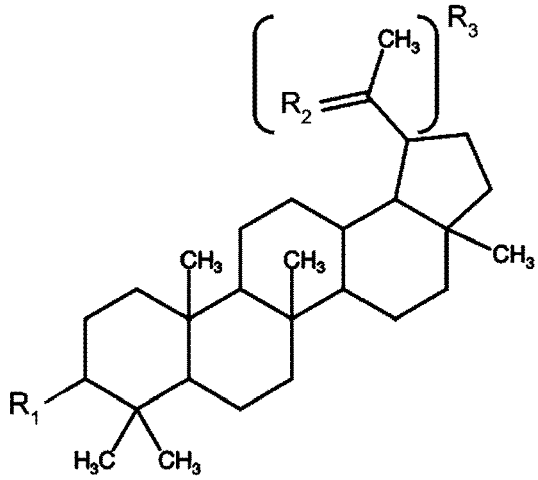

wherein:

$R_1$ = -OH, -O-CO-$CH_2$-OH, -O-CO-$CH_2$-$CH_2$-OH,

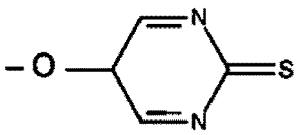 , 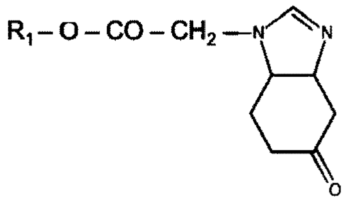

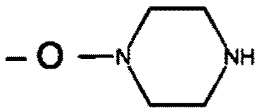 , 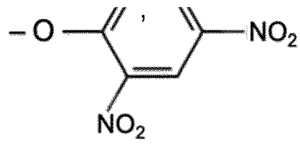 ,

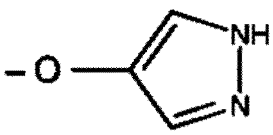 , 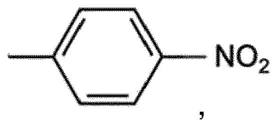 ,

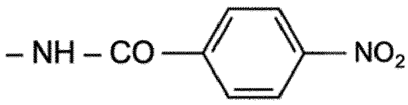 ,

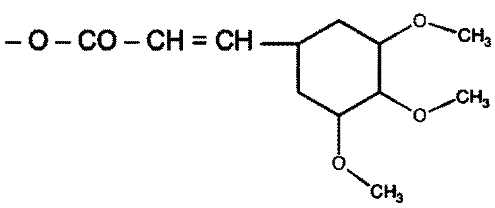

$R_2$ = – O – CO – Me, 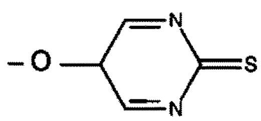 , 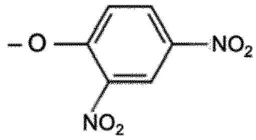 ,

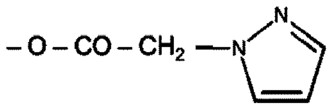 , 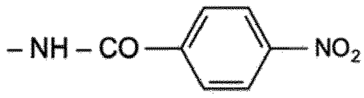 ,

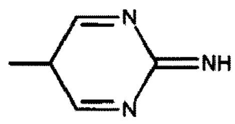

R3 = 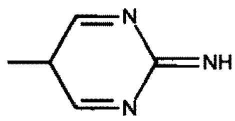

In preferred embodiments, the lupeol derivative of the invention is selected from the group consisting of:

1.

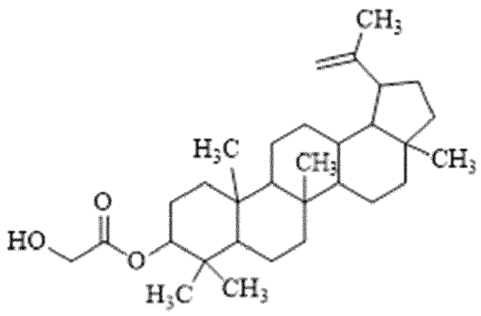

3a,5b,8,8,11a-pentamethyl-1-(prop-1-en-2-yl)icosahydro-1H-cyclopenta[a]chrysen-9-yl 2-hydroxyacetate,

2.

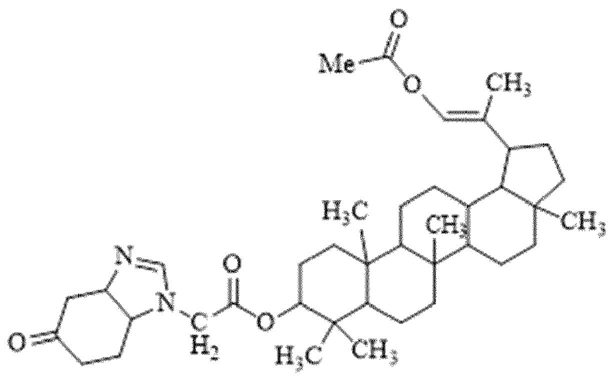

1-(1-acetoxyprop-1-en-2-yl)-3a,5b,8,8,11a-pentamethylicosahydro-1H-cyclopenta[a]chrysen-9-yl 2-(5-oxo-3a,4,5,6,7,7a-hexahydro-1H-benzo[d]imidazol-1-yl)acetate,

3.

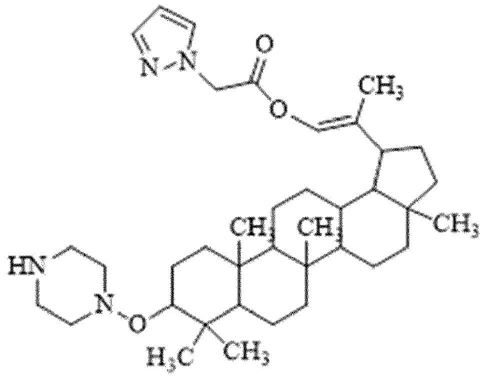

2-(3a,5b,8,8,11a-pentamethyl-9-(piperazin-1-yloxy)icosahydro-1H-cyclopenta[a]chrysen-1-yl)prop-1-en-1-yl 2-(1H-pyrazol-1-yl)acetate,

4.

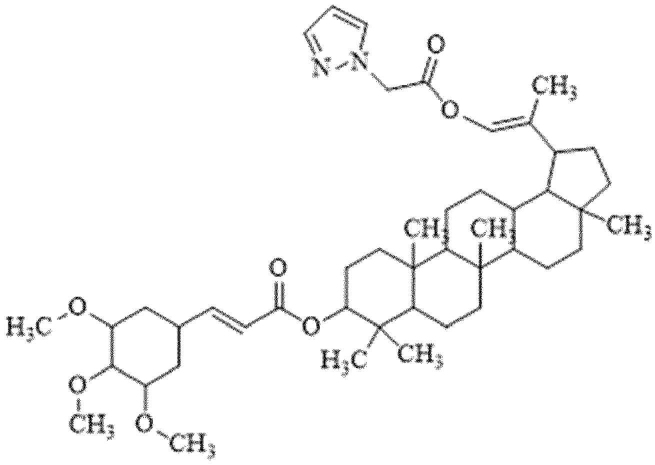

1-((E)-1-(2-(1H-pyrazol-1-yl)acetoxy)prop-1-en-2-yl)-3a,5b,8,8,11a-pentamethylicosahydro-1H-cyclopenta[a]chrysen-9-yl 3-(3,4,5-trimethoxycyclohexyl)acrylate,

5.

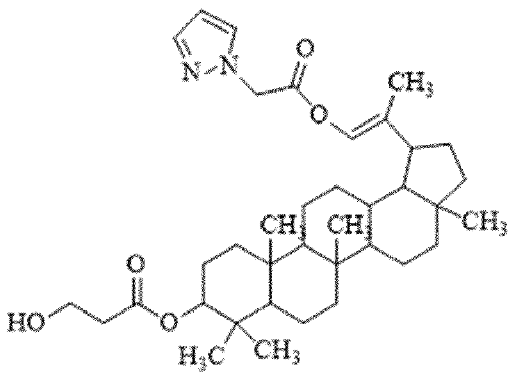

1-(1-(2-(1H-pyrazol-1-yl)acetoxy)prop-1-en-2-yl)-3a,5b,8,8,11a-pentamethylicosahydro-1H-cyclopenta[a]chrysen-9-yl 3-hydroxypropanoate,

6.

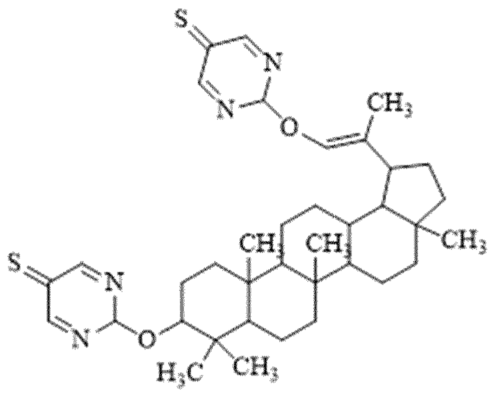

2-((3a,5b,8,8,11a-pentamethyl-1-(1-((5-thioxo-2,5-dihydropyrimidin-2-yl)oxy)prop-1-en-2-yl)icosahydro-1H-cyclopenta[a]chrysen-9-yl)oxy)pyrimidine-5(2H)-thione,

7.

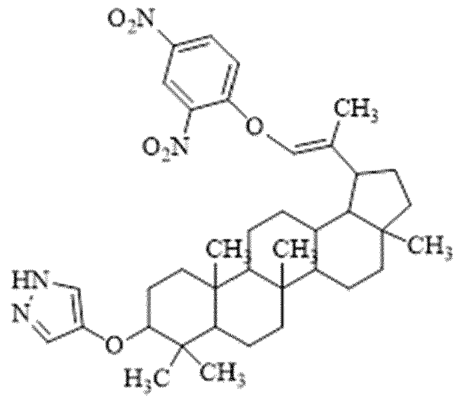

4-((1-(1-(2,4-dinitrophenoxy)prop-1-en-2-yl)-3a,5b,8,8,11a-pentamethylicosahydro-1H-cyclopenta[a]chrysen-9-yl)oxy)-1H-pyrazole,

8.

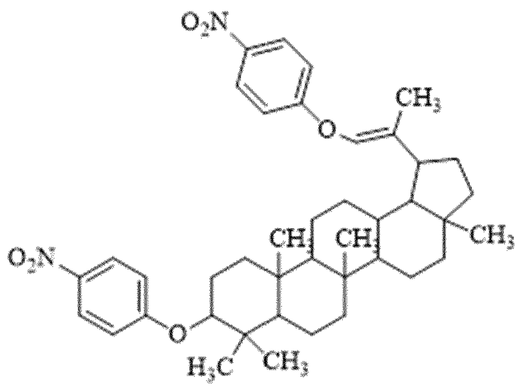

3a,5b,8,8,11a-pentamethyl-9-(4-nitrophenoxy)-1-(1-(4-nitrophenoxy)prop-1-en-2-yl)icosahydro-1H-cyclopenta[a]chrysene,

9.

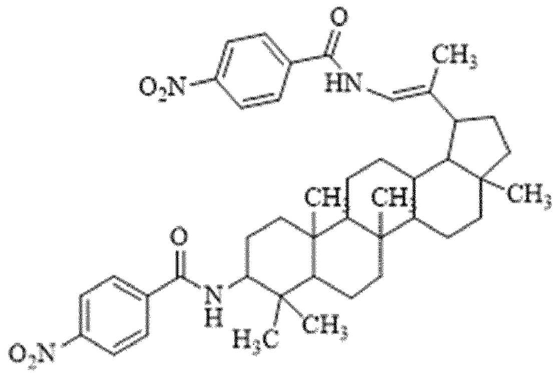

4-nitro-N-(3a,5b,8,8,11a-pentamethyl-1-(1-(4-nitrobenzamido)prop-1-en-2-yl)icosahydro-1H-cyclopenta[a]chrysen-9-yl)benzamide,

10.

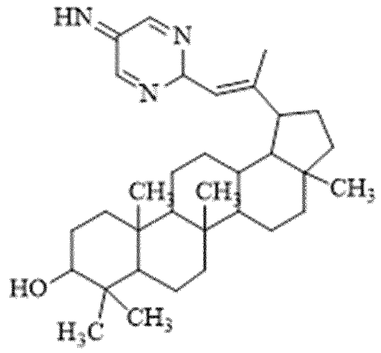

1-(1-(5-imino-2,5-dihydropyrimidin-2-yl)prop-1-en-2-yl)-3a,5b,8,8,11a-pentamethylicosahydro-1H-cyclopenta[a]chrysen-9-ol,

11.

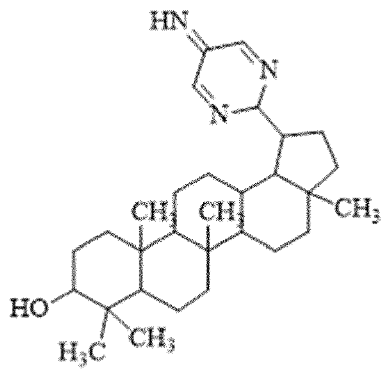

1-(5-imino-2,5-dihydropyrimidin-2-yl)-3a,5b,8,8,11a-pentamethylicosahydro-1H-cyclopenta[a]chrysen-9-ol.

In a preferred embodiment, the active compound for polarization or differentiation of monocytes and repolarization of macrophages into a phenotype with anti-tumor properties is a derivative of indol-3-carbinol.

Indole-3-carbinol is a naturally occurring compound resulting from the hydrolysis of glucobrassicin and is present in exceptionally high concentrations in Brassica vegetables. It is known that oral administration of indol-3-carbinol prevented carcinogenic breast tumor formation in animal models.

An addition of substituents to the nitrogen at position 1 in the indole ring with the more hydrophobic alkyl groups is likely to elicit increased potency with up to several-fold increase in the reaction, suggesting that substituents that increase

lipophilicity and alter the nitrogen of the indole ring may enhance activity. Binding an alkyl group to the nitrogen atom would thus be a conceivable solution for increased efficiency.

In alternative embodiments, a derivatization with an alkyl group would also be conceivable in the synthesis of an indol-3-carbinol derivative of the present invention. For this purpose, the molecule 1-chlorobutane could be used instead of the p-xylylbromide. In the synthesis of 1-benzyl-indole-3-carbinol, indole is first benzylated, then formylated to the aldehyde and finally reduced to the carbinol.

Furthermore, structural analysis of indol-3-carbinol predicted that addition of substituents to the nitrogen at position 1 in the indole ring with a more hydrophobic cyclic alkyl group with another electron-rich nitrogen atom had the highest potency. Accordingly, the indol-3-carbinol derivatives of the present invention exhibit a significantly increased hydrophobic and electron-rich character. The increased hydrophobic nature of the indol-3-carbinol derivative of the present invention as compared to natural indol-3-carbinol enhances its potency through improved stability and/or increased cellular uptake.

Treatment with an indol-3-carbinol derivative of the invention results in a robust inhibition of CDK2-specific enzymatic activity and downregulation of CDK6 transcript and protein levels, which is predicted to lead to loss of overall cellular CDK6 activity. CDK6 is a member of the cyclin-dependent family of protein kinases, which are important regulators of cell cycle progression and essential for tumor growth. This significant effect from the indol-3-carbinol derivative is likely due to an increase in the level of p15, a potent CDK4 inhibitor. In addition, the I3C derivative is thought to stimulate CDK inhibitors p21 and p27.

A preferred indol-3-carbinol derivative of the invention comprises the following general structure:

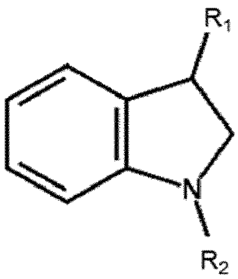

wherein:
$R_1$ = $-CH_2-OH$

$R_2$ =

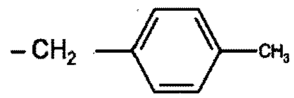
,
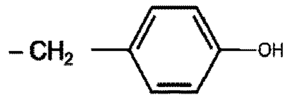

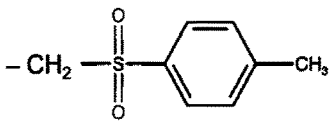
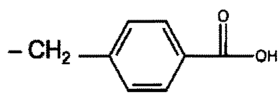
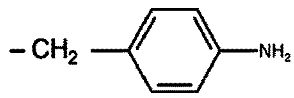

In preferred embodiments, the indol-3-carbinol derivative of the invention is selected from the group consisting of:

1.

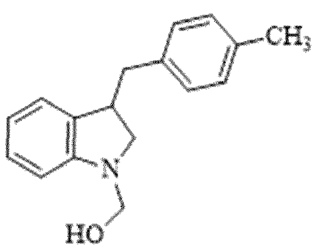

(3-(4-methylbenzyl)indolin-1-yl)methanol,

2.

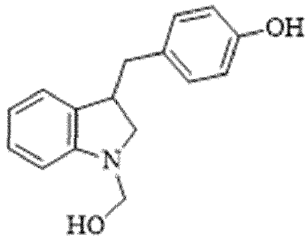

4-((1-(hydroxymethyl)indolin-3-yl)methyl)phenol,

3.

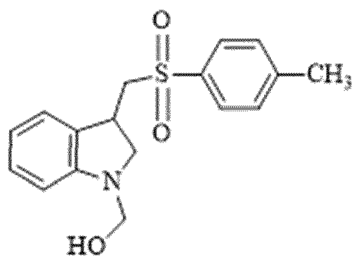

(3-(tosylmethyl)indolin-1-yl)methanol,

4.

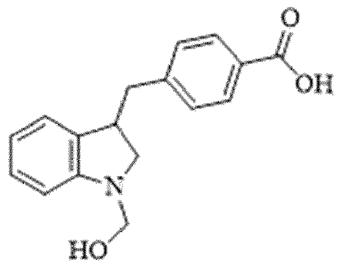

4-((1-(hydroxymethyl)indolin-3-yl)methyl)benzoic acid,

5.

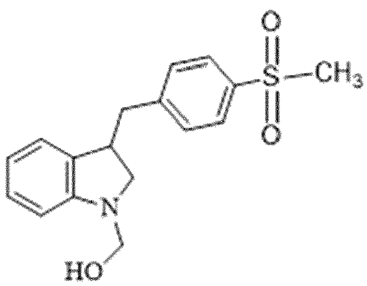

(3-(4-(methylsulfonyl)benzyl)indolin-1-yl)methanol,

6.

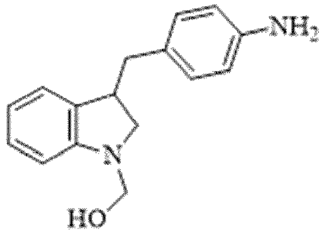

(3-(4-aminobenzyl)indolin-1-yl)methanol.

In a preferred embodiment, the active compound for polarization or differentiation of monocytes and repolarization of macrophages into a phenotype with anti-tumor properties is a derivative of piperlongumin.

Piperlongumin is a naturally occurring alkaloid with a cyclic amide isolated from the species Piper. Natural piperiongumin has anticancer, antidiabetic, antiplatelet, and antifungal properties. The compound also induces cell death in many cancer lines and has excellent oral bioavailability, inhibits tumor growth, and has low systemic toxicity. Piperiongumin inhibits the activity of STAT3 by directly binding to STAT3 and upregulating reactive oxygen species ROS by inhibiting the ubiquitin-proteasome system, an important intracellular protein degradation system. The increase of ROS in cancer cells is a cytotoxic mechanism to kill tumor cells. ROS generated during cellular respiration are active small molecules responsible for maintaining cellular redox homeostasis. ROS mediate various forms of programmed cell death such as apoptosis, autophagy, pyroptosis, necrosis, and ferroptosis. Pyroptosis, as a newly discovered form of programmed cell death, is typically characterized by cell swelling, membrane pore formation, cell lysis, and release of pro-inflammatory molecules.

The piperlongumin derivative of the invention retains oxidative stress-inducing properties while inhibiting microtubule polymerization. Structural analysis revealed that the presence of the 3, 4, 5 trimethoxy-substituted A ring and an electron-rich B ring separated by a double bond in a carbon linker are essential for the antiproliferative activity. The introduction of a di-hydroxide group in the carbon linker could trigger potent anti-microtubule properties. The IKK complex is the master regulator of NF-kB activation and harbors a cysteine 179 (Cys179) in the active site of the IKK catalytic subunit IKKb. This active cysteine residue can serve as a potential target for some electrophilic and hydrophilic molecules containing a Michael acceptor moiety. It is hypothesized that the piperiongumin derivative of the invention exhibits a stronger inhibitory effect on the phosphorylation of IKKa/b compared to natural piperlongumin, leading to a subsequent failure of phosphorylation of IkBa and entry of p65 and p50 into the nucleus due to the increased hydrophilic electrophiles.

A preferred piperiongumin derivative of the invention is selected from the group consisting of one or more of the following compounds:

1.

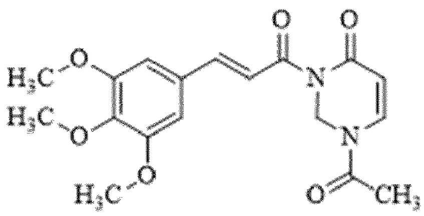

1-acetyl-3-(3-(3,4,5-trimethoxyphenyl)acryloyl)-2,3-dihydropyrimidin-4(1H)-one,

2.

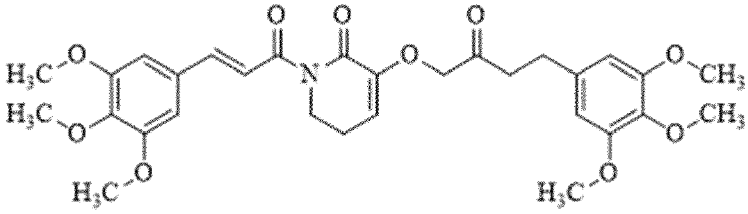

3-(2-oxo-4-(3,4,5-trimethoxyphenyl)butoxy)-1-(3-(3,4,5-trimethoxyphenyl)acryloyl)-5,6-dihydropyridin-2(1H)-one,

3.

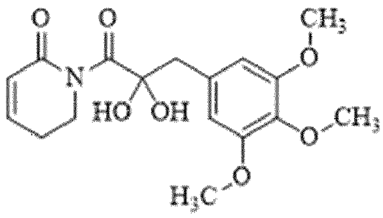

1-(2,2-dihydroxy-3-(3,4,5-trimethoxyphenyl)propanoyl)-5,6-dihydropyridin-2(1H)-one,

4.

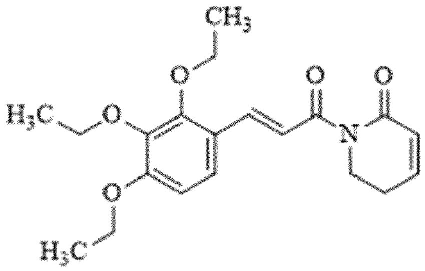

1-(3-(2,3,4-triethoxyphenyl)acryloyl)-5,6-dihydropyridin-2(1H)-one,

5.

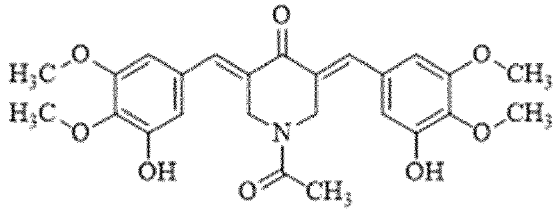

1-acetyl-3,5-bis(3-hydroxy-4,5-dimethoxybenzylidene)piperidin-4-one,

6.

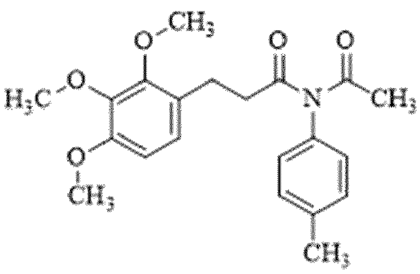

N-acetyl-N-(p-tolyl)-3-(2,3,4-trimethoxyphenyl)propenamide.

In a preferred embodiment, the pharmaceutical composition comprises a mixture of more than two compounds selected from the group consisting of apigenin derivative, indol-3-carbinol derivative, lupeol derivative, piperlongumin derivative, quercetin derivate, curcumin derivative and/or resveratrol derivative. The concentrations of the individual compounds in said mixture may vary in accordance with the individual needs and condition of the subject to be treated. In

some embodiments, the composition is a mixture of all five derivatives apigenin derivative, indol-3-carbinol derivative, lupeol derivative, piperlongumin derivative, quercetin derivate, curcumin derivative and resveratrol derivative. A combination of several derivatives based on different chemical compounds results in synergistic effects and may improve the outcome of an anti-tumor therapy.

In a further preferred embodiment, the pharmaceutical composition of the invention additionally comprises one or more checkpoint inhibitors, preferably selected from the group consisting of CTLA4, PD-1, PD-L1, PD-L2, LAG3, B7-H3, B7-H4, KIR, OX40, IgG, IDO-1, IDO-2, CEACAM1, TNFRSF4, OX40L, TIM3, BTLA, HVEM, TIM3, GAL9, LAG3, VISTA, KIR, 2B4, CD 160, CGEN-15049, CHK 1, CHK2, A2aR, and B-7or combinations thereof.

The checkpoint inhibitors present in said composition preferably target receptors on the membrane of T-lymphocytes that can decrease or increase their immune response. Preferred checkpoint inhibitors that are present in the composition according to the present invention have the ability to switch off immunosuppressive signals by interrupting receptor-ligand binding. In this way, the subject's immune system can recognize and destroy unwanted tumor cells. Preferably, the checkpoint inhibitor is selected from the group consisting of molecules against programmed death-1 (PD-1), PD-Ligand 1 (PD-L1) and cytotoxic T-lymphocyte antigen-4 (CTLA-4). A preferred pharmaceutical composition of the present invention comprises one or more checkpoint inhibitors present in a concentration of between 40 μg/ml and 60pg/ml or any range in between.

A preferred pharmaceutical composition according the present invention comprises a therapeutically effective amount of apigenin derivative, indol-3-carbinol derivative, lupeol derivative, piperlongumin derivative, quercetin derivate, curcumin derivative and/or resveratrol derivative, and a suitable pharmaceutical carrier.

As used herein, the term "pharmaceutically acceptable carrier" includes any and all solvents, dispersion media, coatings, antibacterial and antifungal agents, isotonic and absorption delaying agents, and the like, compatible with pharmaceutical administration. Non-limiting examples of such carriers or diluents include, but are not limited to, water, saline, ringer's solutions, dextrose solution, and 5% human serum albumin. In some embodiments, the liposomes and non-aqueous vehicles such as fixed oils may also be used. In some embodiments, supplementary active compounds are also incorporated into the compositions. The use of such media and agents for pharmaceutically active substances is well known in the art. Except insofar as any conventional media or agent is incompatible with the active compound, use thereof in the compositions is contemplated. In some embodiments, supplementary active compounds are also incorporated into the compositions.

A preferred pharmaceutical composition of the present invention is formulated in a way that fits to its intended route of administration. Solutions or suspensions used for administration are based on the serum-free environment but can include a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose.

The derivatives of the present invention may include classic routes of administration, including, but not limited to oral, nasal, buccal, rectal, vaginal or topical administration. In some embodiments, administration may be by intradermal, subcutaneous, intramuscular, intraperitoneal or intravenous injection.

The pharmaceutical formulations that are suitable for injectable use include sterile aqueous solutions or dispersions and sterile powders for the extemporaneous preparation of sterile injectable solutions or dispersions. The composition to be administered by injection is sterile and fluid to the extent that easy syringability exists. It is stable under the conditions of manufacture and storage and is preserved against the contaminating action of microorganisms, such as bacteria and fungi. The carrier that is used in the context of the present invention can be any solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The composition may also comprise one or more antibacterial or antifungal agents, for example, parabens, chlorobutanol, phenol, sorbic acid, thimerosal, and the like. In some embodiments, isotonic agents, for example, sugars or sodium chloride may be included. Prolonged absorption of the injectable compositions can be achieved by agents that delay absorption, for example, aluminum monostearate and gelatin.

For oral administration, the compositions may be incorporated with excipients and used in the form of non-ingestible mouthwashes and dentifrices. A mouthwash may be prepared incorporating the active ingredient in the required amount in an appropriate solvent, such as a sodium borate solution ("Dobel's Solution"). Alternatively, the active ingredient may be incorporated into an antiseptic wash containing sodium borate, glycerin and potassium bicarbonate. The active compounds of the invention also may be dispersed in dentifrices, including, for example: gels, pastes, powders and slurries. The active compound of the invention may be added in a therapeutically effective amount to a paste dentifrice that may include, for example, water, binders, abrasives, flavoring agents, foaming agents, and humectants.

The compositions may be formulated in a neutral or salt form. Pharmaceutically-acceptable salts include, for example, hydrochloric or phosphoric acids, or such organic acids as acetic, oxalic, tartaric, mandelic, and the like. Salts

formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

Upon formulation, solutions will be administered in a manner compatible with the dosage formulation and in such amount as is therapeutically effective. The formulations are easily administered in a variety of dosage forms such as injectable solutions, drug release capsules and the like. For parenteral administration in an aqueous solution, for example, the solution may be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media can be employed. Some variation in dosage will necessarily occur depending on the condition of the subject being treated. The person responsible for administration will, in any event, determine the appropriate dose for the individual subject.

The administration dosage schedule may be determined as appropriate for the patient. Administration of a dose of a pharmaceutical composition of the invention may occur in one session, or in more than one session.

The pharmaceutical composition according to the present invention is preferably used for the treatment of cancer. Preferred cancer types to be treated include, but are not limited to solid tumors. As used herein, the term "treatment", refers to prophylaxis and/or therapy. When used with respect to a solid tumor, such as a cancerous solid tumor, the term refers to prevention by prophylactic treatment, which increases the subject's resistance to solid tumors or cancer.

Solid tumors are defined as cancers that can originate in different organs. Preferably the solid tumors are selected from the group comprising melanoma, non-small cell lung carcinoma, clear cell renal cell carcinoma, liver carcinoma, pancreas carcinoma and squamous cell carcinoma. Solid tumors from any tissue or organ may be treated, including, but not limited to any tumor expressing PSA, for example, PSMA, in the vasculature, for example, solid tumors present in, for example, lungs, bone, liver, prostate, or brain, and also, for example, in breast, ovary, bowel, testes, colon, pancreas, kidney, bladder, neuroendocrine system, soft tissue, boney mass, and lymphatic system. Other solid tumors that may be treated include, for example, glioblastoma, and malignant myeloma. Treatment for solid tumor cancers, including, for example, prostate cancer, may be optimized by determining the concentration of one or more marker proteins. For example, the amount or concentration of certain biomarkers can be determined during the course of treatment of solid tumors. Predetermined target levels of such biomarkers, or biomarker thresholds may be identified in normal subject, are provided, which allow a clinician to determine whether a subsequent dose of a drug administered to a subject in need thereof, such as a subject with a solid tumor, e.g., a prostate tumor, may be increased, decreased or maintained.

The present invention also relates to *in-vitro* methods for polarization or differentiation of monocytes or peripheral blood mononuclear cells (PBMCs) into macrophages with a M1-like phenotype having anti-tumor properties.

In a first variant of the method of according to the present invention, monocytes or PBMCs are isolated from a blood sample of a human or other mammalian subject. In some embodiments, a sample from a subject may be processed prior to determining the presence, absence or amount of a biomarker. For example, a blood sample from a subject may be processed to yield a certain fraction, including without limitation, plasma, serum, buffy coat, red blood cell layer and the like, and biomarker presence, absence or amount can be determined in the fraction. In a subsequent step, the isolated monocytes and/or PBMCs are first incubated with M-CSF to differentiate them into M0 macrophages, afterwards incubated with one or more active compounds. According to the invention, the active compound is anyone as described herein, or a combination thereof. Preferably, the active compound is selected from the group consisting of apigenin derivative, indol-3-carbinol derivative, lupeol derivative, piperlongumin derivative, quercetin derivate, curcumin derivative, resveratrol derivative, or a mixture thereof. Incubation with one or more active compounds of the invention, or a mixture thereof results in monocytes or PBMCs that are re-programmed in macrophages with a M1-like phenotype having anti-tumor properties.

The present invention also relates to a method of preparation of repolarized macrophages with M1-like phenotype with anti-tumor properties. In a first variant of the method of the invention, monocytes or PBMCs are isolated from a blood sample of a human or animal subject and differentiated with M-CSF into M0 macrophages. In a subsequent step, the isolated monocytes and/or PBMCs are incubated with one or more active compounds. According to the present invention, the active compound is selected from the group consisting of apigenin derivative, indol-3-carbinol derivative, lupeol derivative, piperlongumin derivative, quercetin derivate, curcumin derivative, resveratrol derivative, or a mixture thereof. Incubation of monocytes or PBMCs with one or more derivatives of the present invention results in a re-polarization to macrophages with a M1-like phenotype having anti-tumor properties. In a final step, the repolarized macrophages are isolated and purified for further use.

In preferred embodiments, the isolation of monocytes or PBMCs is carried out by using antibodies against CD14. The CD14 receptor is a surface protein found primarily on monocytes and macrophages. In preferred embodiments of the invention, the polarization or differentiation of monocytes or PBMCs is carried out by incubation with macrophage colony-stimulating factor M-CSF to convert macrophages into M0 macrophages. The M0 macrophages are then incubated with one or more of the active compounds to convert them into M1-like macrophages.

The invention is further illustrated in the following examples. By no means shall the invention be limited to these specific examples. The invention also comprises combinations or modifications of features described for different derivatives. Although only a small number of derivatives have been tested in the following examples, the inventive concept can be generalized because all derivatives of the present invention have in common that they have the capability of polarization or differentiation of monocytes, and repolarization of macrophages into a phenotype with anti-tumor properties. Thus, the concept of polarization or differentiation of monocytes, and repolarization of macrophages into a phenotype with anti-tumor properties is a common feature of the active compounds of the present invention.

## Examples

The examples in the Figures show the gene expression profiles of macrophages incubated with six different tumor cell lines. Gene expression levels were normalized with housekeeping gene beta-actin and 24- and 48-hour control. The gene expression was measured for each macrophage status:
M0 macrophages without further polarization (showed no gene activity).

| | |
|---|---|
| TME: | M2-like macrophages after incubation in a tumor microenvironment |
| MBME: | M1-like macrophages after polarization with active compounds of the present invention (inventive agents = MBME) |
| MBME-TME: | macrophages after treatment with inventive agents and subsequent incubation with the tumor cell lines (MBME-TME). |

The data has a significance between 0.01 and 0.001.

## Brief Description of the Figures

**Figure 1A-C** shows the gene expression of the macrophages plus beta-actin as control. The macrophages were incubated with A549 tumor cells, which are human lung carcinoma epithelial cells.

**Figure 2A-C** shows the gene expression of the macrophages plus beta-actin as control. The macrophages were incubated with HepG2 tumor cells, which are human liver carcinoma cells.

**Figure 3A-C** shows the gene expression of the macrophages plus beta-actin as control. The macrophages were incubated with Hep3B tumor cells, which are human liver carcinoma cells.

**Figure 4A-C** shows the gene expression of the macrophages plus beta-actin as control. The macrophages were incubated with Panc-1 tumor cells, which are human pancreatic carcinoma cells.

**Figure 5A-C** shows the gene expression of the macrophages plus beta-actin as control. The macrophages were incubated with AsPC1 tumor cells, which are human pancreatic adenocarcinoma cells.

**Figure 6A-C** shows the gene expression of the macrophages plus beta-actin as control. The macrophages were incubated with PLC/PRF/5 human hepatocellular carcinoma cell line The inventive agents (MBME) that were used in a tumor microenvironment (TME) in order to generate the data depicted in Figures 1 to 6 were applied at a concentration of 60 μg/ml.

The microenvironment of cancer cells converts M1 polarized macrophages into M2, e.g., by *IL10* or M-CSF release. M2-like anti-inflammatory tumor-associated macrophages (TAM) promote cancer cell proliferation.
The diagrams A, B and C in Figures 1 to 6 show different target genes, which affected by the inventive derivatives (MBME). The diagrams are explained in more detailed below:
Diagram A as illustrated in Figures 1 to 6 shows 27 genes, which are coding for proteins of the macrophages or work as master regulator to control other genes, which can be used by tumors for angiogenesis, tumor growth and immunosuppression. The data shows that the gene expression of all genes is the highest in M2-like macrophages after incubation in a tumor microenvironment. In M1-like macrophages after repolarization with the active compounds (inventive agents = MBME) and macrophages after treatment with inventive agents and subsequent incubation with the tumor cell lines (MBME-TME) the gene expression is significantly lower.

Diagram B as illustrated in Figures 1 to 6 shows 15 genes, which are coding for proteins and/or work as regulator for other genes with immunostimulatory, especially antitumoral effect. The data show the highest level of gene expression in the macrophages after treatment with the inventive agents and subsequent incubation with the tumor cell lines (MBME-TME) and in the M1-like macrophages after repolarization with the active compounds (inventive agents = MBME). The gene expression of M2-like macrophages after incubation in a tumor microenvironment is significantly lower.

Diagram C as illustrated in Figures 1 to 6 shows 19 genes, which are coding for proteins of the macrophages, which can be used by tumors for inhibition of the immune response. The data illustrate that the gene expression of all genes is the highest in M2-like macrophages after incubation in a tumor microenvironment. In M1-like macrophages after repolarization with the active compounds (inventive agents = MBME) and macrophages after treatment with inventive agents and subsequent incubation with the tumor cell lines (MBME-TME) the gene expression is significantly lower.

In summary, the data illustrate that the inventive derivatives have the potential to repolarize M0 macrophages into M1-like macrophages, keep the M1 phenotype in macrophages in the tumor microenvironment (TME-MBME) and to differentiate monocytes into immune cells having anti-tumor properties. The inventive compounds can be used as effective therapeutic agents for the prevention or treatment of cancer, in particular cancer that are characterized by solid tumors.

**Figure 7A-H** shows the kinetics of tumor cell viability measured after treatment with a resveratrol derivative (named as MLB007R1) as an example using eight tumor models.

The resveratrol derivative MLB007R1 has the following structure:

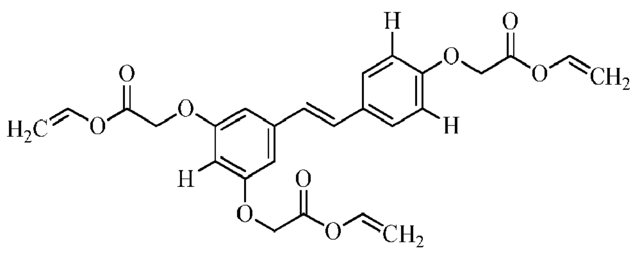

divinyl 2,2'-((5-(4-(2-oxo-2-(vinyloxy)ethoxy)styryl)-1,3-phenylene)bis(oxy))diacetate

A structural analysis revealed that substitution of the hydroxyl groups of resveratrol with methoxy groups could significantly enhance the cytotoxic activity of resveratrol. Therefore, a series of methoxylated analogues of resveratrol were simulated to enhance the antitumor activity of resveratrol.

In addition to substitution with methoxy groups at specific positions, the cis-form of the stilbene scaffold generally showed increased cytotoxic activity over the corresponding trans-isomers. Pterostilbene, for example, is a dimethylated analogue of resveratrol. The substitution of hydroxy groups by methoxy groups increases the lipophilicity of pterostilbene compared to resveratrol, which leads to better bioavailability. These differences in pharmacokinetics could be the reason for the higher biological activity of pterostilbene compared to the parent compound resveratrol. The significant modification consists of a substitution of the hydroxyl groups of resveratrol by methoxy groups to enhance the cytotoxic activity.

Through a structure relationship analysis of resveratrol with the existing SAR analyses of past publications, we were able to find out through an additional Target Screening that the substitution of the hydroxyl groups with electron-withdrawing oxygen atoms and double-bonded alkyl residues could achieve a cytotoxic stronger effect against tumor cells and the polarization of monocytes and macrophages into an M1-like anti-tumoral phenotype. The double-bonded alkyl residues increase the lipophilicity, which increases the bioavailability of the active substance. A more methoxylated structure is expected to increase anti-tumour activity, which is why Alkylresidues are added to achieve an even stronger effect with the terminal groups compared to methylation.

The following tumor models and cell lines have been used to proof the anti-tumoral properties of the derivatives of the invention:

Diagram A: HepG2 (human liver cancer cells),
Diagram B: HUVEC (human umbilical vein endothelial cells,
Diagram C: PaCA-2 (human pancreatic cancer cells),
Diagram D: MDA-MB-231 (highly aggressive, invasive and poorly differentiated triple-negative human breast cancer cells),
Diagram E: MCF-7 (human breast cancer cells),
Diagram F: T24 (human urinary bladder cancer cells),
Diagram G: AsPc-1 (human pancreatic tumor cells) and
Diagram H: Panc-1 (human pancreatic cancer cells).

In the examples, the resveratrol derivative MLB007R1 was applied at concentrations of 0 μg/ml, 10 μg/ml, 20 μg/ml, 40 μg/ml and 60 μg/ml. As a control, an underivatized agent was used at a concentration of 60 μg/ml. The dead cells were measured in percentage after 0, 12, 24 and 36 hours.

The results show 70-85% cell death in all tumor models after 12h, 85-92% cell death in all tumor models after 24 hours and approximately 96% cell death in all tumor models after 36h at a concentration of 60 μg/ml resveratrol derivative.

In all tumor models the concentration of 60 μg/ml resveratrol derivative shows the highest percentage of dead tumor cells after 12, 24 and 36 hours. The effect on tumor cells was significantly higher when the underivatized agent was used.

**Figure 8A-C** shows the kinetics of total cell number and viability after treatment with a resveratrol derivative on three tumor models. The tumor models utilized in these experiments are as follows:

Diagram A: MCF-7 (breast cancer cells),
Diagram B: HUVEC (human umbilical vein endothelial cells) and
Diagram C: HepG2 (human liver cancer cells).

The diagrams demonstrate the effect of MLB007R1 polarized macrophages on tumor cell growth as measured by the number of viable cells per culture, starting at approximately 200 000 cells per well.

The results show that the growth kinetics of untreated control cells show exponential growth of tumor cells. At a concentration of 60 μg/ml, MLB007R1 resulted in a dramatic decrease in the number of tumor cells after 12 hours, confirming its cytotoxic effect at this concentration when tumor cell viability is examined.

As a comparison, a similar assay was performed with the underivatized compound resveratrol on MCF-7 (human breast cancer cells) and HepG2 (human liver cancer cells), which decreased HepG2 cell number by approximately 70% after 24 hours at 60 μM concentration of resveratrol. In contrast, MLB007R1 decreased cell number by more than 90% after only 12 hours of incubation (D'Arcy et al.). Another study analyzed the apoptotic effect of resveratrol on HepG2 and also showed a lower cell viability compared to MLB007R1 (Li et al.). This data comparison shows that the active compounds significantly enhance this effect.

**Figure 9** shows the effect of the resveratrol derivative MLB007R1 on the induction of apoptosis in MCF-7 cells assessed by confocal fluorescence microscopy. Cells were treated with DMSO (A) and 20 (B), 40 (C), and 60 (D) μg/ml MLB007R1 treated macrophages for 12 hours and then stained with PI.

Diagram A shows as a control the cells after treatment with DMSO.
Diagram B shows >50% cell death after 12h treatment time when the cells were incubated in 20 μg/ml MLB007R1 treated macrophages.
Diagram C shows >65% cell death after 12h treatment time when the cells were incubated in 20 μg/ml MLB007R1 treated macrophages.
Diagram D shows >80% cell death after 12h treatment time when the cells were incubated in 60 μg/ml MLB007R1 treated macrophages.

Figure 10 shows ovarian cancer cells (cell line A2780) incubated for 12 h with MLB007R1 60pg/ml polarized macrophages (24 h pre-incubation) assesses by interference light microscopy. Diagrams A and C show as a control the cells after treatment with DMSO.

Diagrams B and D show the cells treated with 60 μg/ml MLB007R1 after 12 h.

The results exemplify that more than 80% of the ovarian cancer cells (cell line A2780) were killed by polarized macrophages (incubated in 60μg/ml MLB007R1).

## Material and Methods

The active compounds were concentrated as a mixture. Monocytes (human PBMCs) were isolated from human blood using a CD14 column, differentiated into primary M0 macrophages by M-CSF and subsequently incubated with the active compounds. Thereby an artificial microenvironment was created for the M0 macrophages, and the macrophages are further polarized into a novel anti-tumor phenotype. The repolarized macrophages were purified and reverse transcription was used to convert macrophage mRNA into cDNA to measure gene expression. As a control experiment, unpolarized macrophages were incubated with tumor cell lines for 24 h and 48 h and their gene expression were measured again. As a neutral sample, the gene expression of M0 macrophages was analyzed and measured, which showed no activity. Subsequently the macrophages were analyzed after 24 and 48 h incubation with tumor cell lines. Macrophages were incubated with the active compounds for 24 hours and subsequently incubated with tumor cell lines for 24 hours.

For macrophage polarization with the active compounds and TCM (tumor-conditioned media) and further gene expression determination, several standard series of the investigating genes were needed. The standards of each gene

were constructed first and afterwards, the primary monocytes were prepared for polarization. After polarization by the active compounds and TCMs, the RNA of macrophages was isolated, reverse transcribed to cDNA, and the gene expression profile was determined with the before constructed standards of the genes *via* RT-PCR. The small active ingredients were added as an extract or as a mixture with a selected concentration. The active ingredients were first prepared, see protocol, and then used in the respective concentration and formulation and are referred to as small molecules in the following. Synthesis, galenic formulation, concentration and active ingredient composition are in a separate document.

**From primer design to a final standard series**

**Material:**

| | |
|---|---|
| EvaGreen | Biotium, Cat.31019 |
| Nuclease - free water | PanReal AppliChem, Lot.8R014578 |
| Forward primer | See Table 1 |
| Reverse primer | See Table 1 |
| Thermocycler | BioRad FX96 Real Time System, BioRad C1000 Touch<br>CFX1 Thermal Cycler: Inv No. 1000056998 (CT003245)<br>CFX2 Thermal Cycler, Inv No. 1000054727 (CT001316) |
| Thermocycler software | BioRad CFX Manage 2.0 |

*Table 1: Primer, utilized for the experiment, corresponding to an appropriate gene, including the primer sequences.*

| Primer | General function | Primer sequence | |
|---|---|---|---|
| | | **Forward (5' → 3')** | **Reverse (3' → 5')** |
| ***ACTB*** | **Housekeeping** | TGCGTGACATTAAGGAGAAG | GTCAGGCAGCTCGTAGCTCT |
| ***VEGFA*** | **Angiogenesis** | CGCTTACTCTCACCTGCTTCTG | GGTCAACCACTCACACACACAC |
| ***HIF1A*** | | CGGGGACCGATTCACCAT | TTTCGAACGTTCAGAACTTATCTTTT |
| ***CCL2*** | **Chemokines** | TTGATGTTTTAAGTTTATCTTTCATGG | CAGGGGTAGAACTGTGGTTCA |
| ***IL8*** | | GAGAAGTTTTTGAAGAGGGCTGA | GCTTGAAGTTTCACTGGCATCT |
| ***Il12B*** | | GTGCCCTGCAGTTAGGTTCT | TGGGTCTATTCCGTTGTGTCTT |
| ***HMGCR*** | **Cholesterol biosynthesis & transport** | GCAGGACCCCTTTGCTTAGA | GCACCTCCACCAAGACCTAT |
| ***ABCA-1*** | | CATCTGGTTCTATGCCCGCT | TCTGCATTCCACCTGACAGC |
| ***LDLR*** | | CTACCCCTCGAGACAGATGGT | TCTCAGGAAGGGTTCTGGGC |
| ***MVK*** | | GGAGCCATGTTGTCAGAAGTC | TACAGCCAGTGCTACCTTGC |
| ***SREBF2*** | | GCCCTTCAAGTACCAACCCT | GGTTGTCCGCCTTTCTCCTT |
| ***MMP2*** | **Matrix remodeling** | CGTCGCCCATCATCAAGTTC | GAAGGTGTTCAGGTATTGCACTG |
| ***MMP9*** | | CTTTGAGTCCGGTGGACGAT | TCGCCAGTACTTCCCATCCT |

Primer stocks (100 $\mu M$) were prepared with TE buffer (1×, pH=8, Applichem Pancreac, Lot.40017591).

Further tested gene expressions are: CCR2, CSF1, SIRPα, LILRB1, LCN, CD36, CD163, PD-1, ARG-1, FGF2, TGFβ, MMP9, MMP3, HIF1α, VEGFA, IL4, IL6, EGFR; IL8, STAT3, IL10, PI3Kγ, CXCL1, CXCL1, CXCR4, CXCL9, MCP-1, STAT1, IL12, LDLR, G-6-P, NKκB, TLR4, IRF3, IRF1, TNFα, $CD8^+$, $CD4^+$, IFNγ, IL13, (RNI & ROI), bFGF, Her2, GLUT-1 and more.

**Experiment:**

Primers were designed *via* NCBI nucleotide (www.ncbi.nlm.nih.gov/nucleotide) and the Primer Design Oligo tool *via* Eurofins, see Table 1. The PCR product size was limited from 70 bp to 130 bp with an optimal $T_M$ of 60°C. Before starting the RT-PCR the pipettes (Thermo Scientific Finnpipette, Version 1.8.26.72, Version 5.0.29.66 and Gilson Pipetman) were radiated by UV (Minuvis Desaga Heidelberg, No.80955) at the wavelengths 254 nm and 366 nm. The gloves were treated with DNA Away (Molecular Bio Products, Lot.18230235), and the working place was cleaned by disinfection. The primer pair, nuclease - free water, EvaGreen, and the samples were thawed on ice, vortexed (Vortex Genie 2, Scientific Industries, G-560 E, Ser.2-12073), and further centrifuged (Labnet Intern, C1301B). The water and the 96-well-plates were UV sterilized for 20 min. The mastermix consisted of primers, EvaGreen and nuclease - free water, see Table 2. Each reaction had a final volume of 20 *µl,* Table 2. For each experiment, a no template control and a no - RT-control was added, and the EvaGreen protocol was applied, see Table 3.

*Table 2: Composition of the utilized mastermix*

| **Ingredients** | **Volume** |
|---|---|
| **EvaGreen** | 4.0 µl |
| **Primer (100 nm/µl)** | 0.05 µl |
| **Water** | 12.9 µl |
| **Template** | 3.0 µl |

*Table 3: Thermocycler protocol for EvaGreen*

| | | | | |
|---|---|---|---|---|
| **1** | **Initial Denaturation** | | 95°C | 15 min |
| **2** | **Denaturation** | **40 x** | 95°C | 15 sec |
| **3** | **Annealing** | | 60°C | 20 sec |
| **4** | **Extension** | | 72°C | 20 sec |
| **5** | **Plate read and melting curve** | | 65 - 95°C (0.5°C steps) | 5 sec |
| **6** | **Hold** | | 4° | Ad infinitum |

**Agarose gel electrophoresis**

Performing a PCR with predicted templates and the appropriate primers does not ensure a proper PCR product. Therefore, a gel was prepared, and the PCR product was separated through migration. This method was used for the separation of DNA fragments according to different molecular sizes. It uses an electric field, and due to the negatively charged phosphate backbones of DNA, fragments migrate to the positive anode through the agarose gel. The size of the pores can be modified by changing the concentration of agarose. Ethidium bromide was added to the sample in the gel, which intercalates into nucleic acids, thereby altering its excitation spectrum and thus increasing the intensity of fluorescence (Lee et al. 2012). Therefore, the DNA could be visualized in the gel using a UV transilluminator.

**Material:**

| | |
|---|---|
| Agarose powder | |
| TBE - buffer | See below |
| Ethidium bromide (10 mg/ml) | Sigma, E1510 |
| Marker | GeneRuler DNA Ladder (50 bp) |
| Loading Dye | Gel Loading Dye Purple 6x #B70245, Biolabs, Lot.0441607 |
| Microwave | Sharp, R202 |
| Gel chamber | Mupid One Advance, Nr.071224 BioStep |

(continued)

| | |
|---|---|
| Agarose powder | |
| Dark hood | BioStep DH-40/50, Inv. Nr, 1000020993 |
| Software | Argus X1, Biostep, (Version 4.1.10) |

10 x TBE buffer (TRIS - Borate - EDTA buffer):

| | | |
|---|---|---|
| Boric acid | Fluka, Cat:31146, Lot.H1440 | 35.0 g |
| TRIS base | Sigma T1503 | 108.0 g |
| $Na_2EDTA$ | Roth 4984.1 | 7.4 g |
| $ddH_2O$ | | Fill up to 1,000.00 ml |

**Experiment:**

For 1.5% agarose gels, 1.5 grams of agarose powder was filled into an Erlenmeyer flask with 100 ml TBE Buffer (1x), and put into a microwave for 3 - 4 min with 400 - 600 watt until the mixture was completely transparent. The mixture was cooled down until 60°C, and 5 $\mu l$ ethidium bromide was added. The agarose gel mixture was filled into two chambers with the corresponding comb for the wells and cured for 20 min. The chamber was filled with TBE buffer (1x), and the samples were filled into the gel pockets with loading dye to a final volume of 20 $\mu l$. The electric potential was set to 100 V for 40 min, and the software Argus X1 was utilized to take pictures of the gel.

The PCR product inside the agarose gel was cleaned up with the PCR CleanUp Gel Extraction kit (PCR - CleanUp Gel Extraction Nucleospin Gel and PCR, Macherey & Nagel, Lot. 1501/002, Ref.740609.250).

**PCR cleanup**

**Material:**

| | |
|---|---|
| Binding buffer NTI | Guanidinium thiocyanate |
| Wash buffer NT3 | 80% Ethanol |
| Elution buffer NE | 5 mM TRIS/HCl, pH 8.5 |
| NucleoSpin® Gel and PCR clean - up columns | |

**Experiment:**

The DNA fragment was sliced out of the gel with a clean scalpel under UV light, weighed (Sartorius AZ3102), and transferred into an Eppendorf tube. For 100 mg of agarose gel (< 2%), 200 $\mu l$ of the Buffer NTI was added and heated for 5 - 10 min at 50°C (Heatblock, Accublock, Digital Axon, Labnet D1200, Ser.42B09007). NTI ensured the binding of the PCR product to the silica membrane. The melting gel slice was transferred into a NucleoSpin Column in a collection tube. The tube was centrifuged (Hermle, Z233 MK-2) for 30 sec at 11,000 $\times$ g and the flow - through was discarded. 700 $\mu l$ of NT3 buffer were added twice for washing the membrane and centrifuged at the same conditions, and the silicon membrane was dried for 1 min, at 11,000 $\times$ g. The PCR product was incubated for 1 min with elution buffer and eluted with 15 $\mu l$ of NE into a fresh Eppendorf tube at 11,000 $\times$ g for 1 min.

**Ligation**

The purified PCR product was integrated into a vector (pGEM T-easy, Promega Cat. A1360, Lot.000337384). The p-GEM T-Easy cloning vector possesses a T-overhang for efficient ligation process through avoiding recircularization.

The ends are modified with 3' thymidine residues, see Figure 3.

**Material:**

| | |
|---|---|
| pGEM-T easy Vector (50 ng/μl) | |
| Control insert DNA (4 ng/μl) | |
| T4 DNA ligase | |
| 2X Rapid ligation buffer | |
| Nuclease free water | |
| PCR product | |

**Experiment:**

The different reaction agents were mixed together (Table 4) and incubated overnight at 4°C.

*Table 4: Reaction component with the amount for one ligation reaction and positive control.*

| Reaction agents | Standard reaction | Positive control |
|---|---|---|
| **2X Rapid ligation buffer** | 5 μl | 5 μl |
| **pGEM®-T easy vector (50 ng/μl)** | 1 μl | 1 μl |
| **PCR product** | See calculation | - |
| **Control insert DNA (4 ng/μl)** | - | 2 μl |
| **T4 DNA ligase** | 1 μl | 1 μl |
| **Nuclease free water** | Filled up to 10 μl | Filled up to 10 μl |

The required amount of the PCR product is dependent on different variables, see below. The molar ratio of pGEM®-T easy Vector Systems to insert DNA into the vectors is 3:1.

$$\text{m (pGEM}^{\circledR}\text{-T Easy Vector)} = 3\ \text{kb}$$

$$\text{c (pGEM}^{\circledR}\text{-T Easy Vector)} = 50\ \text{ng}/\mu l$$

$$\frac{ng\ of\ vector\ \times\ kb\ size\ of\ insert}{kb\ size\ of\ vector} \times \frac{3}{1} = ng\ of\ insert$$

The concentration of the PCR products was measured by Nanodrop (Nanodrop lite, SN1945, Thermo Scientific, Inv. No. 1000067546). The kilobase data of all required PCR products were calculated and applied to this formula.

**Transformation**

**Material:**

| | |
|---|---|
| LB - medium (sterilized) | |
| LB - agar plates with Ampicillin | |
| Ligation reaction formulation | |

(continued)

| | |
|---|---|
| Top10 competent *Escherichia coli* (*E. coli*) | |

LB - medium

| | | |
|---|---|---|
| Tryptone (from Casein) | Sigma, 95039 | 10 g |
| NaCl | VWR, 27810.295 | 5 g |
| Yeast extract | Carl Roth Art. 2904.1 | 10 g |
| Agar (for plates) | Carl Roth, Art. 5210.2 | 15 g |
| Ampicillin (for culture inoculation) | 100 mg/ml | 500 $\mu l$ |
| $ddH_2O$ | | Fill up to 1,000.00 ml |

**Experiment:**

50 $\mu l$ of competent *E. coli* bacteria were thawed on ice for 5 - 6 min and mixed with 2 $\mu l$ of the ligation reaction mixture. The *E. coli* bacteria with the vector containing the PCR products were incubated on ice for 20 min, heat - shocked at 42°C for 47 sec and cooled down again for 2 min. 950 $\mu l$ of pre - warmed LB - media were added and shook (New Brunswick Scientific, C24, Incubator shaker, Ser.500891877) for 1.5 h, 150 rpm at 37°C. The LB plates with Ampicillin were pre - warmed and treated with 100 $\mu l$ of the new culture, the plates were incubated overnight at 37°C. Five colonies were picked and inoculated in 10 ml of LB - media with Ampicillin, incubating them overnight (16 h) at 180 rpm, 37°C for further plasmid isolation.

**Plasmid isolation**

The bacterial culture was centrifuged at 2000 × g for 2 min at room temperature, and the supernatant was discarded. The plasmid isolation kit (QIAGEN, QIAPrep Spin Miniprep Kit, Cat.27106, Lot.136230794) was applied for isolating and purifying the plasmid containing the cDNA of interest.

**Material:**

| | |
|---|---|
| Buffer P1 | 50 mM TRIS·Cl, pH 8.0; 10 mM EDTA; + RNase A (2-8°C) 100 $\mu g$/ml |
| Buffer P2 | 1% Sodium dodecyl sulfate; 200 mM NaOH |
| Buffer N3 | 4.2 M Guanidine hydrochloride; 0.9 M Acetic acid |
| Buffer PB | 5 M Guanidine hydrochloride; 30% 2-Propanol |
| Buffer PE | 10 mM TRIS-HCl pH 7.5; 80% Ethanol |
| Buffer EB | 10 mM TRIS-Cl, pH 8.5 |

**Experiment:**

After discarding the supernatant, the pellet was resuspended and vortexed in 250 $\mu l$ of P1 buffer and transferred into a fresh tube. Another 250 $\mu l$ of P2 buffer were added for lysis and mixed by inverting 4 - 6 times for 1 - 2 min. 350 $\mu l$ of buffer N3 was added and immediately mixed for avoiding local precipitation. Now the mixture was centrifuged (Sigma, 3-18k Ser.140282, Art. 10290 Rotor:19776-H) at 17,500 × g for 10 min. 800 $\mu l$ of the supernatant was carefully transferred on top of a new QIAprep Spin Column and centrifuged for 30 - 60 sec at 17,500 × g. The flow - through was discarded after each step. 500 $\mu l$ of PB buffer were added on top of the column and again centrifuged for 30 sec as washing step. In the end, the column with the plasmid DNA in the membrane was treated with 750 $\mu l$ of PE buffer and again centrifuged, following 1 min empty centrifugation step. The purified DNA was eluted with 50 $\mu l$ elution buffer into a new fresh tube.

**Standard series**

The plasmids of the investigating genes were purified containing our DNA of interest for determination of the gene expression profile of the polarized macrophages *via* RT-PCR. Therefore, standards of the DNA (S1 to S8) were created with the following calculation:

$$\frac{Plasmid\ DNA\ concentration\ \frac{ng}{\mu l}}{660\,\frac{g}{mol} \times [Insert[kb] + Vector[kb]]} = X$$

$$X \times 10^{12} = Y\ \frac{\mu mol}{ml}$$

$$Y\ \frac{\mu mol}{ml} \times 10^{7} = Z\ \frac{attomol}{\mu l}$$

**Macrophage polarization**

The standard series of the genes to be tested were finally constructed. Afterwards, the macrophage polarization procedure started. Seven different tumor cell lines (TCL) were cultivated (see Table 5), and their conditioned media were utilized as treatment for the macrophage polarization after six days. The procedure starts with TCL Seeding.

**TCL seeding (day 1)**

**Material:**

| Cell line | Starting passage | |
|---|---|---|
| PLC/PRF/5 | # 18 | 1 million |
| A549 | # 37 | 1 million |
| HepG2 | # 15 | 2 million |
| Hep3B | # 19 | 2 million |
| PANC-1 | # 14 | 2 million |
| AsPC | # 13 | 2 million |

Culture medium:

| | |
|---|---|
| RPMI-1640 | Sigma, R0883 Lot.RNBH0704 |
| Fetal calf serum (FCS) | 10% |
| Glutamine | 2 mM |
| Penicillin G (P) | 100 U/ml |
| Streptomycin (S) | 100 $\mu g$/ml |

Phosphate buffered saline (PBS) 10x:

| | | |
|---|---|---|
| NaCl | VWR, 27810.295 | 80.0 g |
| KCl | Merck 1.04936.0500 | 2.0 g |
| $Na_2HPO_4$ | Carl Roth 4984.1 | 14.4 g |
| $KH_2PO_4$ | Grüssing, Kat.120321000 | 2.4 g |
| Bidest. water | | Filled up to 1,000 ml |

The pH of PBS was first adjusted to 7.4 (pHenomenal VWR, Ser.12101471) and then PBS was sterilized (Fedegan Autoclav spa FNR 3926E).

**Preparation:**

The TCLs were taken out of a liquid nitrogen tank and thawed in a water bath (Haake FS2, P180424) at 37°C for 1 - 2 min. Each following step was performed under sterile conditions (S2 lab). The cells were inoculated in a culture flask with pre-warmed medium and kept overnight in an incubator at 37°C. The cultures were split twice a week 1:5. Cells were washed with PBS and incubated for 3 - 5 min with Trypsin (EDTA-solution Sigma SLBZ9716) for detaching them. First, TCLs were seeded to a certain number of cells in a 75 $cm^3$ cell flask, see above, with 20 ml culture media. The cells were counted under the microscope (Axiovert 40 CFL Zeiss, No.3831000966) using a Neubauer hemocytometer (Eppendorf) and were cultivated at 37°C for three days to ensure their later confluency. Their supernatant served as later TCM.

**Isolation of primary monocytes**

Peripheral blood mononuclear cells (PBMC) were isolated from human blood, so-called buffy coats that were collected from the blood donation centre Saar-Pfalz GmbH in Saarbrücken, an affiliated company of the Klinikum Saarbrücken gGmbH. The investigation of the blood, notably the monocyte isolation, is approved and confirmed by the State Medical Board of Registration, Saarland (permission no. 173/18). PBMCs exhibit the CD14 glycoprotein that was used for isolation purposes. The buffy for isolation was never older than two days.

**Material:**

| | |
|---|---|
| PBS on ice | |
| Scalpel | |
| LEUCOSEP tubes | Greiner BIO-ONE, Lot.E19023PY |
| Lymphocyte separation media | PromoCell, Lot.442P005, No.1077 |
| Cell strainer | |
| CD14 microbeads | MACS Miltenyi Biotec, Lot.5181004603 |
| LS columns | MACS Miltenyi Biotec, Lot.5180928086 |
| Magnetic appliance | QuadroMACS™ Separator, 028751 |
| QuadroMACS separation unit | MACS Miltenyi Biotec, Lot.5150212003 |
| Human M-CSF premium grade (100 ng/$\mu m$) | MACS Miltenyi Biotec, Lot.5181023574 |
| RPMI-1640 | + Glutamin, + FCS |

Bead buffer:

| | |
|---|---|
| PBS | 50 ml |
| FCS | 250 $\mu l$ (0.5%) |

(continued)

| | |
|---|---|
| EDTA (100 mM) | 1 ml (2mM) |

**Preparation:**

The buffy coat was first opened with a scalpel, and the blood was transferred into a culture flask (175 $cm^3$) and filled with ice-cold PBS to 120 ml. Afterwards, the *separation* tubes were filled with 15 ml of the separation media and centrifuged at 400 x g for 1 min, resulting in segregation of the separation media under the membrane. The blood with PBS was filled in *separation* tubes and again centrifuged (Sigma, Rotor: 19776-H), for 30 min at 300 x g without a break to segregate the PBMCs from the erythrocytes and granulocytes, see Figure 4. The blood and its components distributed due to their density on different phases.

The plasma was discarded and the ring, containing PBMCs, was carefully transferred into a new Falcon tube that was filled with ice-cold PBS up to 15 ml for washing purpose and continued by centrifugation for 10 min at 320 x g. The supernatant was discarded, and the pellet was washed with 5 ml PBS and run through a cell strainer (100 $\mu m$). Now the cells were counted with a Neubauer chamber under the microscope. The cells were again centrifuged for 10 min at 200 x g, and the pellet was resuspended in bead buffer in a specific ratio. The monocytes were collected and bound to magnetic CD14 beads, also in a specific ratio. Each 100 million cells required 1 ml bead buffer and 20 $\mu l$ magnetic CD14 beads. The mixture of bead buffer, magnetic beads, and PBMCs were incubated for 15 min at 4°C. After incubation, the bead buffer mixture was filled with PBS up to 40 ml, then centrifuged for 10 min at 280 x g, and the supernatant was carefully discarded. The monocyte-magnetic CD14 bead pellet was resuspended in 500 $\mu l$ bead buffer per 100 million cells and purified through a 40 μm cell strainer and afterwards transferred into an LS-column that was filled with ferromagnetic spheres.

The column was never allowed to run empty. The CD14 magnetic beads, bound to the monocytes, were forced to the magnetic field and thereby remained in the LS column. After the bead buffer from the cell suspension flowed through the LS columns, the column was equilibrated with 3 ml of PBS three times. The column was removed from the magnetic device, and 5 ml of bead buffer were pushed through with an appropriate piston. The monocytes were collected in a tube. The cells were again counted and centrifuged for 10 min at 320 x g. The pellet was resuspended in RPMI-1640 medium containing FCS, glutamine and P/S. The culture medium was mixed with M-CSF (20 ng/ml) for differentiation to M0 polarized macrophages. Finally, the monocytes were seeded into a 12-well-plate with 250,000 cells per well. The monocytes differentiated to macrophages, adhered to the well surface and polarized to M0 in six days. For maintenance of the developing monocytes, the medium was changed every two days. After six days, the macrophage polarization with tumor cell-conditioned media can be started.

**Polarization (day 4/day 6)**

The before seeded TCLs were confluent and the TCM treatment (day 6) or polarization started. Therefore, the supernatant (20 ml) of the seeded TCLs was sterile filtered (0.22 $\mu m$), and 1 ml was applied to the primary macrophages in the wells. The primary macrophages were polarized for only 24 h by the compounds and the TCMs, afterwards, their RNA was isolated and reverse transcribed.

RNA isolation (day 7/8)

**Material:**

| | |
|---|---|
| Lysis/-binding buffer | 4.5 M guanidine-HCl, 50 mM TRIS-HCl, 30% Triton X-100 (w/v), pH 6.6 (25°C) |
| DNase incubation buffer | 1 M NaCl, 20 mM TRIS-HCl and 10 mM MgCl2, pH 7.0 (25°C) |
| DNAse deoxyribonuclease I from bovine pancreas | 50,000 U/ml; Sigma, D5025-150 KU, 126 K7675 (4°C) |
| Wash buffer I | 5 M guanidine hydrochloride and 20 mM TRIS-HCl, pH 6.6 (25° C); final concentration after addition of 20 ml absolute ethanol |
| Wash buffer II | 20 mM NaCl, 2 mM TRIS-HCl, pH 7.5 (25°C); final concentration after addition of 40 ml absolute ethanol |
| Elution buffer | Water, PCR grade |

(continued)

| | |
|---|---|
| PBS | See above |

**Experiment:**

The RNA Iso kit (High Pure RNA Iso kit, Roche D5025, Cat.11 828 665 001) was performed. The TCM and compounds were first removed, and the macrophages were washed with 200 *µl* PBS and lysed with 700 *µl* Lysis/-Binding buffer and mixed by pipetting. The lysate was transferred to the provided high pure filter column and centrifuged at 13,000 g for 1 min, and the flowthrough was discarded. 100 *µl* of the DNAse and incubation buffer (1:10) mixture was added to the column and incubated for 15 min at room temperature. Afterwards 500 *µl* and 200 *µl* of Wash Buffer (WB) I were pipetted onto the column and centrifuged for 15 sec at 8000 x g and 2 min at 8000 x g and finally for 1 min empty centrifugation step. The purified RNA of the macrophages was eluted with 50 *µl* of Elution Buffer at 8000 x g for 1 min into a fresh chloroform treated autoclaved tube, and the RNA concentration was measured with Nanodrop.

**Reverse transcription**

For gene expression determination *via* RT-PCR the cDNA was needed. Reverse transcription converts the macrophage RNA to cDNA with the High Capacity cDNA Reverse Transcription kit (Applied Biosystems, Ref.4368813, Lot.00748674). RNase inhibitor (RNase OUT, Invitrogen, Ref.10777019, Lot.1734457) was utilized for inhibition of ribonucleases.

**Material:**

| **Agents** | **Single reaction** |
|---|---|
| 10X RT buffer | 2.0 *µl* |
| 10X RT random primer | 2.0 *µl* |
| 25X dNTP Mix (100 mM) | 0.8 *µl* |
| MultiScribe Reverse Transcriptase (50 U/µL) | 1.0 *µl* |
| RNase inhibitor (40 U/ *µl*) | 1.0 *µl* |
| Nuclease - free water | 3.2 *µl* |

**Experiment:**

The above-mentioned agents were thawed on ice and mixed together resulting in the mastermix. The mastermix was distributed on PCR tubes with 10 *µl*/well and 10 *µl* of macrophage RNA (20 ng/*µl*) to get a final volume of 20 *µl*. The samples in the wells were mixed properly, and the plate was sealed, centrifuged, and set up into a thermocycler (BioRad T100 Inv. 1000060210) with the following protocol, see Table 6. The resulting cDNA was diluted 1:10 with TE buffer and further used finally for gene expression determination *via* RT-PCR.

*Table 6: PCR protocol for reverse transcription consisting of four steps.*

| **Initiation** | **Phase 1** | **Phase 2** | **Phase 3** | **Phase 4** |
|---|---|---|---|---|
| **Temperature** | 25°C | 37°C | 85°C | 4°C |
| **Time** | 10 min | 120 min | 5 min | Hold |

**Quantitative real time RT-PCR**

The quantitative real time PCR is a method quantifying DNA. New synthesized double - stranded (ds) DNA sequences consist of a new and the old strand and serve as a template for the next PCR cycles, and the cDNA target segment is multiplied exponentially (Kück U., 2015). The PCR is subdivided into three steps 1. denaturation for separating the dsDNA into single strands, 2. annealing for linking the primer to complementary parts on the template and 3. extension,

which incorporates the DNA - polymerase elongation by adding nucleotides (dNTPs). The quantification part is achieved through real time fluorescence measurements. For analysis of gene expression, RT-PCR is an often-utilized application and measures the abundance of a certain gene transcript relative to a housekeeping gene through normalization. The house - keeping gene used in this work was *Beta - Actin (ACTB).* The RT-PCR can only measure transcripts with reverse transcription. The analysis provides a statement to the expression change of one gene at a certain moment at specific conditions in time and space (Holzapfel and Wickert, 2007).

**Fluorescence dye EvaGreen**

The PCR mixture contains the dye, EvaGreen, which binds to dsDNA. The correct quantification determination *via* fluorescence is performed during the exponential stage of amplification due to optimal reaction conditions. The fluorescence dye intercalates into the minor groove binder of dsDNA. Consequently, the increasing cycle number leads to a higher fluorescence signal due to an exponential rise of the dsDNA. For relative quantification, a normalization as a reference (*ACTB*) is needed. The fluorescence dye used for this experiment was EvaGreen that provides accurate amplification and melting point analysis with its "release - on - demand" mechanism, see Figure 6.

The dye consists of two monomers that are connected by a spacer. If there is no DNA present, the dye structure exists as an inactive loop. In the presence of DNA, the loop transforms into a random conformation binding to the DNA forming a dye - DNA complex and sending out detectable fluorescence signals.

**Ct value and $R^2$**

The Ct value, also called the threshold cycle, describes the initial exponential growth of the fluorescence signal. The more amplicons emerge, the more fluorescence signals are detected, and the threshold value is passed even faster (Holzapfel and Wickert 2007). The Ct value is influenced by the efficiency and the cDNA concentration. At a lower cDNA concentration, the Ct value is increased, and vice versa. The cDNA (1:10 standard dilution) amplified under a reduced efficiency, could cause a standard curve with a deviated slope compared to the dilution, amplified under a high - efficiency.

*Table 7: cDNA standard series in attomol/$\mu$l.*

| **S1** | **S2** | **S3** | **S4** | **S5** | **S6** | **S7** | **S8** | **NTC** |
|---|---|---|---|---|---|---|---|---|
| 20,000 | 2000 | 200 | 20 | 2 | 0.2 | 0.02 | 0.002 | 0 |

After adding the mastermix and the cDNA dilution sample (Table 7) into the 96-well-plate (96 Fast PCR-Platte, Halbrand, Sarstedt A6, Ref.72.1981.202) the plate was sealed with a special foil (Klebefolie, optisch klar, Sarstedt, Lot.202010DB), and centrifuged (Peqlab, Perfect Spin P, Inv.100055542). The plate was inserted into the thermocycler, and the RT-PCR program mentioned above was applied, Table 3. Finally, the expression profile of the primary polarized by different TCMs and the Small Molecules was measured and can be further examined.

**Cell Viability Assay**

Tumor cell lines HUVEC (human umbilical vein endothelial cells), paCA-2 (human pancreatic cancer cells), MDA-MB-231 (highly aggressive, invasive and poorly differentiated triple-negative breast cancer cells), HepG2 (human liver cancer cells), MCF-7 (breast cancer cells), T24 (human urinary bladder cancer cells), AsPc-1 (human pancreatic tumor cells), Panc-1 (human pancreatic cancer cells) were seeded in 96-well plates (1x106 cells/well) and divided into two groups: the untreated control group and the active compound resveratrol derivative treated group. Cells in the normal group were incubated under normal growth conditions. In the resveratrol derivative (MLB007R1) treated group, the cells were pre-incubated with differentiated and polarized macrophages (5x105 cells/well) for 24 hours with different final concentrations of the resveratrol derivative.

Wells were then washed three times with phosphate buffered saline (PBS) and incubated again for 4 hours with 180 ml RPMI 1640 containing 10% fetal bovine serum. The medium was then replaced with fresh medium serially incubated with different concentrations of the lead compound and incubated with the macrophages, and the plates were incubated for 12, 24, and 36 hours. The medium containing the resveratrol derivative polarized macrophages was replaced every 12 h with freshly prepared medium. Wells were then washed three times with PBS and incubated again for 4 h, adding 180 mL of RPMI 1640 and 20 mL of MTT solution (5 mg/mL). After removal of the culture medium, 150 mL of DMSO was added to dissolve the precipitate, and the absorbance at 570 nm of the resulting solutions was measured using a CODA Automated EIA Analyzer (Bio-Rad Laboratories, Hercules, CA, USA).

The cytotoxicity of the resveratrol derivative compound was evaluated on all cancer cell lines using the MTT

method.

### The determination of cell growth and viability

Cell growth of an untreated and resveratrol derivative (MLB007R1) treated cell culture was compared. Tumor cell lines (MCF-7 (breast cancer cells), HUVEC (human umbilical vein endothelial cells) and HepG2 (human liver cancer cells)) were cultured in RPMI 1640 cell medium and exposed to 60 μg/ml resveratrol derivative polarized macrophages (10 000/ well). Cell growth, cell cycle disruption and apoptosis were assessed using the trypan blue exclusion method.
Cells were seeded in 24-well tissue culture plates (Falcon) at a concentration of 1×105 cells/ml and allowed to adhere overnight. Cells were incubated with MLB007R1 derivative and polarized macrophages at final concentrations of 60 μg/ml (3 wells for each treatment) or with DMSO alone as control. Plates were incubated at 37°C in an atmosphere containing 5% CO2 for 12, 24, and 36 hours. Cell growth and viability were assessed every 12 hours. Trypsinized cells were manually counted using a hemocytometer, and cell viability was determined using the trypan blue dye exclusion assay.
Initially, cell number was measured after incubation to 200 000 cells per culture.
The kinetics of cell growth on human MCF-7 breast cancer cells were cultured and measured for 12, 24 and 36 hours in culture medium containing the indicated drug-polarized macrophages. The control groups were treated with DMSO only. Tumor cell viability and cell growth were determined using the trypan blue assay and cell count, respectively. All measurements were performed with three different cultures in triplicate.

### Flow cytometric analysis

For apoptosis and cell cycle analysis, both floating and adherent MCF-7 cells that had been detached by trypsinization were washed in PBS and fixed with 50% acetone: methanol 1:4 in PBS at 4°C for at least 20 h. The cells were then rinsed in PBS. After washing twice in PBS, cells (1x106/ml) were suspended in PBS containing 50 μg/ml propidium iodide (PI) and 100 KU/ml RNase A and incubated for 30 min in the dark at room temperature. DNA content per cell was assessed by PI fluorescence measured on a linear scale using a FACScan flow cytometer with an argon ion laser at a wavelength of 488 nm. Data acquisition of 10,000 cells/sample was controlled using forward and side light scatter to exclude cell debris and aggregates. All data were recorded using CellQuest software (Becton-Dickinson). Cell cycle analysis was performed using ModFit LT software (Becton-Dickinson). Apoptotic cells were determined by their hypochromic sub-G1 staining profiles.
To confirm the flow cytometry results, the induction of apoptotic cell death by our MLB007R1 compound was examined by confocal fluorescence microscopy. MCF-7 tumor cells were treated with the compound-polarized macrophages (10 000/well) at concentrations of 0, 20, 40, and 60 μg/ml for 12 hours. Induction of apoptotic cell death was accompanied by characteristic ultrastructural changes, such as chromatin condensation, nuclear fragmentation, and collapse of the cell into membrane-bound apoptotic bodies.
The results confirmed that MLB007R1 at the concentration of 40 (C) and 60 (D) μg/ml increased the number of apoptotic cells. No to little apoptosis induction was observed at the lower concentration (20 μg/ml -B) of MLB007R1 (B) and in the untreated control (A). The original magnification of the confocal photomicrographs was x40.
Confocal laser scanning microscopy (CLSM). Analysis of apoptosis-like morphological changes was performed on cells grown on coverslips and fixed with 4% paraformaldehyde in PBS (pH 7.4) for 10 minutes at room temperature (RT). After washing in PBS, the samples were stained for nuclear labeling with 2 μg/ml PI and 0.1 mg/ml RNase for 5 minutes at RT. Coverslips were mounted on glass slides in the presence of Glycerol/PBS at a ratio of 4:1 and observed with a LEICA TCS 4D confocal laser scanning microscope equipped with an argon/cripton laser and oil immersion objectives. The excitation and emission wavelengths were 488 and 590 nm, respectively. The images were recorded in pseudocolor.
The present study demonstrates that treatment of the breast cancer cell line MCF-7 with MLB007R1 can reduce the number of viable cells by preventing exponential growth and inducing apoptosis and cell cycle disruption. The biological effects of MLB007R1 on cell growth and apoptosis, as evidenced by cytofluorimetric analysis and confocal fluorescence microscopy observations, appear to be concentration- and time-dependent.

### Analysis of viability and apoptosis of ovarian cancer cells (A2780)

The present study explores the response of ovarian cancer cells to MLB007R1. MLB007R1 macrophages inhibited growth and induced death in human ovarian cancer cell lines. In a further course, A2780 cells were co-incubated with MLB007R1 derivative polarized macrophages and treated. MLB007R1 induces cell death and inhibits ovarian cancer cell growth. Treatment of A2780 cells with MLB007R1 induced cell death after 12 hours. Proliferation of A2780 cultures treated with MLB007R1 was analyzed over 12 hours of continuous treatment. No further growth occurred during this time.
Cells were plated out and 60 μg/ml MLB007R1 treated macrophages were added to the culture medium 12 h after

plating out (24 h preincubation period). The percentage of apoptotic cells was determined at the indicated time points by propidium iodide (PI) staining on semipermeabilized cells as previously described. In this assay, apoptotic cells are identified by hypodiploid DNA content resulting from DNA fragmentation. To assess viability, intact cells were harvested, stained with PI, and analyzed for their plasma membrane permeability to PI by flow cytometry. Cell number was determined by interference light microscopy. The ovarian cancer cells (A2780) were incubated with 60μg/ml MLB007R1 concentration treated macrophages. This assay was performed twice.

**Statistics**

The experiments, including primary human macrophages were performed with n = 3, triplicates from three donors. The mean value of the results is displayed with error bars in the graphs, provided with the "standard error of the mean" (SEM) of the average value. The normal distribution was tested with the Shapiro - Wilk test and the Kolmogorov - Smirnov test. For data sets that were not normally distributed, the Mann - Whitney test was performed. In case of a normal distribution, the ANOVA test was performed.

The statistical significance was evaluated by following terms:
$p < 0.05$ (*), $p < 0.01$ (**), or $p < 0.001$ (***). The significance tests were performed with the Origin software (OriginPro 2019).

**References**

Biswas, S. K., Allavena, P., & Mantovani, A. (2013). Tumor-associated macrophages: functional diversity, clinical significance, and open questions. Seminars in Immunopathology, 35(5), 585-600. doi:10.1007/s00281-013-0367-7

Cassol, E., Cassetta, L., Rizzi, C., Alfano, M., & Poli, G. (2009). "M1 and M2a Polarization of Human Monocyte-Derived Macrophages Inhibits HIV-1 Replication by Distinct Mechanisms." The Journal of Immunology, 182(10), 6237-6246. doi:10.4049/jimmunol.0803447

Cook, J., & Hagemann, T. (2013). Tumour-associated macrophages and cancer. Current Opinion in Pharmacology, 13(4), 595-601. doi:10.1016/j.coph.2013.05.017

D'Arcy, MS., Pike CVS., Coussons PJ. (2021). A novel combined resveratrol/berberine phytochemotheraputic using the HePG2 cell line as a model for the treatment of hepatocarcinoma. Cell Biol Int. 2021 Dec;45(12):2499-2509. doi: 10.1002/cbin.11695.

Holzapfel, B., & Wickert, L. (2007). "Die quantitative Real-Time-PCR (qRT-PCR). Methoden und Anwendungsgebiete." Biologie in Unserer Zeit, 37(2), 120-126. doi:10.1002/biuz.200610332

Kück, PCR-Analytik. (n.d.). Springer-Lehrbuch, 221-245. (2005) PCR-Analytik. In: Kück U. (eds) Praktikum der Molekulargenetik. Springer, Berlin, Heidelberg, doi:10.1007/3-540-26469-8_4

Lee, P. Y., Costumbrado, J., Hsu, C. Y., & Kim, Y. H. (2012). "Agarose gel electrophoresis for the separation of DNA fragments. Journal of visualized experiments" JoVE, (62), 3923. doi:10.3791/3923

Lewis, C. E., & Pollard, J. W. (2006). Distinct Role of Macrophages in Different Tumor Microenvironments. Cancer Research, 66(2), 605-612. doi:10.1158/0008-5472.can-05-4005

Li, Y., Zhu, W., Li, J., Liu, M., Wei, M. (2013). Resveratrol suppresses the STAT3 signaling pathway and inhibits proliferation of high glucose-exposed HepG2 cells partly through SIRT1. Oncol Rep. 2013 Dec;30(6):2820-8. doi: 10.3892/or.2013.2748.

Loegl, J., Hiden, U., Nussbaumer, E., Schliefsteiner, C., Cvitic, S., Lang, I., Desoye, G. (2016). "Hofbauer cells of M2a, M2b and M2c polarization may regulate feto-placental angiogenesis. Reproduction", 152(5), 447-455. doi:10.1530/rep-16-0159

Martinez, F. O., & Gordon, S. (2014). The M1 and M2 paradigm of macrophage activation: time for reassessment. F1000Prime Reports, 6. doi:10.12703/p6-13

Quatromoni, Jon G und Evgeniy Eruslanov. 2012. "Tumor-Associated Macrophages: Function, Phenotype, and Link to Prognosis in Human Lung Cancer." American Journal of Translational, Research 4 (4): 376-89. doi:2012;4(4):376-389.

Sica, A., & Mantovani, A. (2012). Macrophage plasticity and polarization: in vivo veritas. Journal of Clinical Investigation, 122(3), 787-795. doi:10.1172/jci59643

Sica, A., Saccani, A., Bottazzi, B., Polentarutti, N., Vecchi, A., Damme, J. V., & Mantovani, A. (2000). "Autocrine Production of IL-10 Mediates Defective IL-12 Production and NF- B Activation in Tumor-Associated Macrophages". The Journal of Immunology, 164(2), 762-767. doi:10.4049/jimmunol.164.2.762

Solinas, G, G Germano, A Mantovani, and P Allavena. 2009. "Tumor-Associated Macrophages (TAM) as Major Players of the Cancer-Related Inflammation." Journal of Leukocyte Biology 86 (5): 1065-73. doi:10.1189/jlb.0609385.

Wynn, T. A., Chawla, A., & Pollard, J. W. (2013). Macrophage biology in development, homeostasis and disease. Nature, 496(7446), 445-455. doi:10.1038/nature12034.

## Claims

**1.** A pharmaceutical composition comprising at least one active compound having the property of polarization or differentiation of monocytes, and repolarization of macrophages into a phenotype with anti-tumor properties, the active compound being selected from the group consisting of apigenin derivative, indol-3-carbinol derivative, lupeol derivative, piperlongumin derivative, quercetin derivative, curcumin derivative and/or resveratrol derivative, or a mixture thereof, and a suitable pharmaceutical carrier.

**2.** The pharmaceutical composition according to claim 1 or 2, wherein the

1. apigenin derivative comprises the following general structure:

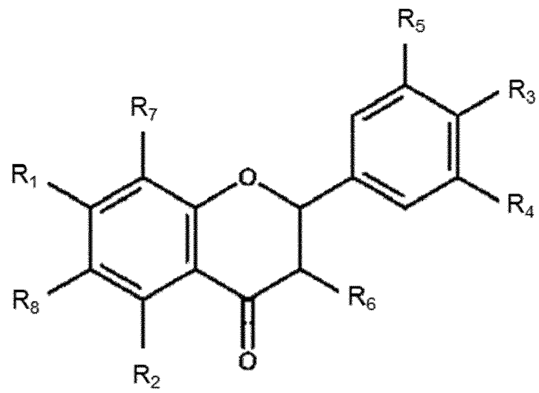

wherein

$R_1$ = - H, - OH, - O - Me,

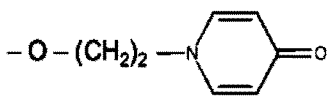

$R_2$ = - H, -OH, - O - Me, - O - CO - $CH_2$ - OH,
$R_3$ = - H, - OH, - O - Me,

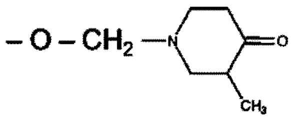 , 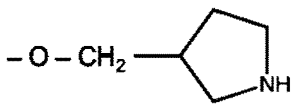 ,

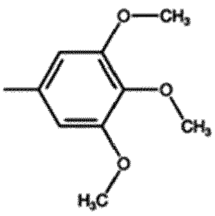 , 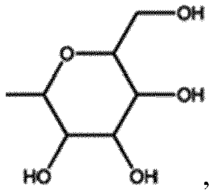 , 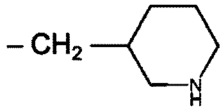

$R_4$ = - H, - O - Me
$R_5$ = - H, - O - Me
$R_6$ = - H, -OH
$R_7$ = - H
$R_8$ = - H.

2. quercetin derivative comprises the following general structure:

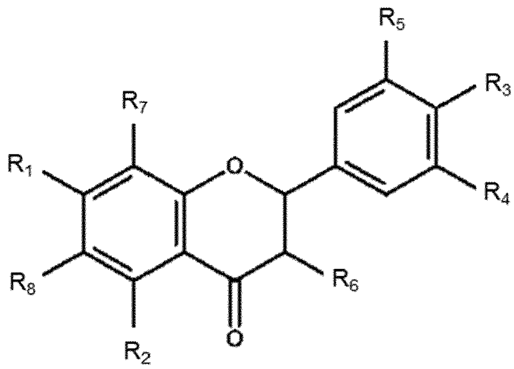

wherein

$R_1$ = - O - $CH_2$ - OH, - OH, - O - CO - $CH_2$ - CO - O - $CH_3$
$R_2$ = - O - $CH_2$ - OH, - OH, - O - CO - $CH_2$ - CO - O - $CH_3$
$R_3$ = - O - $CH_2$ - OH, - H, - O - $CH_2$ - O - CO - $(CH_2)_2$ - OH, - OH,
- O - CO - $CH_2$ - CO - O - $CH_3$,
$R_4$ = - O - $CH_2$ - OH, - O - $CH_3$, - O - $CH_2$ - O - CO - $(CH_2)_2$ - OH, - OH,

- O - $CH_2$ - O - CO - O - CH - $(CH_3)_2$,
- O - CO - $CH_2$ - CO - O - $CH_3$

$R_5$ = - OH, - O - $CH_2$ - OH, - O - $CH_3$, -H, - O - CO - $CH_2$ - CO - O - $CH_3$
$R_6$ = - H, -OH,
$R_7$ = - H, - OH,
$R_8$ = - H, - O - $CH_3$, - O - $CH_2$ - O - CO - $(CH_2)_2$ - OH, - OH,
- O - CO - $CH_2$ - CO - O - $CH_3$

3. curcumin derivative comprises the following general structure:

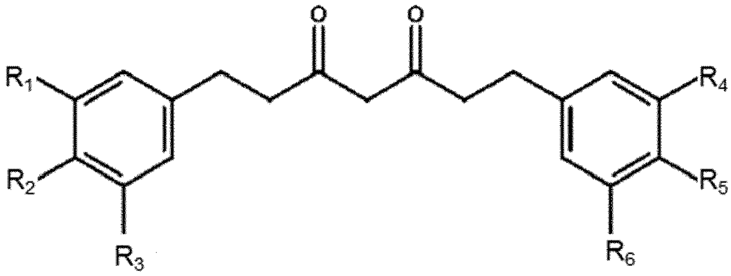

wherein

$R_1$ = - H, -OH, - O, - Me,
$R_2$ = - $CH_2$ - OH, - O, - O - CH = CH - CO - O - $CH_2$ - Me, - O - $CH_2$ - CH2 - CO - OH, - O - $CH_2$ - CH = $CH_2$,

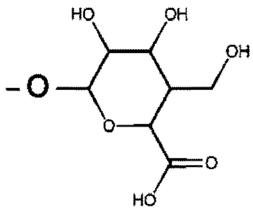

,

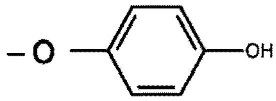

$R_3$ = - H, -OH
$R_4$ = - H, - OH, - O - Me
$R_5$ = - H, - $CH_2$ - OH, - O, - O - CH = CH - CO - O - $CH_2$ - Me, - O - $CH_2$ - $CH_2$ - CO - OH, - O - $CH_2$ - CH = $CH_2$,

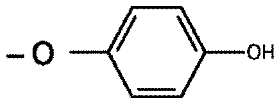

$R_6$ = - H.

4. resveratrol derivative comprises the following general structure:

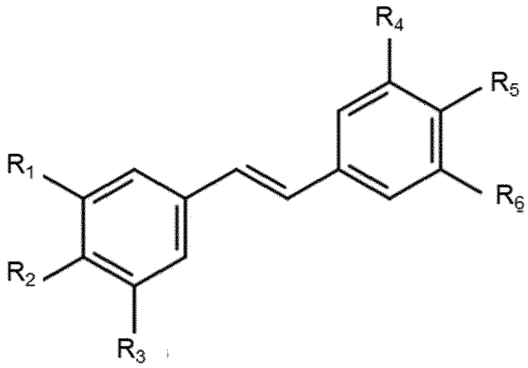

wherein

$R_1$ = - H, -OH, - O-Me, - O - $CH_2$ - CO - O - CH = $CH_2$, -O-CO-Me,

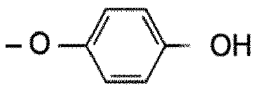

$R_2$ = - H, - OH, - O-Me,

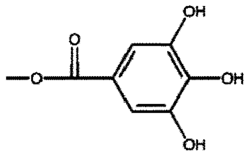

$R_3$ = - H, - OH, - O- Me, - O - CO - Me, - O - $CH_2$ - CO - O - CH = $CH_2$,

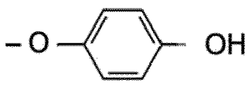 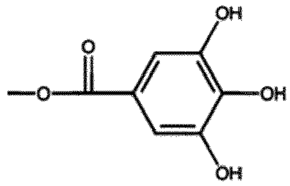

$R_4$ = - H, - OH, - O - Me, - O - CO - Me,

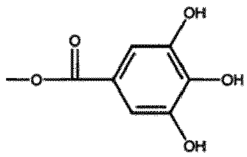

$R_5$ = - H, - OH, - O - Me, - O - CO - Me, - O - $CH_2$ - CO - O - CH = $CH_2$

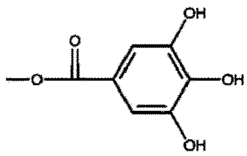

$R_6$ = - H, -OH, - O - Me, - O - CO - Me,

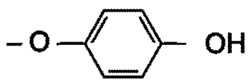 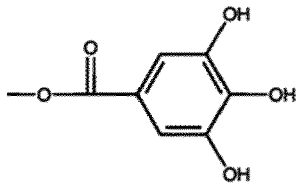

5. lupeol derivative comprises the following general structure:

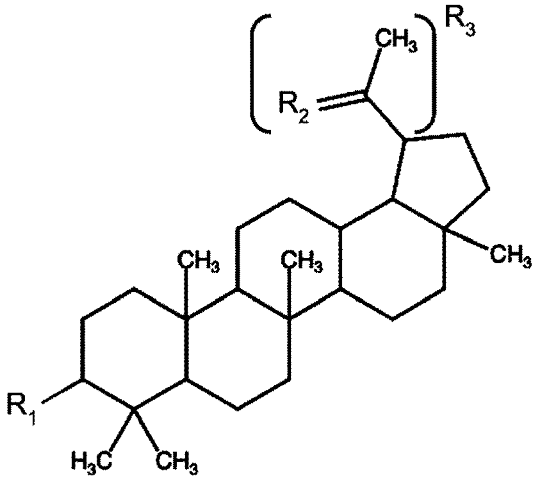

wherein

$R_1$ = - OH, - O - CO - $CH_2$ - OH, - O - CO - $CH_2$ - $CH_2$ - OH,

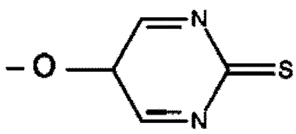

,

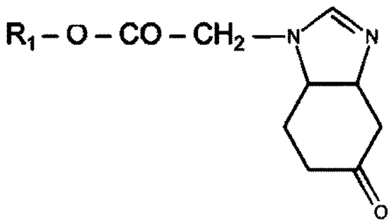

,

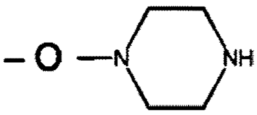

,

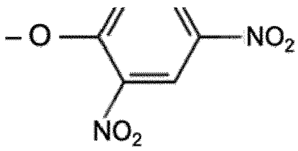

,

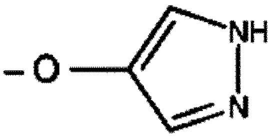

,

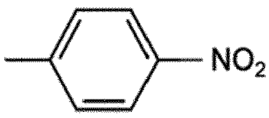

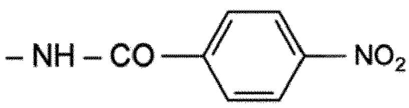

,

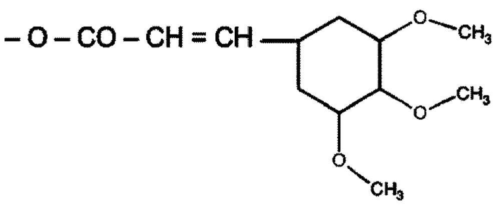

$R_2$ = - O - CO - Me,

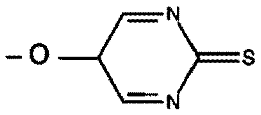 ,

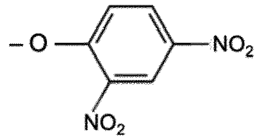 ,

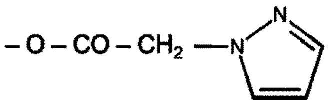 ,

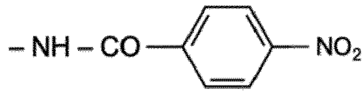 ,

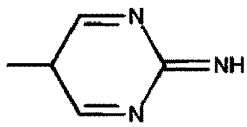

$R_3$ = 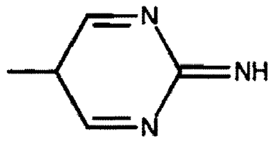

6. indol-3-carbinol derivative comprises the following general structure:

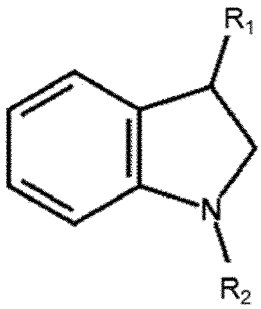

wherein

$R_1$ = - $CH_2$ - OH

$R_2$ = 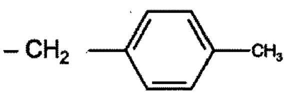 , 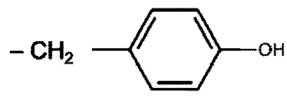 ,

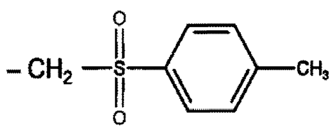 , 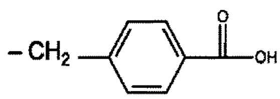 ,

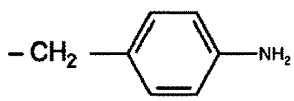

**3.** The pharmaceutical composition according to claim 1, wherein the apigenin derivative is selected from the group consisting of:

12.

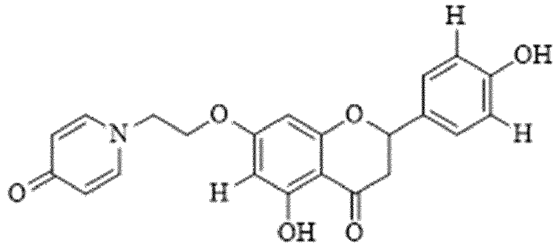

1-(2-((5-hydroxy-2-(4-hydroxyphenyl)-4-oxochroman-7-yl)oxy)ethyl)pyridin-4(1H)-one,

13.

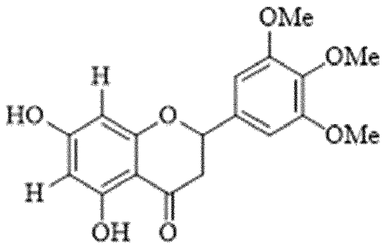

5,7-dihydroxy-2-(3,4,5-trimethoxyphenyl)chroman-4-one,

14.

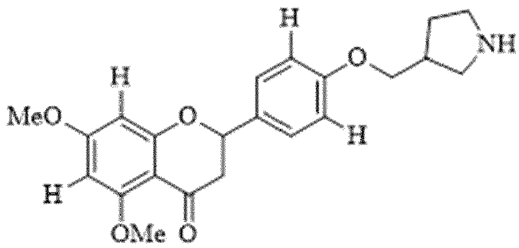

5,7-dimethoxy-2-(4-(pyrrolidin-3-ylmethoxy)phenyl)chroman-4-one,

15.

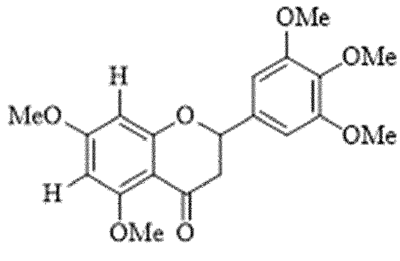

5,7-dimethoxy-2-(3,4,5-trimethoxyphenyl)chroman-4-one,

16.

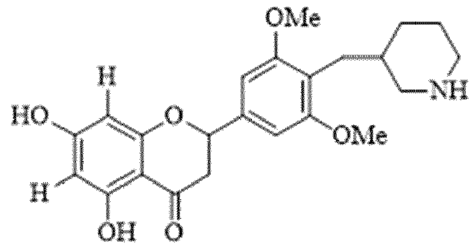

2-(3,5-dimethoxy-4-(piperidin-3-ylmethyl)phenyl)-5,7-dihydroxychroman-4-one,

17.

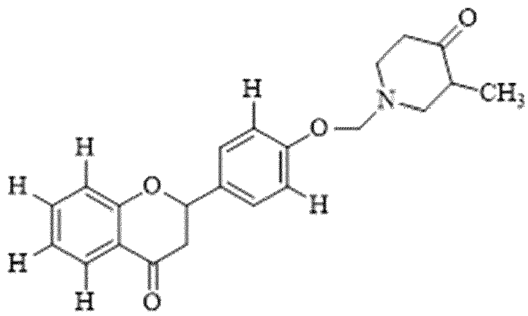

3-methyl-1-((4-(4-oxochroman-2-yl)phenoxy)methyl)piperidin-4-one,

18.

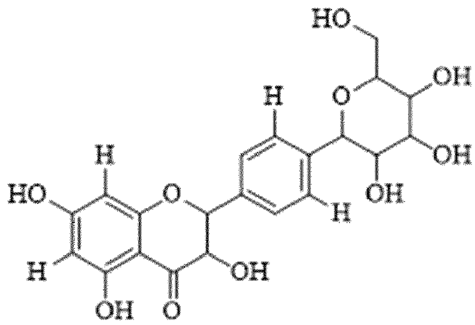

3,5,7-trihydroxy-2-(4-(3,4,5-trihydroxy-6-(hydroxymethyl)tetrahydro-2H-pyran-2-yl)phenyl)chroman-4-one,

19.

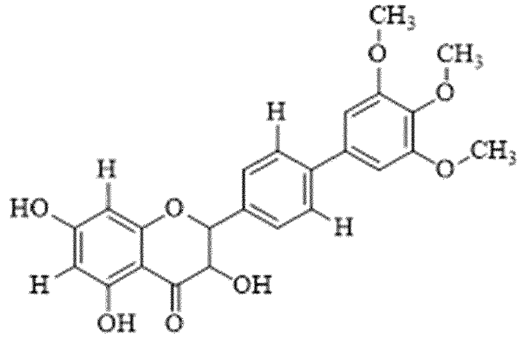

3,5,7-trihydroxy-2-(3',4',5'-trimethoxy-[1,1'-biphenyl]-4-yl)chroman-4-one,

20.

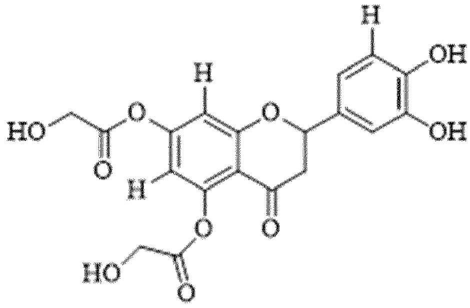

2-(3,4-dihydroxyphenyl)-4-oxochroman-5,7-diyl bis(2-hydroxyacetate),

21.

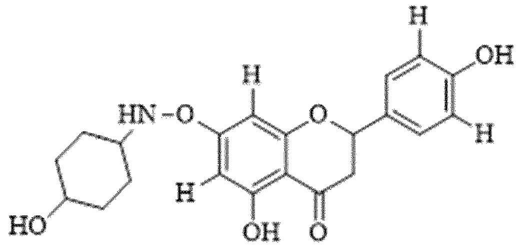

5-hydroxy-7-(((4-hydroxycyclohexyl)amino)oxy)-2-(4-hydroxyphenyl)chroman-4-one,

22.

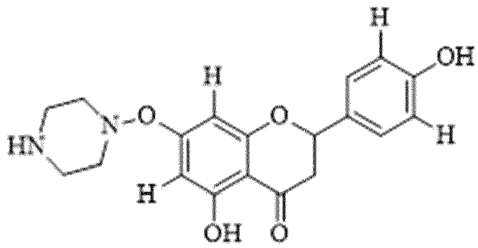

5-hydroxy-2-(4-hydroxyphenyl)-7-(piperazin-1-yloxy)chroman-4-one.

**4.** The pharmaceutical composition according to claim 1, wherein the piperlongumin derivative is selected from the group consisting of:

7.

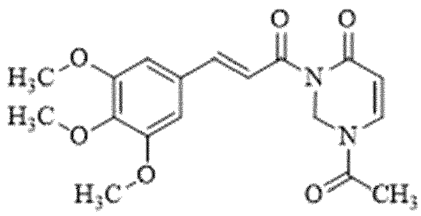

1-acetyl-3-(3-(3,4,5-trimethoxyphenyl)acryloyl)-2,3-dihydropyrimidin-4(1H)-one,

8.

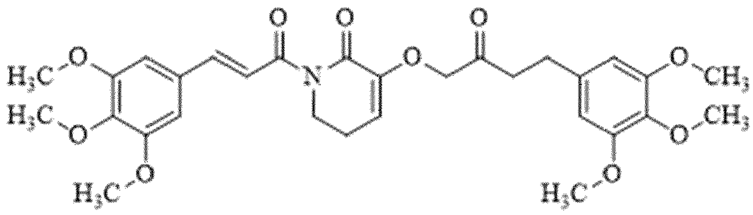

3-(2-oxo-4-(3,4,5-trimethoxyphenyl)butoxy)-1-(3-(3,4,5-trimethoxyphenyl)acryloyl)-5,6-dihydropyridin-2(1H)-one,

9.

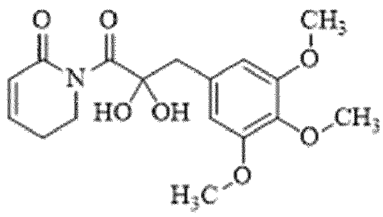

1-(2,2-dihydroxy-3-(3,4,5-trimethoxyphenyl)propanoyl)-5,6-dihydropyridin-2(1H)-one,

10.

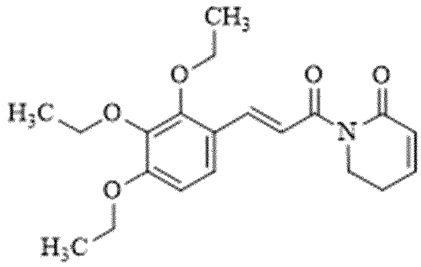

1-(3-(2,3,4-triethoxyphenyl)acryloyl)-5,6-dihydropyridin-2(1H)-one,

11.

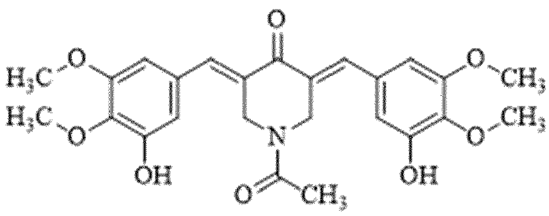

1-acetyl-3,5-bis(3-hydroxy-4,5-dimethoxybenzylidene)piperidin-4-one,

12.

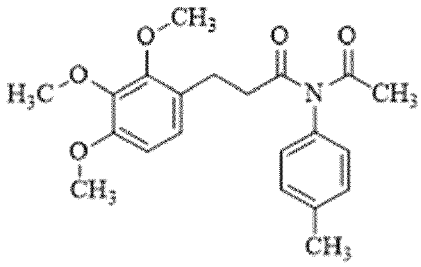

N-acetyl-N-(p-tolyl)-3-(2,3,4-trimethoxyphenyl)propenamide.

**5.** The pharmaceutical composition according to claim 1, wherein the quercetin derivative is selected from the group consisting of:

11.

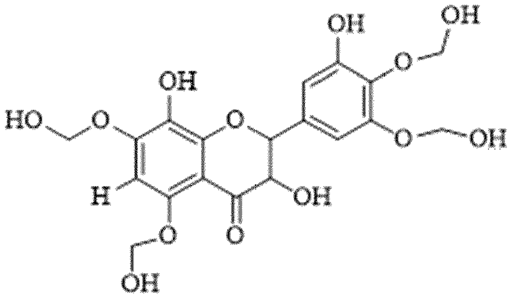

3,8-dihydroxy-2-(3-hydroxy-4,5-bis(hydroxymethoxy)phenyl)-5,7-bis(hydroxymethoxy)chroman-4-one,

12.

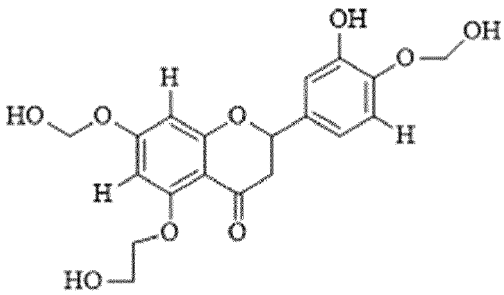

2-(3-hydroxy-4-(hydroxymethoxy)phenyl)-5-(2-hydroxyethoxy)-7-(hydroxymethoxy)chroman-4-one,

13.

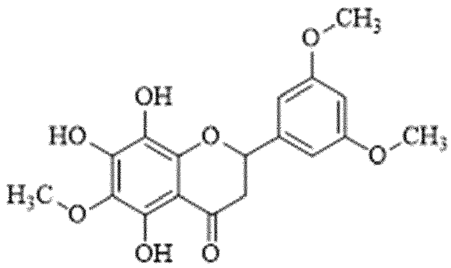

2-(3,5-dimethoxyphenyl)-5,7,8-trihydroxy-6-methoxychroman-4-one,

14.

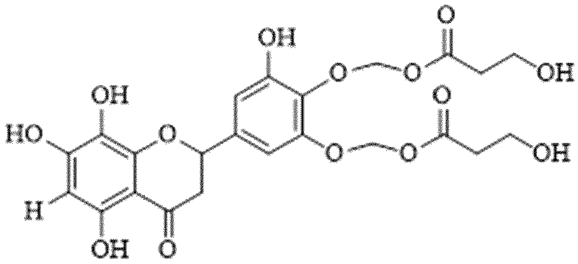

((3-hydroxy-5-(5,7,8-trihydroxy-4-oxochroman-2-yl)-1,2-phenylene)bis(oxy))bis(methylene) bis(3-hydroxypropanoate),

15.

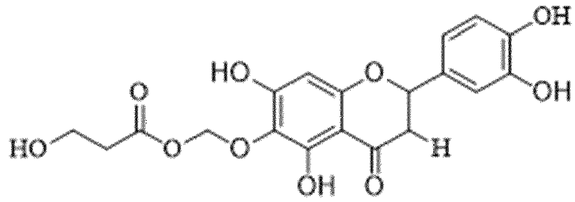

((2-(3,4-dihydroxyphenyl)-5,7-dihydroxy-4-oxochroman-6-yl)oxy)methyl 3-hydroxypropanoate,

16.

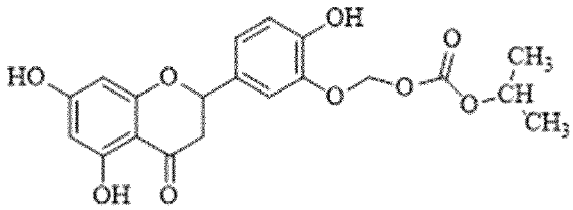

(5-(5,7-dihydroxy-4-oxochroman-2-yl)-2-hydroxyphenoxy)methyl isopropyl carbonate,

17.

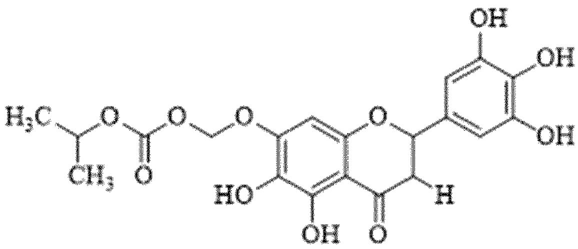

((5,6-dihydroxy-4-oxo-2-(3,4,5-trihydroxyphenyl)chroman-7-yl)oxy)methyl isopropyl carbonate,

18.

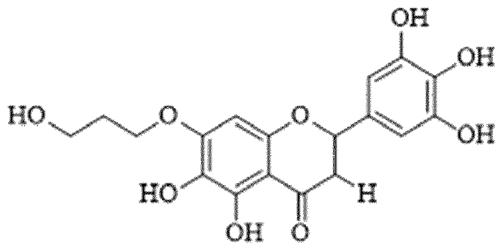

5,6-dihydroxy-7-(3-hydroxypropoxy)-2-(3,4,5-trihydroxyphenyl)chroman-4-one,

19.

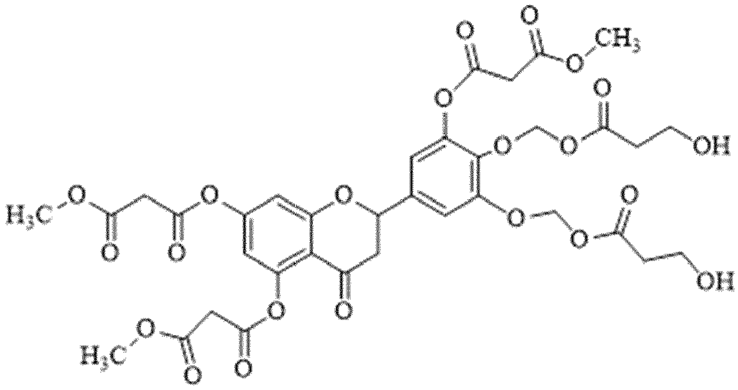

O,O'-(2-(3,4-bis(((3-hydroxypropanoyl)oxy)methoxy)-5-((3-methoxy-3-oxopropanoyl)oxy)phenyl)-4-oxochroman-5,7-diyl) dimethyl dimalonate,

20.

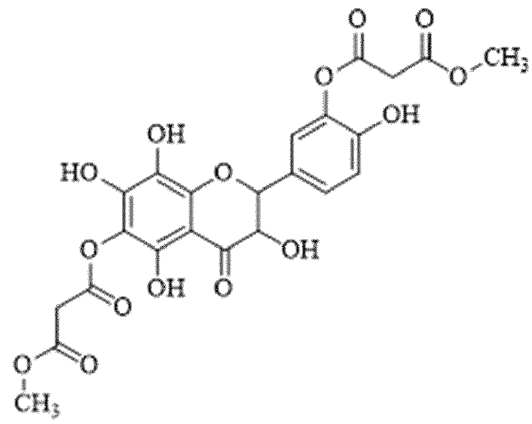

2-hydroxy-5-(3,5,7,8-tetrahydroxy-6-((3-methoxy-3-oxopropanoyl)oxy)-4-oxochroman-2-yl)phenyl methyl malonate.

**6.** The pharmaceutical composition according to claim 1, wherein the curcumin derivative is selected from the group consisting of:

8.

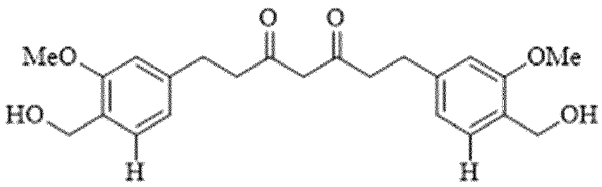

1,7-bis(4-(hydroxymethyl)-3-methoxyphenyl)heptane-3,5-dione,

9.

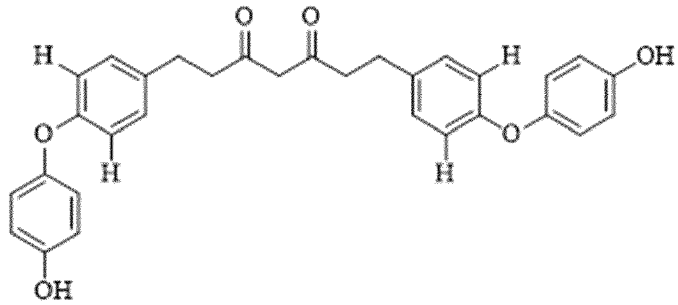

1,7-bis(4-(4-hydroxyphenoxy)phenyl)heptane-3,5-dione,

10.

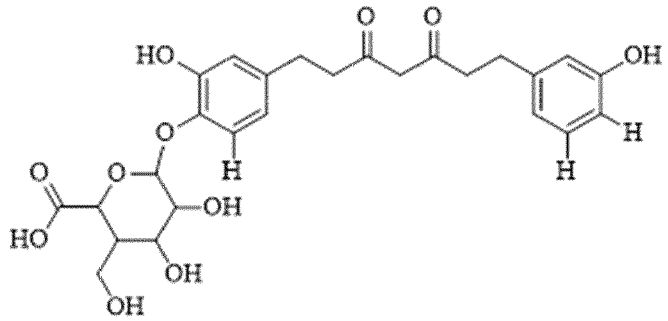

4,5-dihydroxy-6-(2-hydroxy-4-(7-(3-hydroxyphenyl)-3,5-dioxoheptyl)phenoxy)-3-(hydroxymethyl)tetrahydro-2H-pyran-2-carboxylic acid,

11.

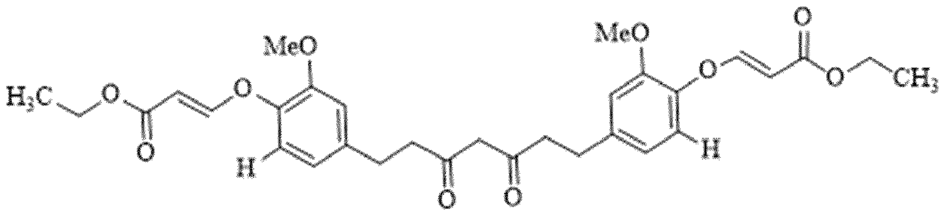

(2E,2'E)-diethyl 3,3'-(((3,5-dioxoheptane-1,7-diyl)bis(2-methoxy-4,1-phenylene))bis(oxy))diacrylate,

12.

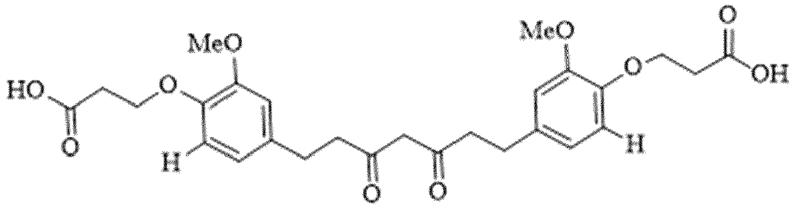

3,3'-(((3,5-dioxoheptane-1,7-diyl)bis(2-methoxy-4,1-phenylene))bis(oxy))dipropanoic acid,

13.

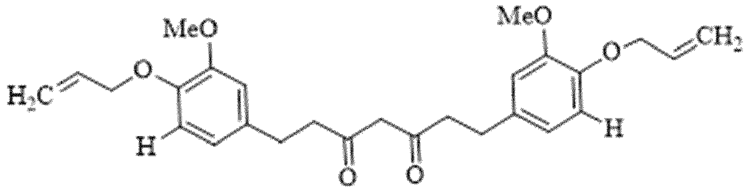

1,7-bis(4-(allyloxy)-3-methoxyphenyl)heptane-3,5-dione,

14.

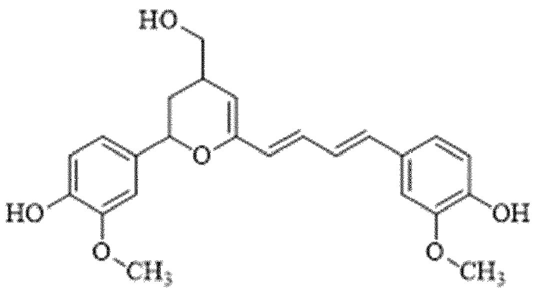

4-((1E,3E)-4-(2-(4-hydroxy-3-methoxyphenyl)-4-(hydroxymethyl)-3,4-dihydro-2H-pyran-6-yl)buta-1,3-dien-1-yl)-2-methoxyphenol.

**7.** The pharmaceutical composition according to claim 1, wherein the resveratrol derivative is selected from the group consisting of:

10.

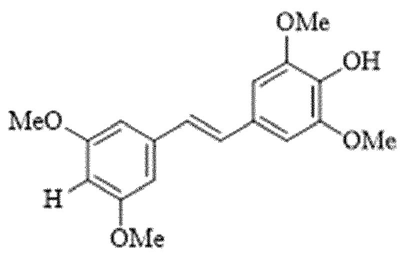

4-(3,5-dimethoxystyryl)-2,6-dimethoxyphenol,

11.

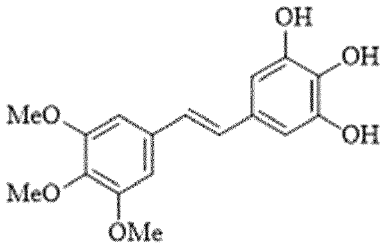

5-(3,4,5-trimethoxystyryl)benzene-1,2,3-triol,

12.

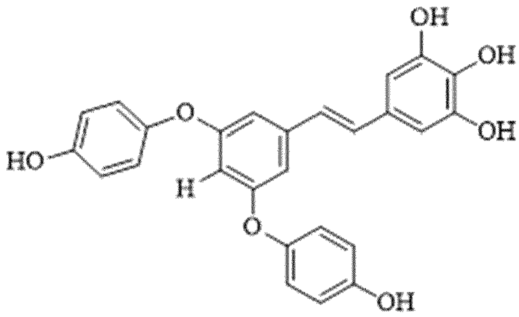

5-(3,5-bis(4-hydroxyphenoxy)styryl)benzene-1,2,3-triol,

13.

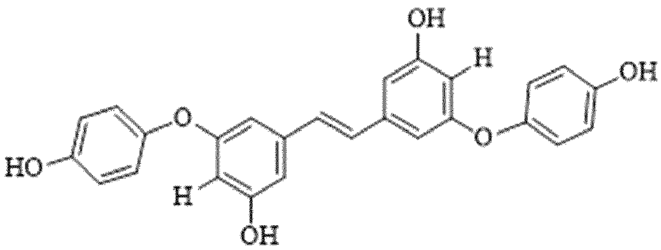

(5,5'-(ethene-1,2-diyl)bis(3-(4-hydroxyphenoxy)phenol),

14.

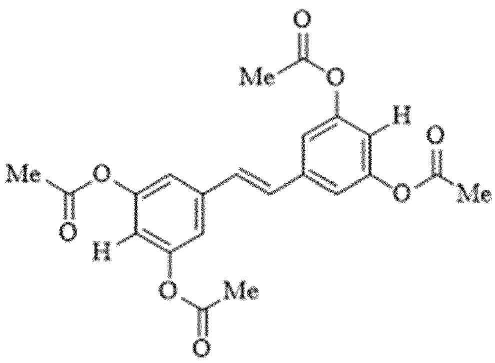

ethene-1,2-diylbis(benzene-5,3,1-triyl) tetraacetate,

15.

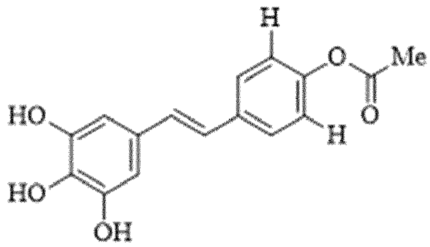

4-(3,4,5-trihydroxystyryl)phenyl acetate,

16.

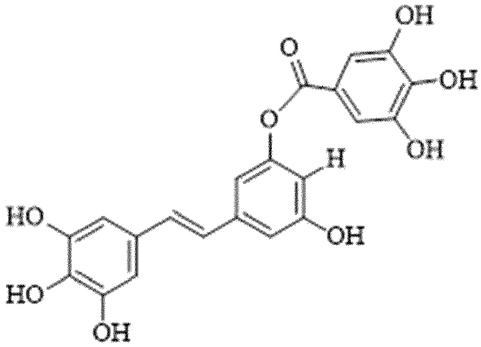

3-hydroxy-5-(3,4,5-trihydroxystyryl)phenyl 3,4,5-trihydroxybenzoate,

17.

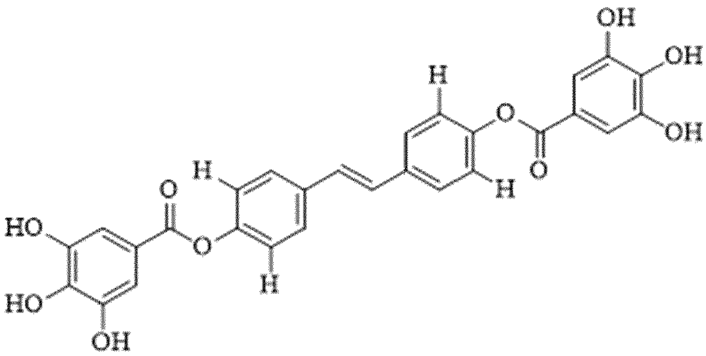

ethene-1,2-diylbis(4,1-phenylene) bis(3,4,5-trihydroxybenzoate),

18.

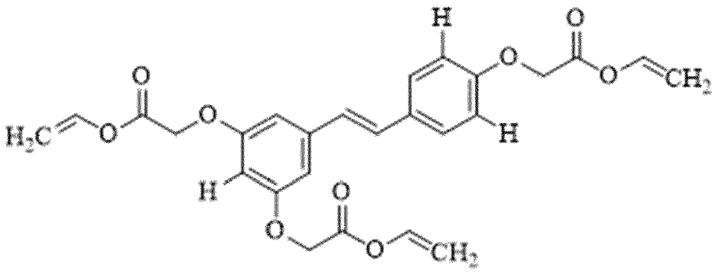

divinyl 2,2'-((5-(4-(2-oxo-2-(vinyloxy)ethoxy)styryl)-1,3-phenylene)bis(oxy))diacetate.

**8.** The pharmaceutical composition according to claim 1, wherein the lupeol derivative is selected from the group consisting of:

12.

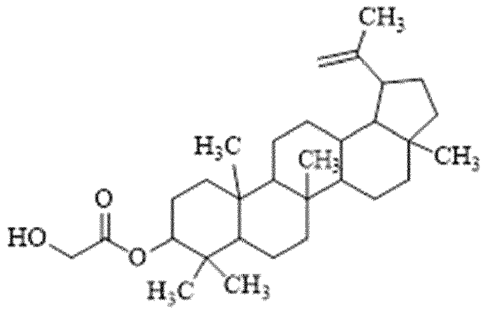

3a,5b,8,8,11a-pentamethyl-1-(prop-1-en-2-yl)icosahydro-1H-cyclopenta[a]chrysen-9-yl 2-hydroxyacetate,

13.

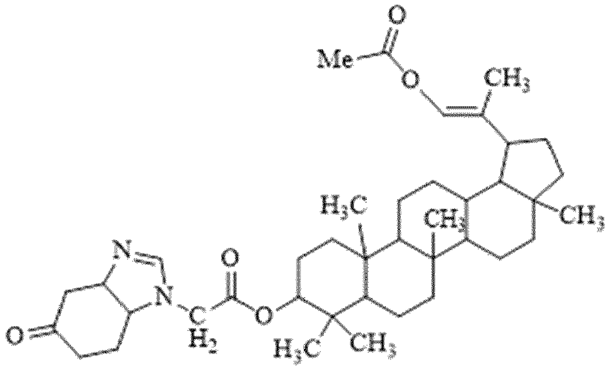

1-(1-acetoxyprop-1-en-2-yl)-3a,5b,8,8,11a-pentamethylicosahydro-1H-cyclopenta[a]chrysen-9-yl 2-(5-oxo-3a,4,5,6,7,7a-hexahydro-1H-benzo[d]imidazol-1-yl)acetate,

14.

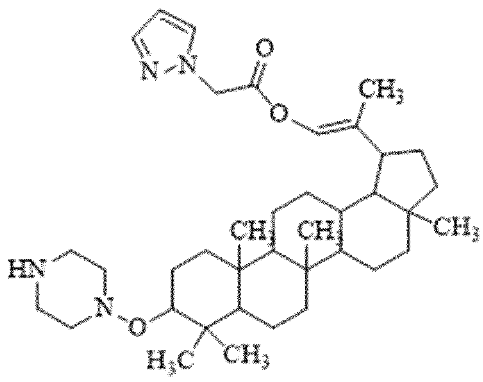

2-(3a,5b,8,8,11a-pentamethyl-9-(piperazin-1-yloxy)icosahydro-1H-cyclopenta[a]chrysen-1-yl)prop-1-en-1-yl 2-(1H-pyrazol-1-yl)acetate,

15.

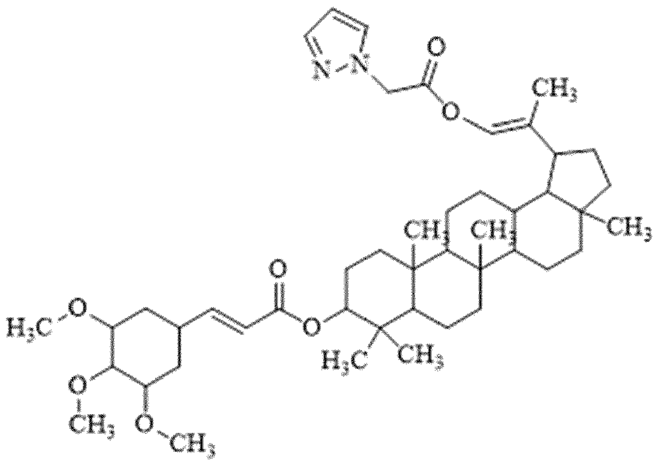

1-((E)-1-(2-(1H-pyrazol-1-yl)acetoxy)prop-1-en-2-yl)-3a,5b,8,8,11a-pentamethylicosahydro-1H-cyclopenta[a]chrysen-9-yl 3-(3,4,5-trimethoxycyclohexyl)acrylate,

16.

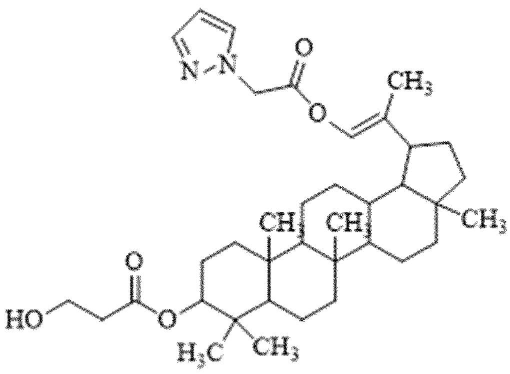

1-(1-(2-(1H-pyrazol-1-yl)acetoxy)prop-1-en-2-yl)-3a,5b,8,8,11a-pentamethylicosahydro-1H-cyclopenta[a]chrysen-9-yl 3-hydroxypropanoate,

17.

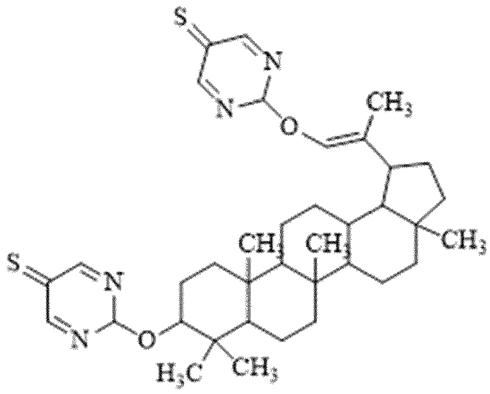

2-((3a,5b,8,8,11a-pentamethyl-1-(1-((5-thioxo-2,5-dihydropyrimidin-2-yl)oxy)prop-1-en-2-yl)icosahydro-1H-cyclopenta[a]chrysen-9-yl)oxy)pyrimidine-5(2H)-thione,

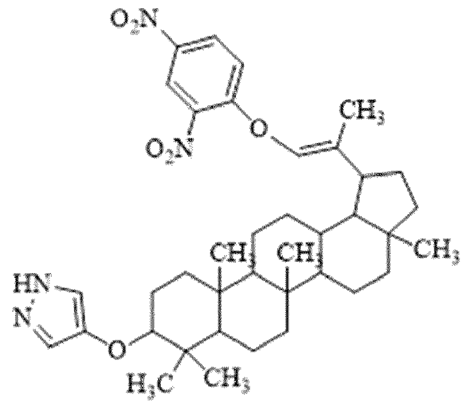

18.

4-((1-(1-(2,4-dinitrophenoxy)prop-1-en-2-yl)-3a,5b,8,8,11a-pentamethylicosahydro-1H-cyclopenta[a]chrysen-9-yl)oxy)-1H-pyrazole,

19.

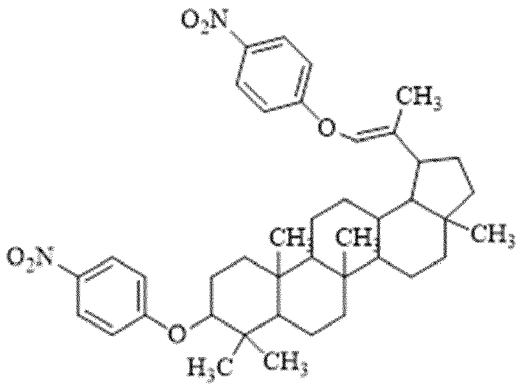

3a,5b,8,8,11a-pentamethyl-9-(4-nitrophenoxy)-1-(1-(4-nitrophenoxy)prop-1-en-2-yl)icosahydro-1H-cyclopenta[a]chrysene,

20.

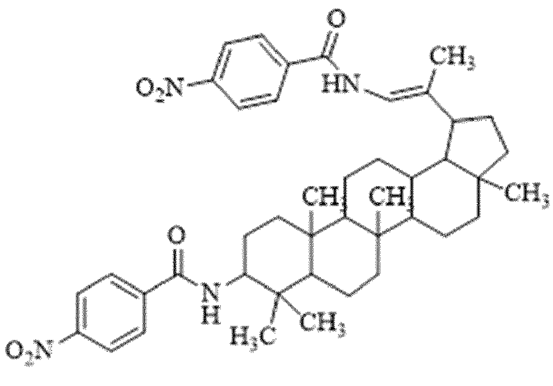

4-nitro-N-(3a,5b,8,8,11a-pentamethyl-1-(1-(4-nitrobenzamido)prop-1-en-2-yl)icosahydro-1H-cyclopenta[a]chrysen-9-yl)benzamide,

21.

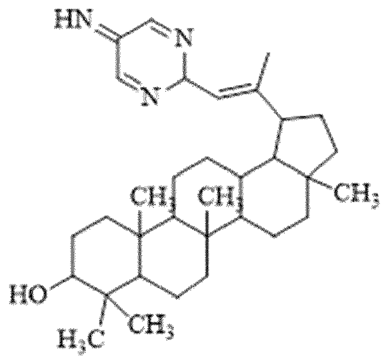

1-(1-(5-imino-2,5-dihydropyrimidin-2-yl)prop-1-en-2-yl)-3a,5b,8,8,11a-pentamethylicosahydro-1H-cyclopenta[a]chrysen-9-ol,

22.

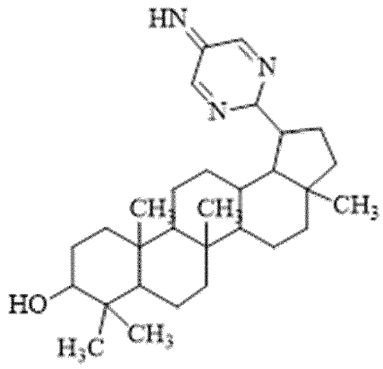

1-(5-imino-2,5-dihydropyrimidin-2-yl)-3a,5b,8,8,11a-pentamethylicosahydro-1H-cyclopenta[a]chrysen-9-ol.

**9.** The pharmaceutical composition according to claim 1, wherein the indol-3-carbinol derivative is selected from the group consisting of:

7.

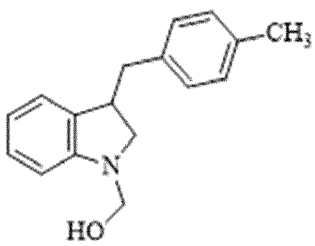

(3-(4-methylbenzyl)indolin-1-yl)methanol,

8.

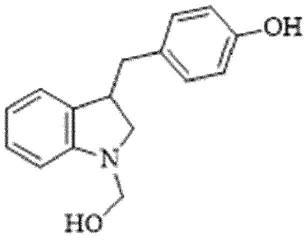

4-((1-(hydroxymethyl)indolin-3-yl)methyl)phenol,

9.

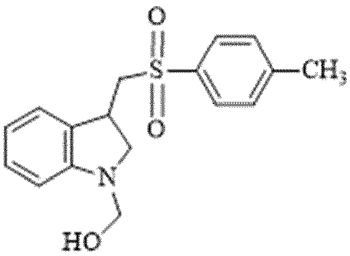

(3-(tosylmethyl)indolin-1-yl)methanol,

10.

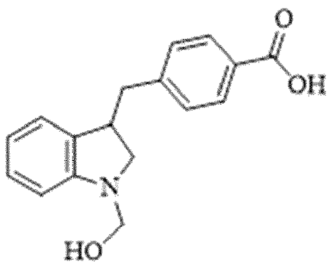

4-((1-(hydroxymethyl)indolin-3-yl)methyl)benzoic acid,

11.

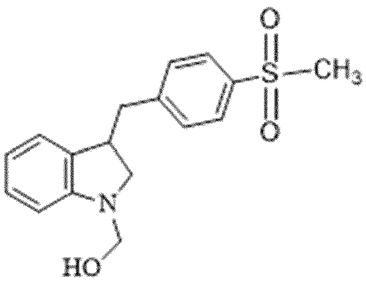

(3-(4-(methylsulfonyl)benzyl)indolin-1-yl)methanol,

12.

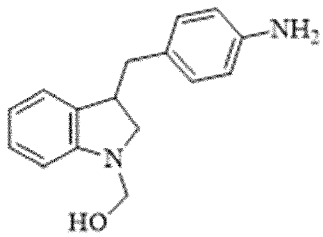

(3-(4-aminobenzyl)indolin-1-yl)methanol.

**10.** The pharmaceutical composition according to any one of claims 1 to 9, wherein the composition comprises a mixture of one or more apigenin derivative, indol-3-carbinol derivative, lupeol derivative, piperlongumin derivative, quercetin derivate, curcumin derivative, resveratrol derivative and one or more checkpoint inhibitors selected from the group consisting of CTLA4, PD-1, PD-L1, PD-L2, LAG3, B7-H3, B7-H4, KIR, OX40, IgG, IDO-1, IDO-2, CEACAM1, TNFRSF4, OX40L, TIM3, BTLA, HVEM, TIM3, GAL9, LAG3, VISTA, KIR, 2B4, CD 160, CGEN-15049, CHK 1, CHK2, A2aR, and B-7or combinations thereof.

**11.** A pharmaceutical composition according to any one of claims 1 to 10 for use in the treatment or prevention of cancer.

**12.** An in-vitro method for polarization or differentiation of monocytes or peripheral blood mononuclear cells (PBMCs) into macrophages with a M1-like phenotype with anti-tumor properties, comprising the steps of:

a) isolation of monocytes or PBMCs from a blood sample of a subject,
b) Polarization or differentiation of the isolated monocytes and/or PBMCs into macrophages M1-like phenotype having anti-tumor properties by incubating the isolated monocytes-derived macrophages with at least one active compound selected from the group consisting of apigenin derivative, indol-3-carbinol derivative, lupeol derivative, piperlongumin derivative, quercetin derivate, curcumin derivative and/or resveratrol derivative, or a mixture thereof.

**13.** A method of preparation of repolarized macrophages with M1-like phenotype with anti-tumor properties, comprising the steps of:

a) Isolation of monocytes or peripheral blood mononuclear cells (PBMCs) from a blood sample of a subject,
b) Polarization or differentiation of the isolated monocytes and/or PBMCs into macrophages M1-like phenotype having anti-tumor properties by incubating the isolated monocytes-derived macrophages with at least one active compound selected from the group consisting of apigenin derivative, indol-3-carbinol derivative, lupeol derivative, piperlongumin derivative, quercetin derivate, curcumin derivative and/or resveratrol derivative, or a mixture thereof;
c) Isolation and purification of the repolarized macrophages.

**14.** The method according to claim 13, wherein the active compound utilized in the method for polarization or differentiation of the isolated monocytes and/or PBMCs into macrophages M1-like phenotype having anti-tumor properties is any one as defined in claims 1 to 10, or a mixture of these compounds.

**15.** The method of claim 12 or claim 13, wherein the isolation of monocytes or PBMCs is carried out by using CD14 antibodies, and the polarization or differentiation of monocytes or PBMCs is carried out

a) by incubation with of macrophage colony-stimulating factor (M-CSF) to convert the monocytes or PBMCs in M0-type macrophages;
b) by incubation of the generated M0-type macrophages into M1-like macrophages.

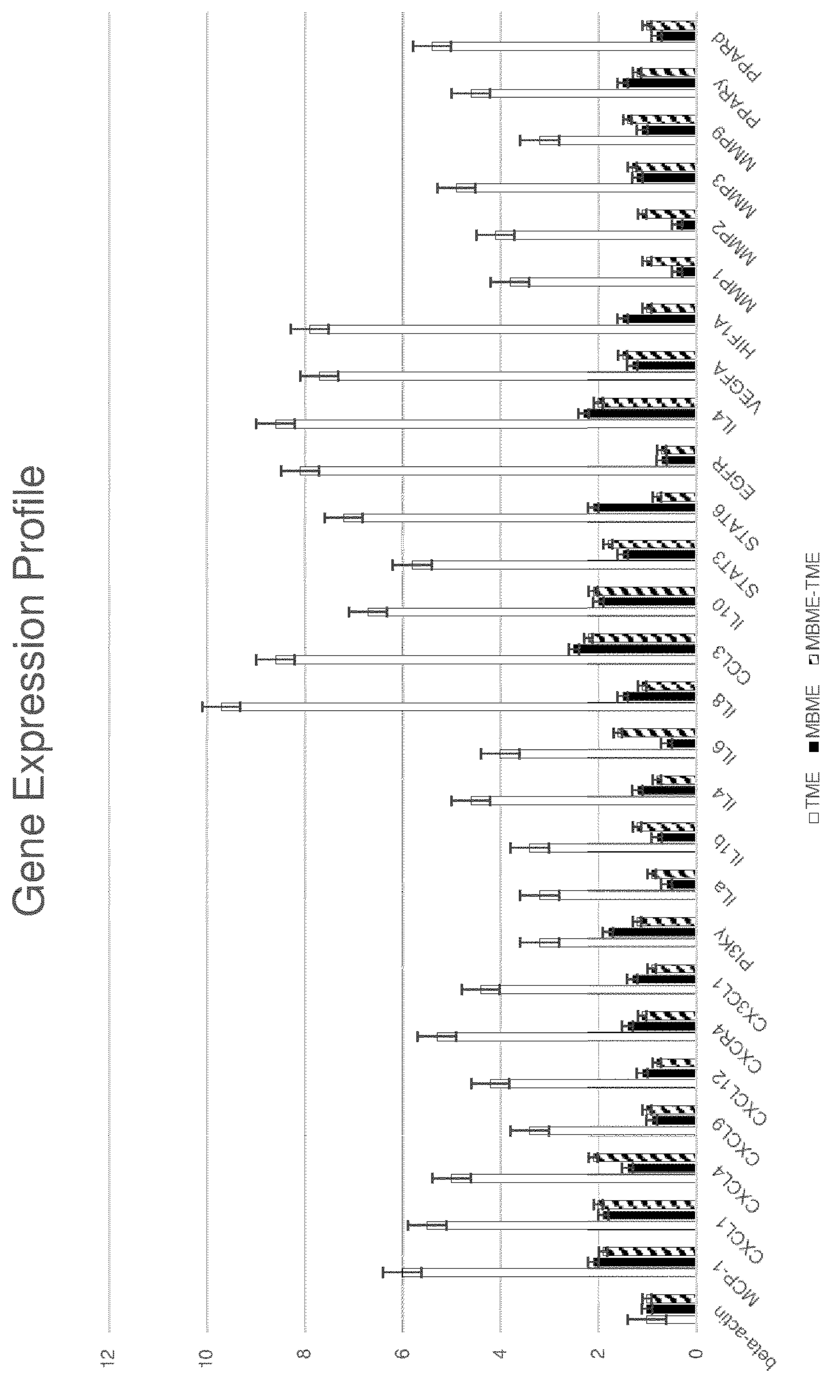
Gene Expression Profile
12
10
8
6
4
2
0
beta-actin
MCP-1
CXCL1
CXCL4
CXCL9
CXCL12
CXCR4
CX3CL1
PI3Kγ
ILa
IL1b
IL4
IL6
IL8
CCL3
IL10
STAT3
STAT6
EGFR
IL4
VEGFA
HIF1A
MMP1
MMP2
MMP3
MMP9
PPARγ
PPARd
TME
MBME
MBME-TME

Figure 1A

Figure 1B

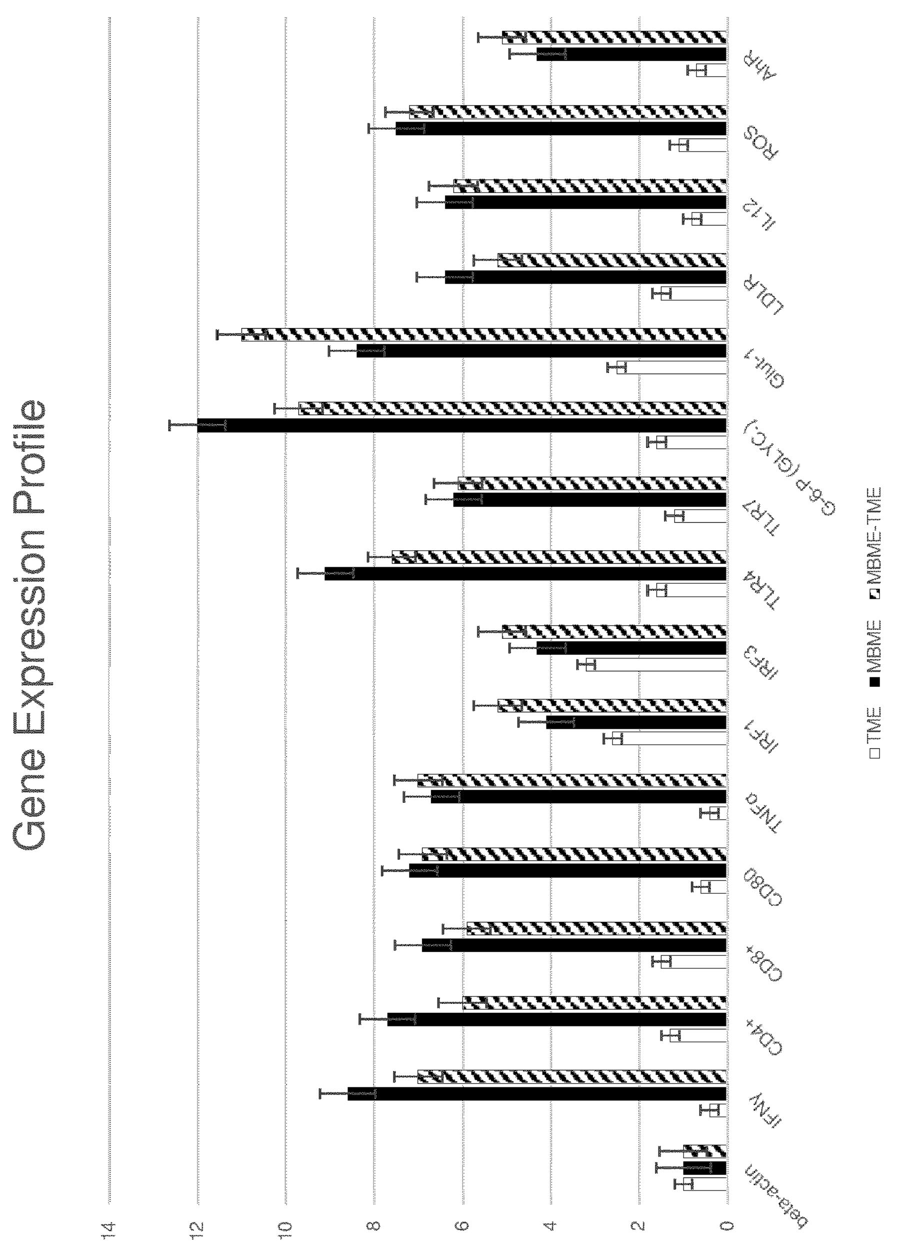
Gene Expression Profile
0
2
4
6
8
10
12
14
beta-actin
IFNγ
CD4+
CD8+
CD80
TNFα
IRF1
IRF3
TLR4
TLR7
G-6-P (GLYC.)
Glut-1
LDLR
IL12
ROS
AhR
TME
MBME
MBME-TME

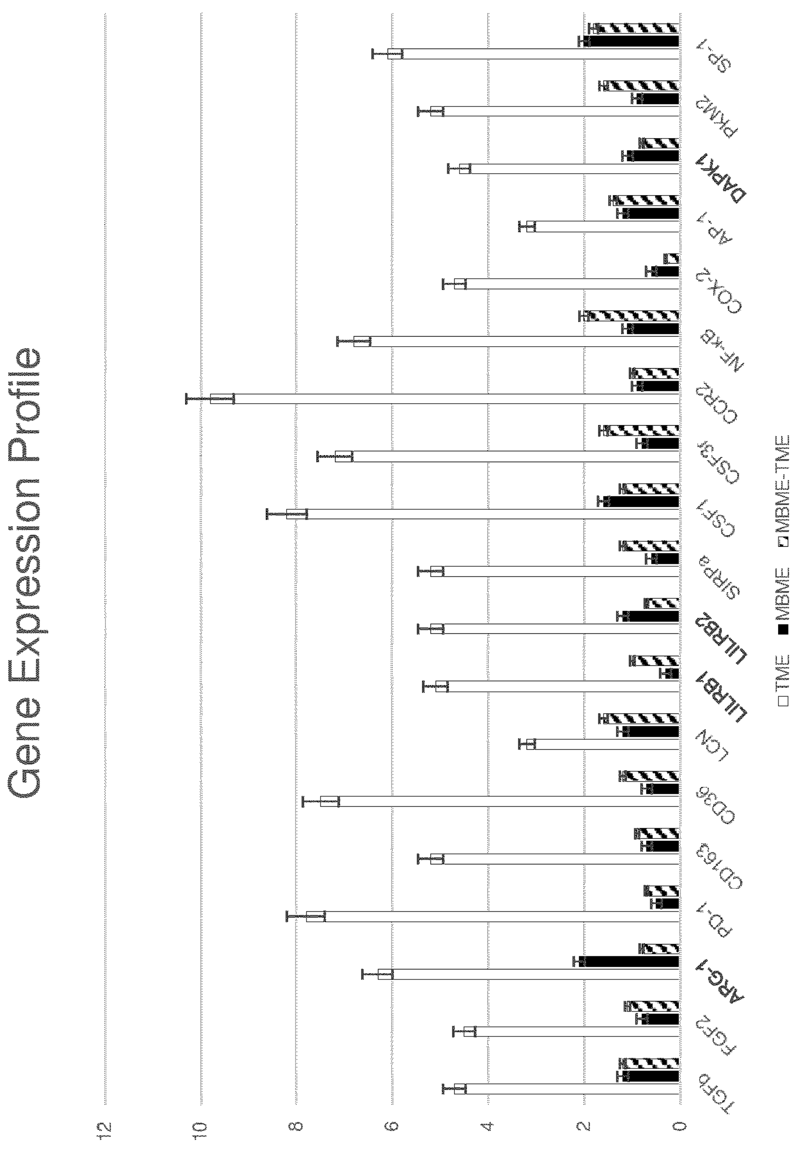
Gene Expression Profile
12
10
8
6
4
2
0
TGFb
FGF2
ARG-1
PD-1
CD163
CD36
LCN
LILRB1
LILRB2
SIRPa
CSF1
CSF3r
CCR2
NF-κB
COX-2
AP-1
DAPK1
PKM2
SP-1
TME
MBME
MBME-TME

Figure 1C

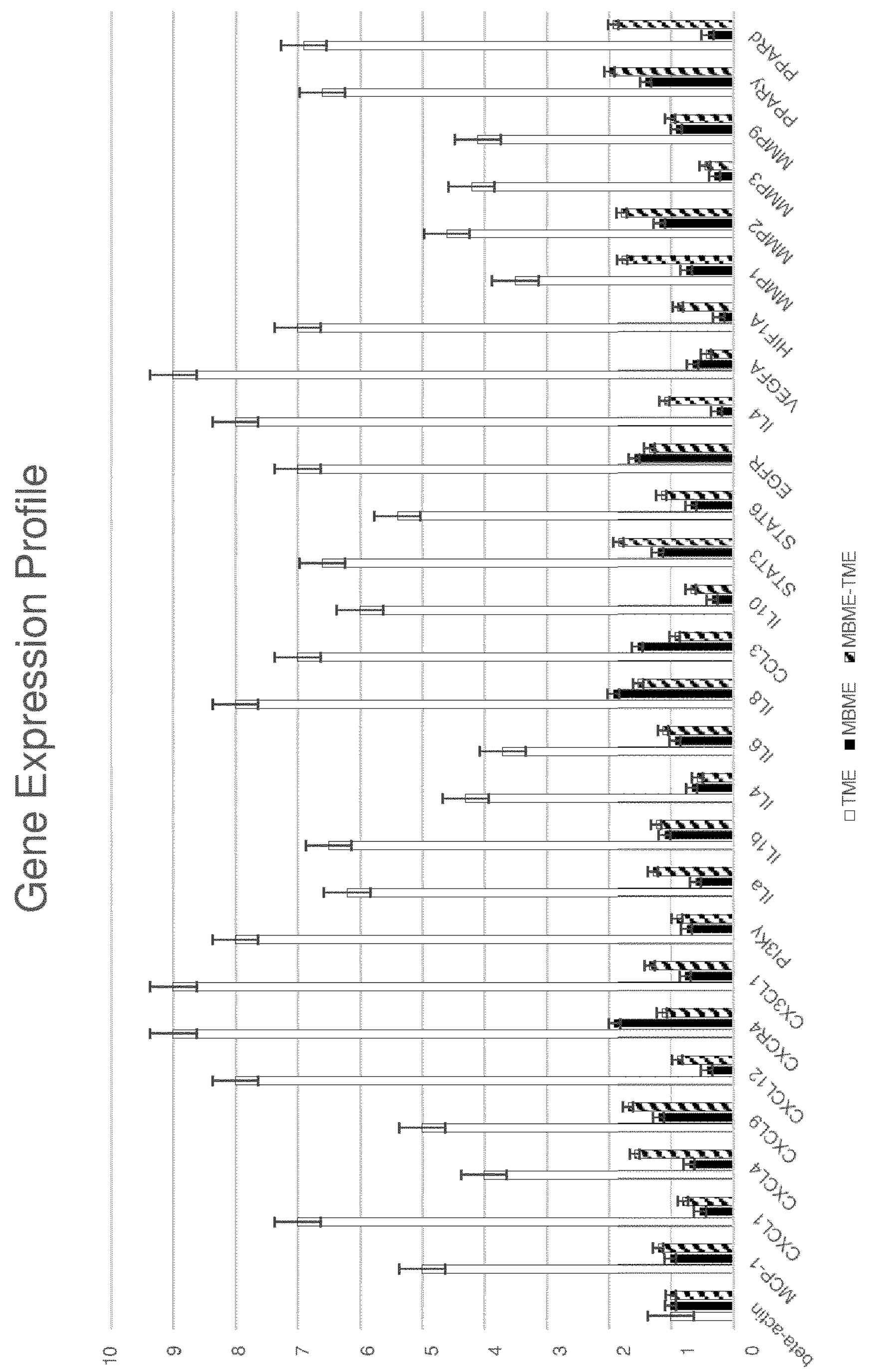
Gene Expression Profile
10
9
8
7
6
5
4
3
2
1
0
beta-actin
MCP-1
CXCL1
CXCL4
CXCL9
CXCL12
CXCR4
CX3CL1
PI3Kγ
ILa
IL1b
IL4
IL6
IL8
CCL3
IL10
STAT3
STAT6
EGFR
IL4
VEGFA
HIF1A
MMP1
MMP2
MMP3
MMP9
PPARγ
PPARd
□ TME ■ MBME ▨ MBME-TME

Figure 2A

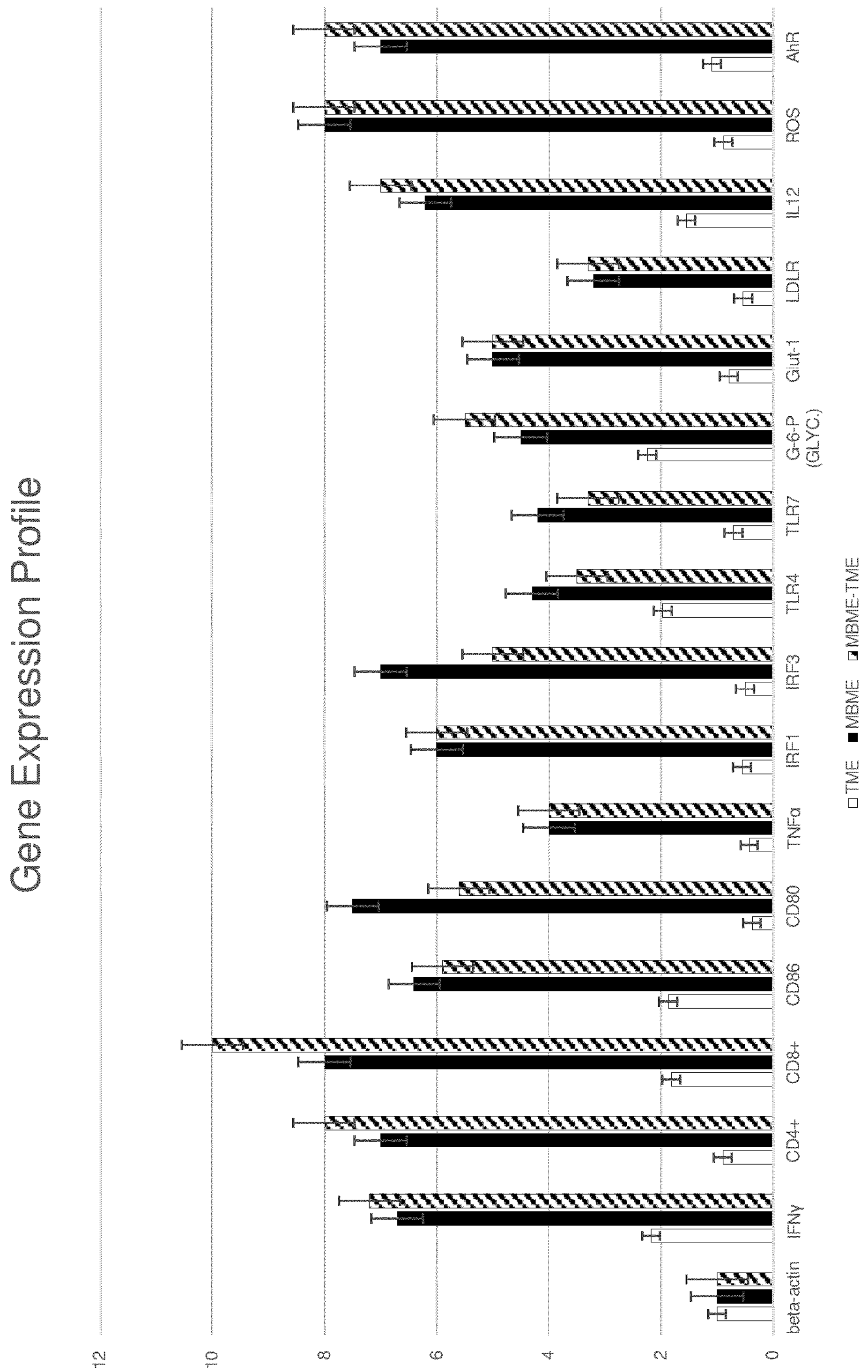
Gene Expression Profile
12
10
8
6
4
2
0
beta-actin
IFNγ
CD4+
CD8+
CD86
CD80
TNFα
IRF1
IRF3
TLR4
TLR7
G-6-P (GLYC.)
Glut-1
LDLR
IL12
ROS
AhR
TME
MBME
MBME-TME

Figure 2B

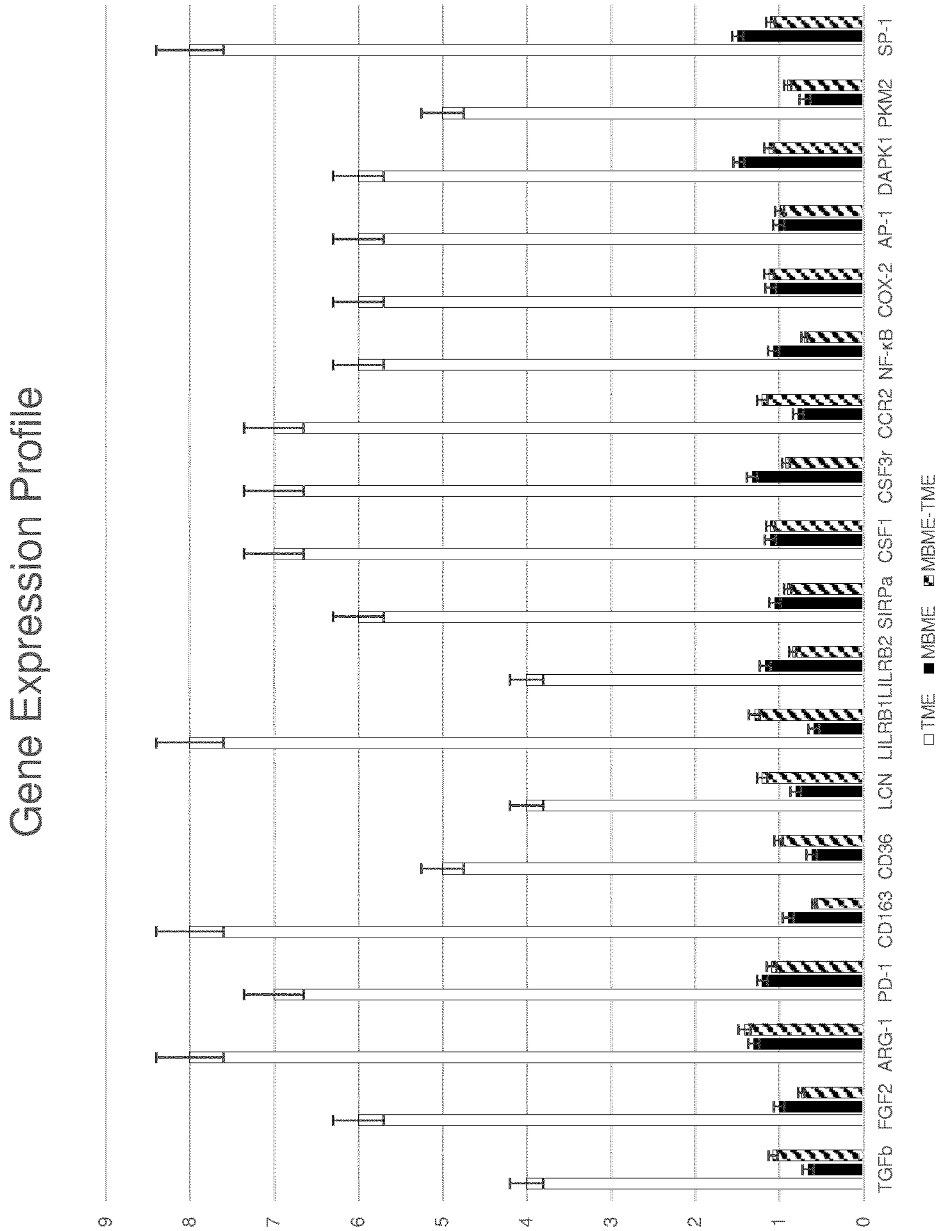
Gene Expression Profile
0
1
2
3
4
5
6
7
8
9
TGFb
FGF2
ARG-1
PD-1
CD163
CD36
LCN
LILRB1
LILRB2
SIRPa
CSF1
CSF3r
CCR2
NF-κB
COX-2
AP-1
DAPK1
PKM2
SP-1
□ TME ■ MBME ▨ MBME-TME

Figure 2C

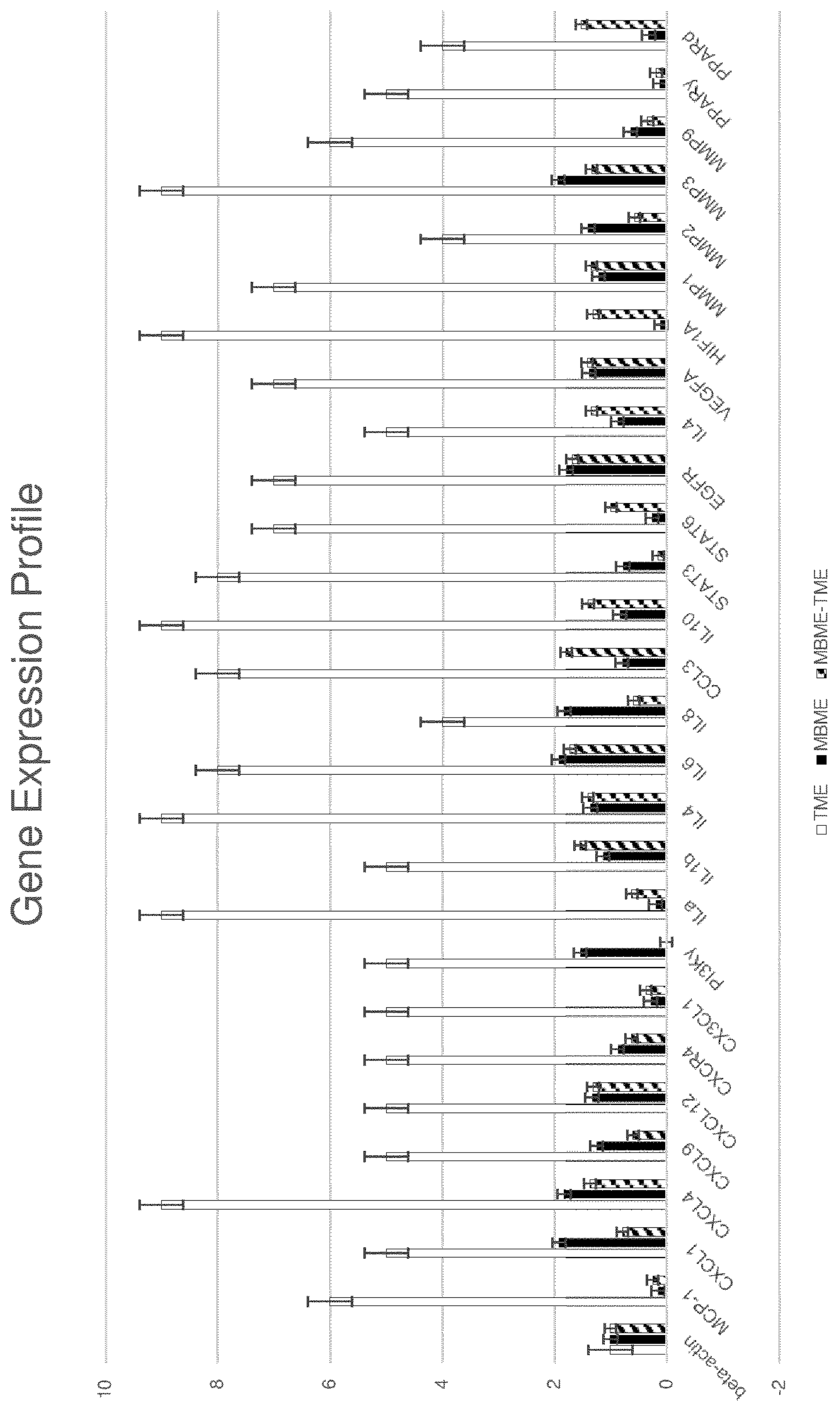
Gene Expression Profile
10
8
6
4
2
0
-2
beta-actin
MCP-1
CXCL1
CXCL4
CXCL9
CXCL12
CXCR4
CX3CL1
PI3Kγ
ILa
IL1b
IL4
IL6
IL8
CCL3
IL10
STAT3
STAT6
EGFR
IL4
VEGFA
HIF1A
MMP1
MMP2
MMP3
MMP9
PPARγ
PPARd
TME
MBME
MBME-TME

Figure 3A

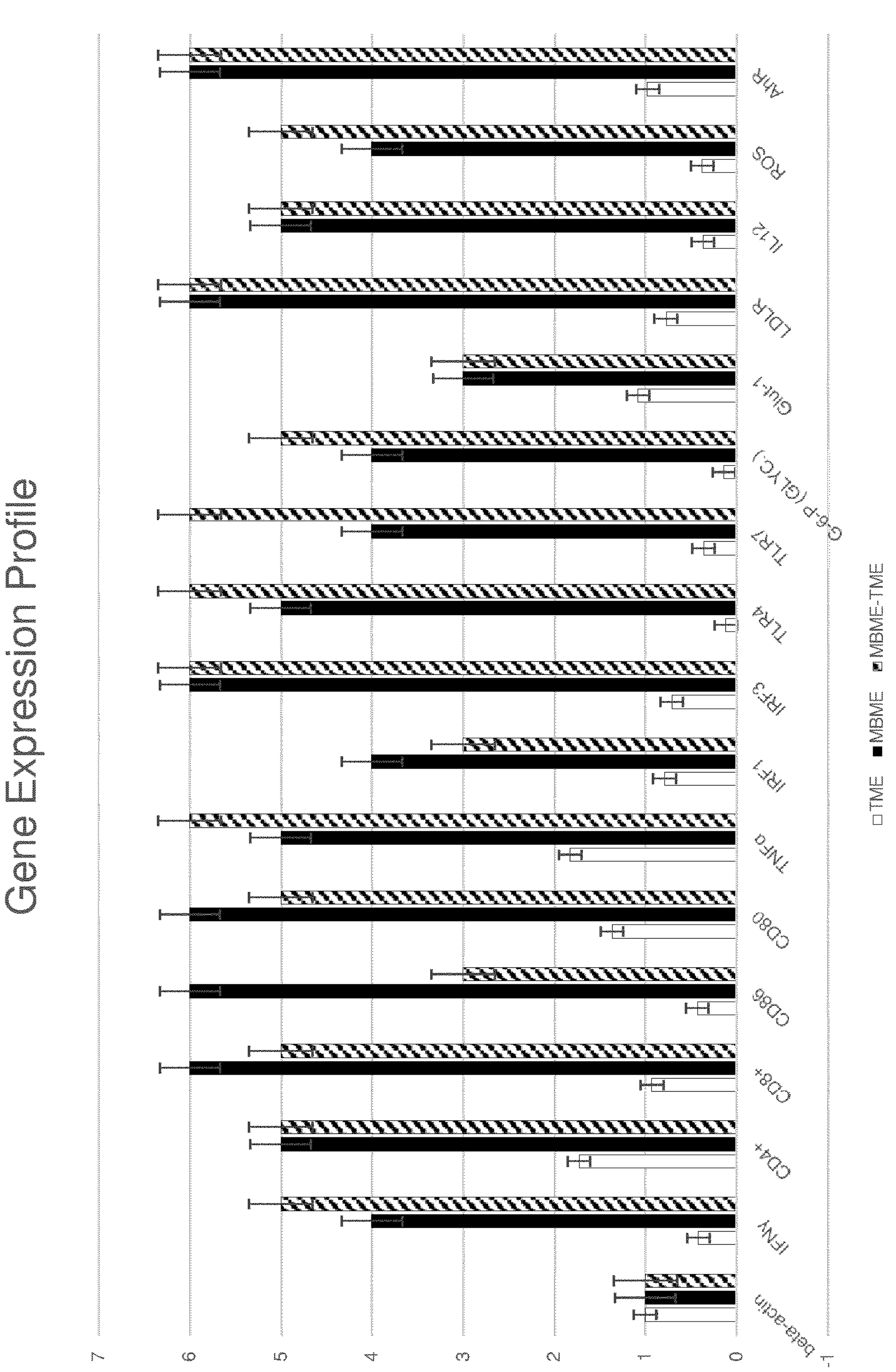
Gene Expression Profile
7
6
5
4
3
2
1
0
-1
beta-actin
IFNγ
CD4+
CD8+
CD86
CD80
TNFα
IRF1
IRF3
TLR4
TLR7
G-6-P (GLYC.)
Glut-1
LDLR
IL12
ROS
AhR
TME
MBME
MBME-TME

Figure 3B

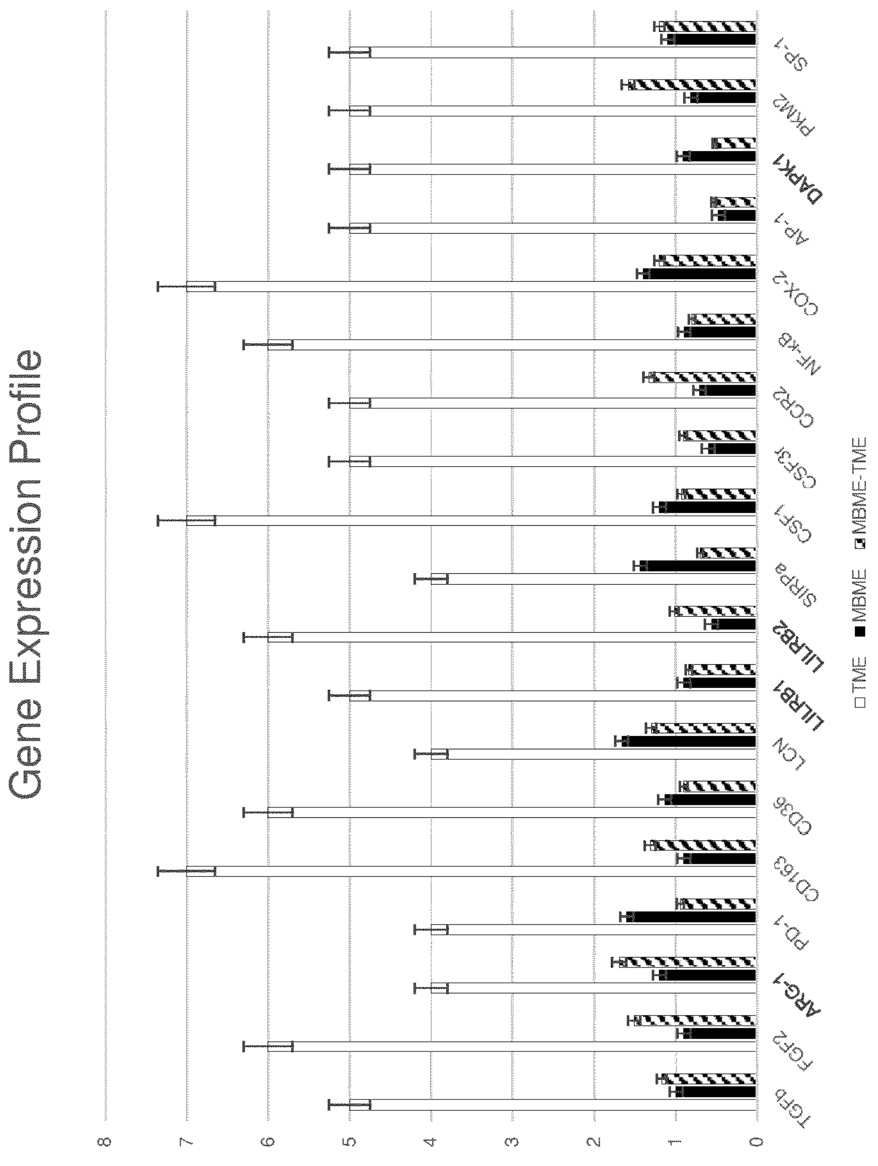
Gene Expression Profile
0
1
2
3
4
5
6
7
8
TGFb
FGF2
ARG-1
PD-1
CD163
CD36
LCN
LILRB1
LILRB2
SIRPa
CSF1
CSF3r
CCR2
NF-κB
COX-2
AP-1
DAPK1
PKM2
SP-1
□TME ■MBME ▨MBME-TME

Figure 3C

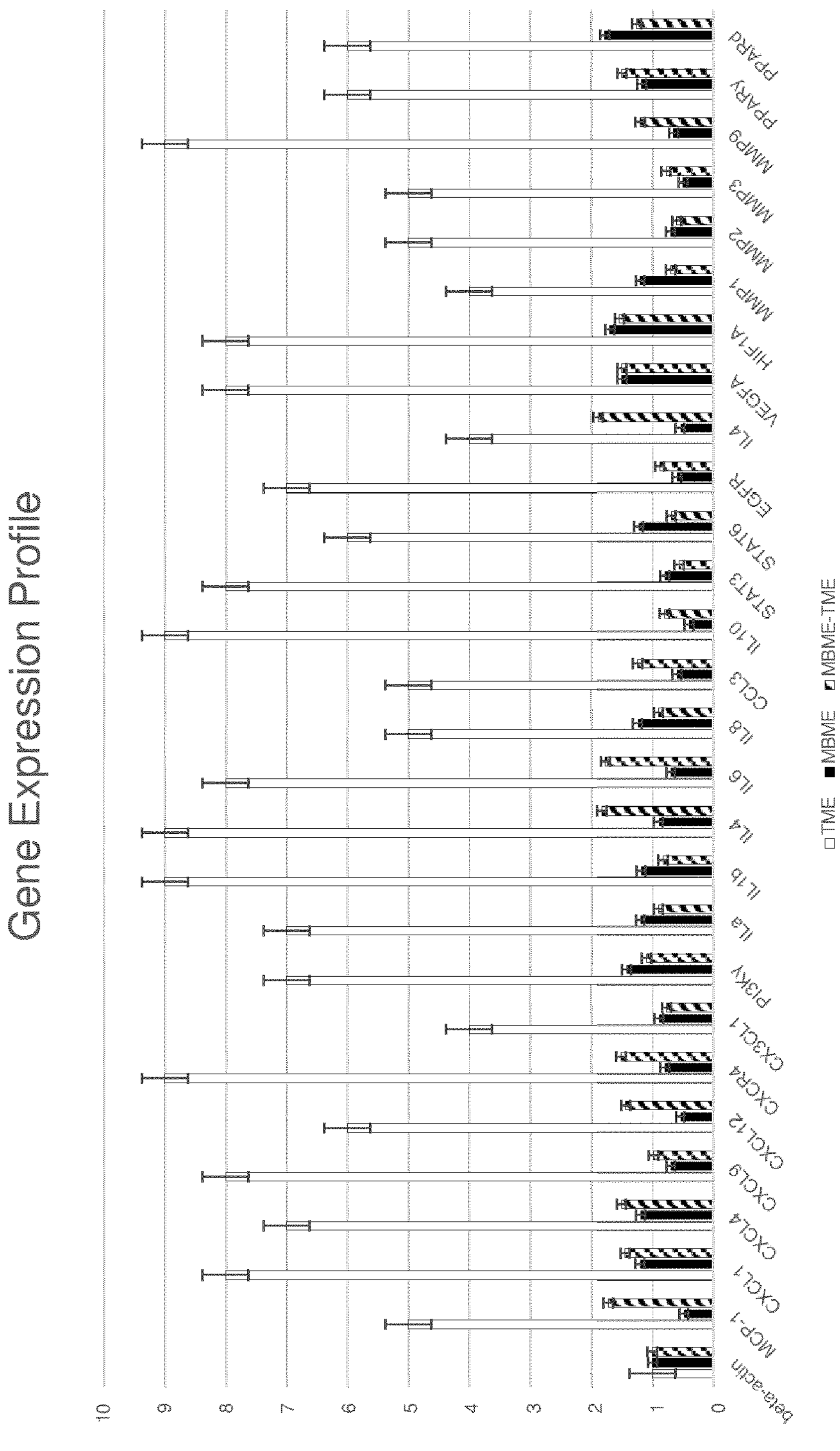
Gene Expression Profile
□TME ■MBME ◩MBME-TME
10
9
8
7
6
5
4
3
2
1
0
beta-actin
MCP-1
CXCL1
CXCL4
CXCL9
CXCL12
CXCR4
CX3CL1
PI3Kγ
ILa
IL1b
IL4
IL6
IL8
CCL3
IL10
STAT3
STAT6
EGFR
IL4
VEGFA
HIF-1A
MMP1
MMP2
MMP3
MMP9
PPARγ
PPARd

Figure 4A

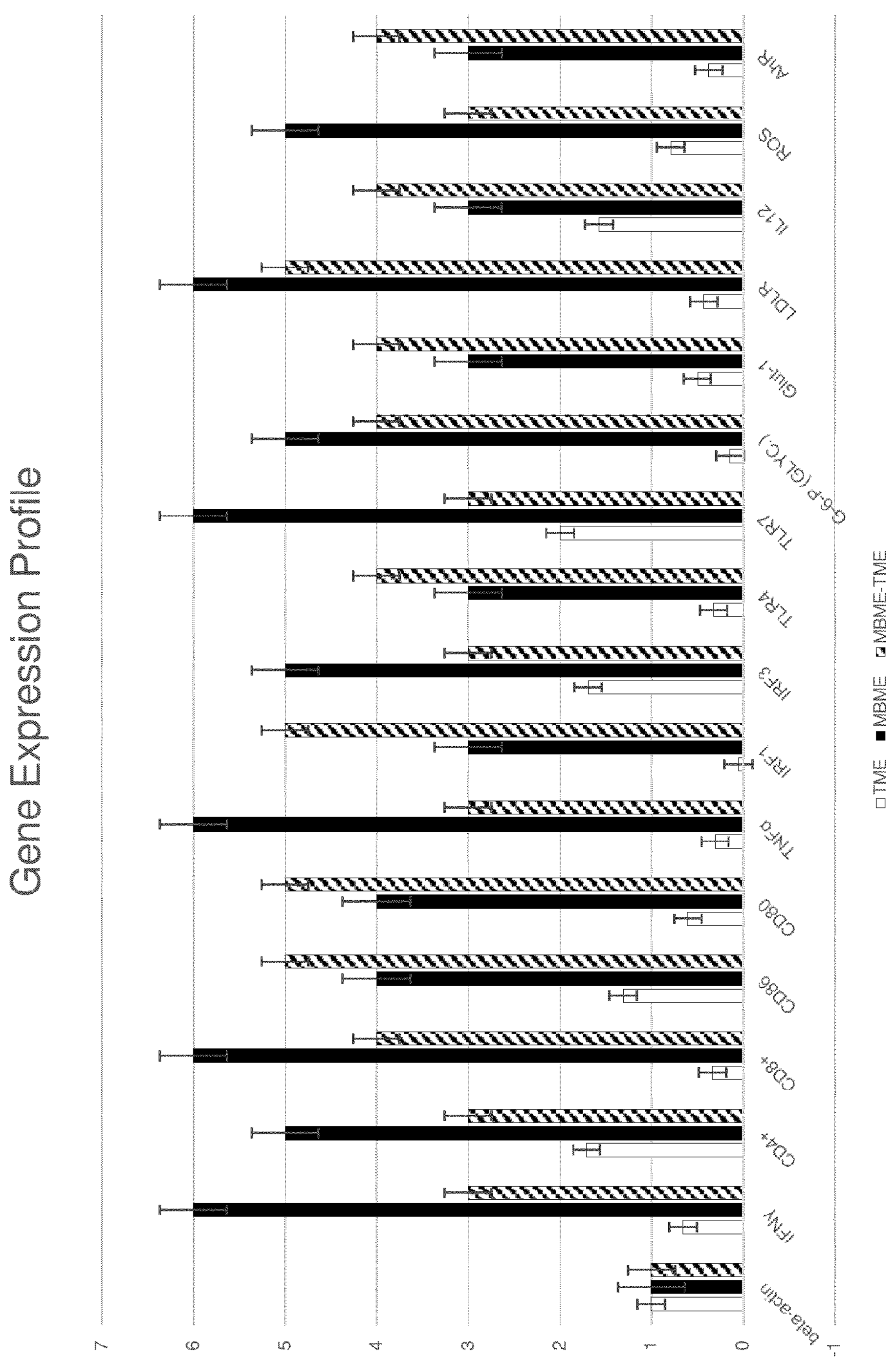
Gene Expression Profile
7
6
5
4
3
2
1
0
-1
beta-actin
IFNγ
CD4+
CD8+
CD86
CD80
TNFα
IRF1
IRF3
TLR4
TLR7
G-6-P (GLYC.)
Glut-1
LDLR
IL12
ROS
AhR
□TME
■MBME
▨MBME-TME

Figure 4B

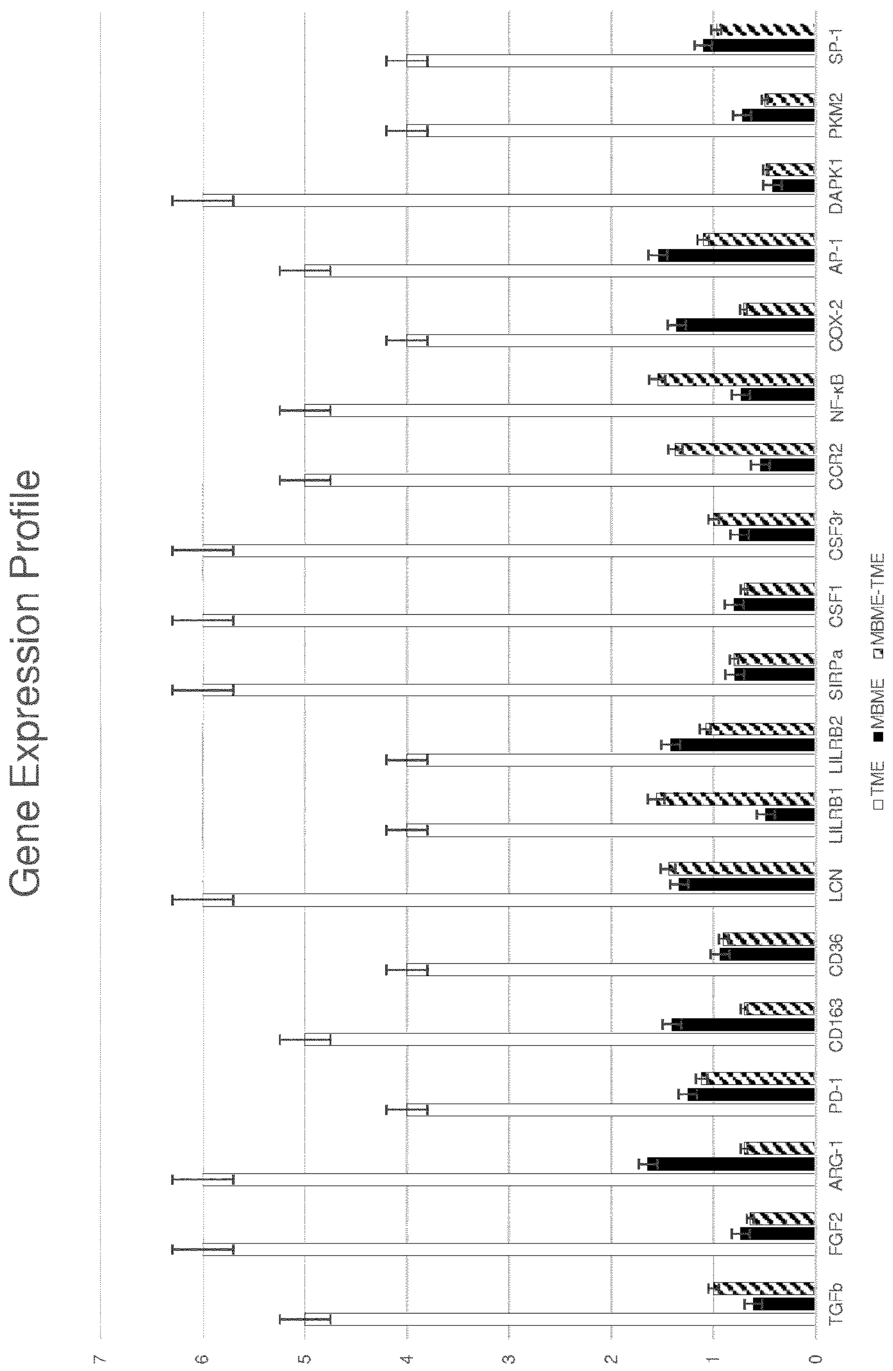
Gene Expression Profile
7
6
5
4
3
2
1
0
TGFb
FGF2
ARG-1
PD-1
CD163
CD36
LCN
LILRB1
LILRB2
SIRPa
CSF1
CSF3r
CCR2
NF-κB
COX-2
AP-1
DAPK1
PKM2
SP-1
□TME ■MBME ◪MBME-TME

Figure 4C

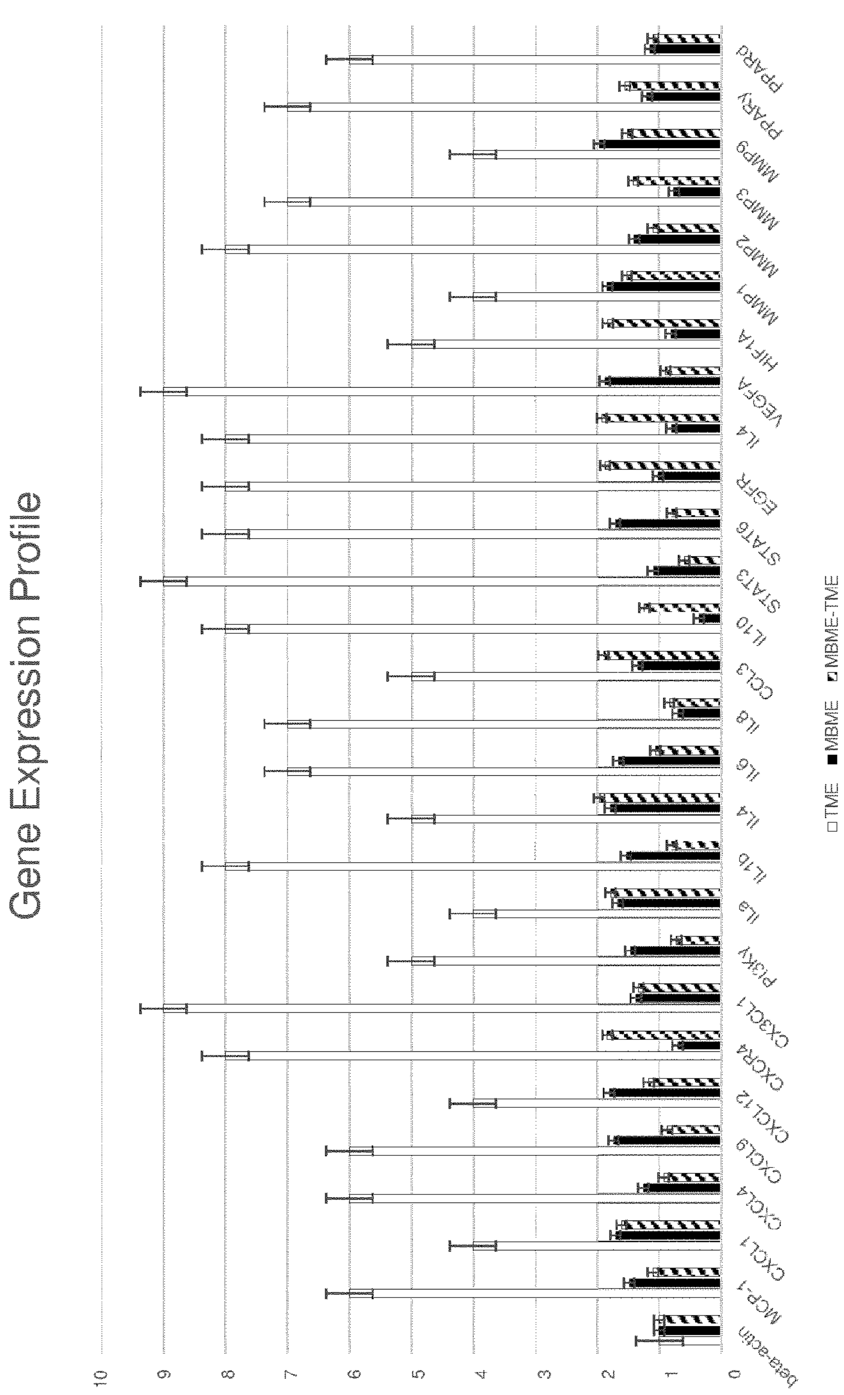
Gene Expression Profile
10
9
8
7
6
5
4
3
2
1
0
beta-actin
MCP-1
CXCL1
CXCL4
CXCL9
CXCL12
CXCR4
CX3CL1
PI3Kγ
IL1a
IL1b
IL4
IL6
IL8
CCL3
IL10
STAT3
STAT6
EGFR
IL4
VEGFA
HIF1A
MMP1
MMP2
MMP3
MMP9
PPARγ
PPARd
□TME ■MBME ▨MBME-TME

Figure 5A

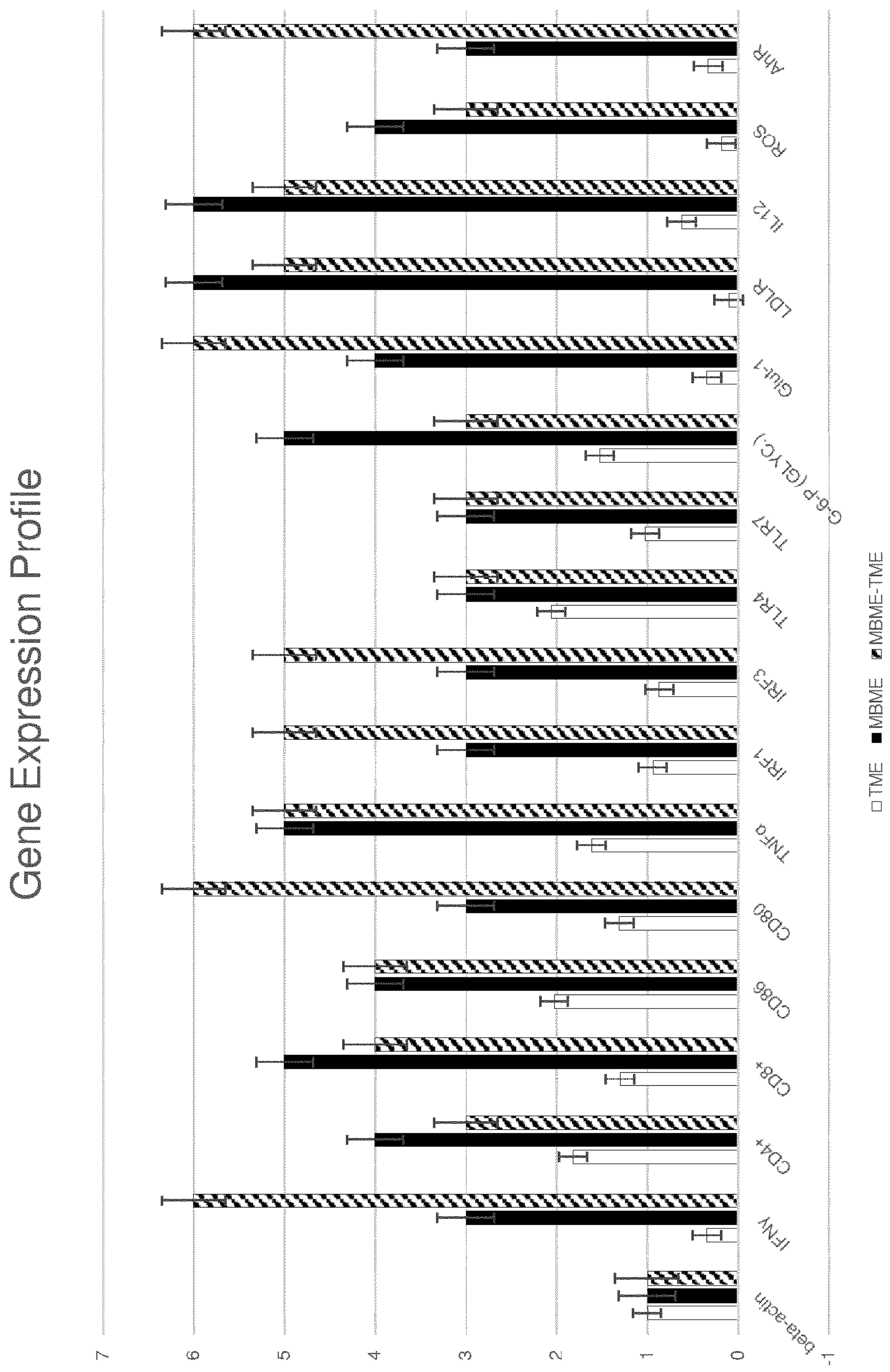
Gene Expression Profile
7
6
5
4
3
2
1
0
-1
beta-actin
IFNγ
CD4+
CD8+
CD86
CD80
TNFα
IRF-1
IRF-3
TLR4
TLR7
G-6-P (GLYC.)
Glut-1
LDLR
IL-12
ROS
AhR
□ TME ■ MBME ◪ MBME-TME

Figure 5B

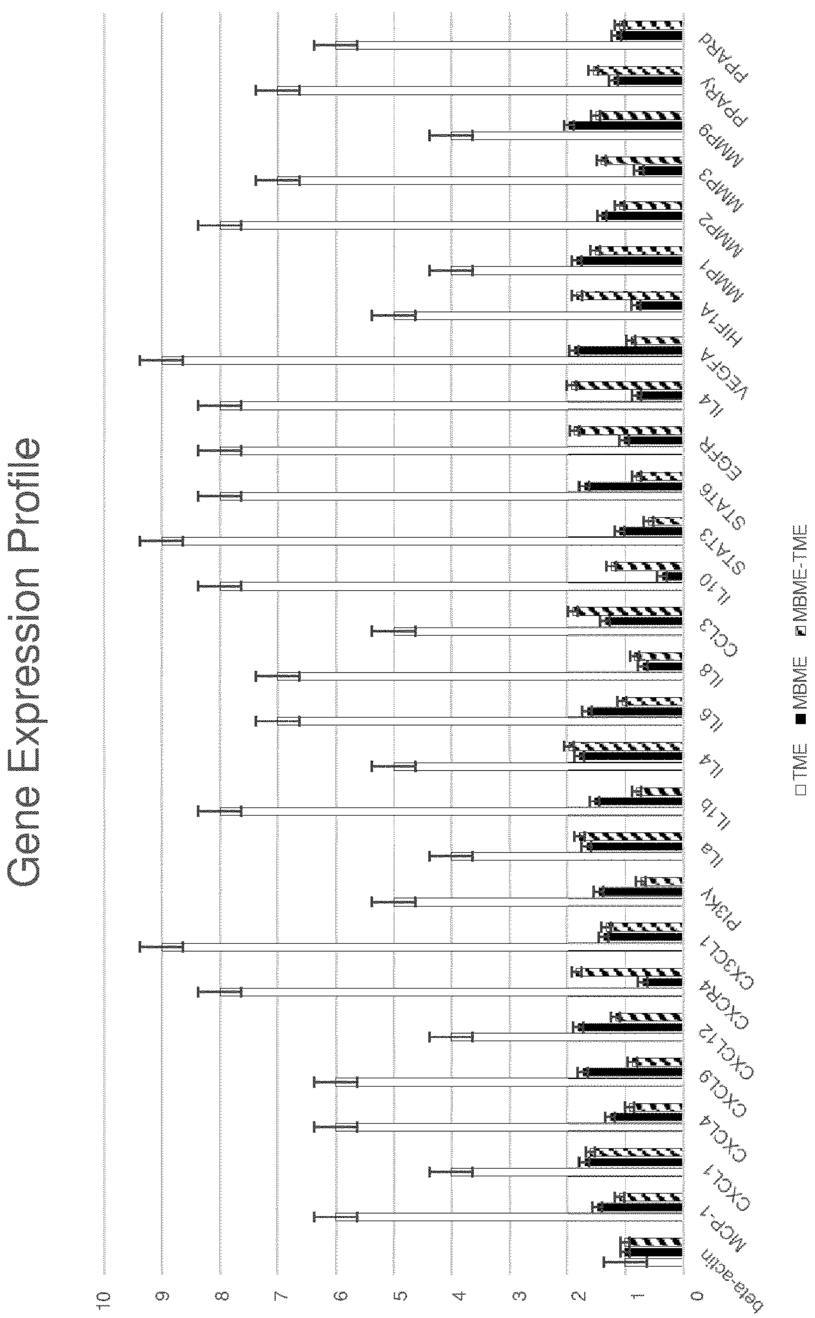
Gene Expression Profile
10
9
8
7
6
5
4
3
2
1
0
beta-actin
MCP-1
CXCL1
CXCL4
CXCL9
CXCL12
CXCR4
CX3CL1
PI3Kγ
ILa
IL1b
IL4
IL6
IL8
CCL3
IL10
STAT3
STAT6
EGFR
IL4
VEGFA
HIF-1A
MMP1
MMP2
MMP3
MMP9
PPARγ
PPARd
TME
MBME
MBME-TME

Figure 5C

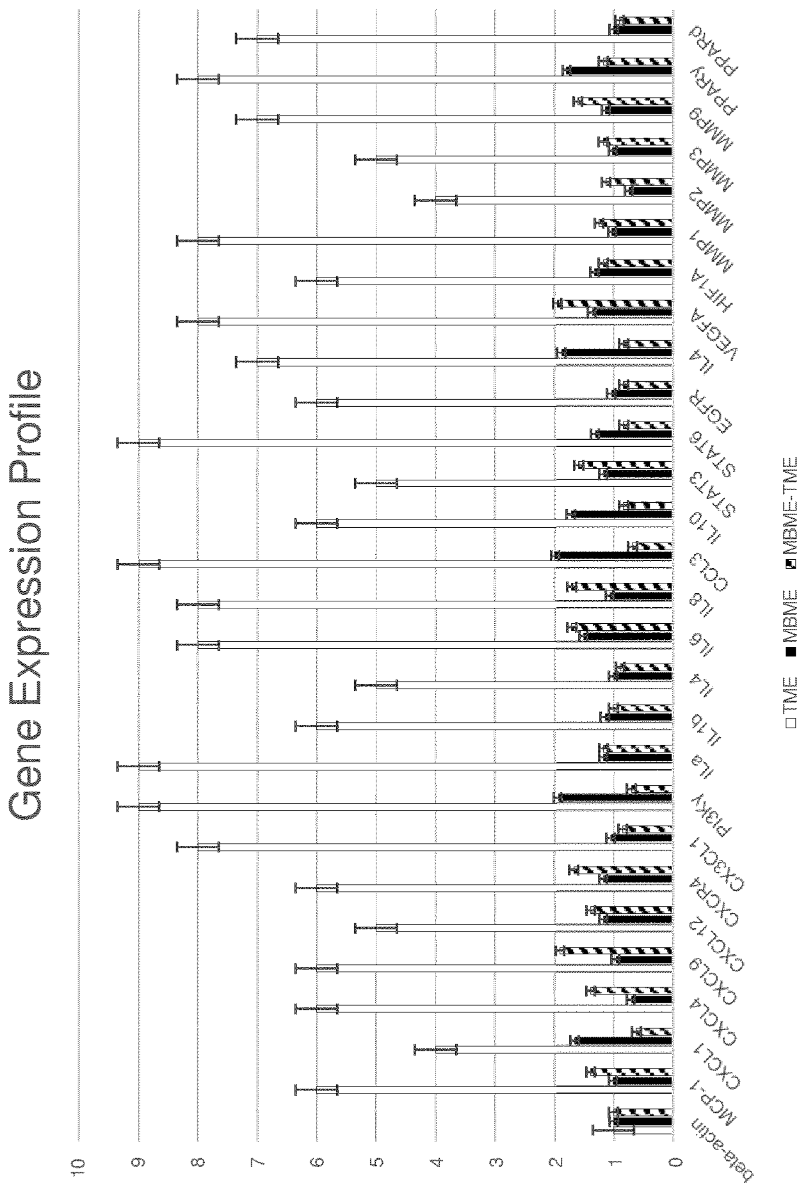
Gene Expression Profile
10
9
8
7
6
5
4
3
2
1
0
beta-actin
MCP-1
CXCL1
CXCL4
CXCL9
CXCL12
CXCR4
CX3CL1
PI3Kγ
ILa
IL1b
IL4
IL6
IL8
CCL3
IL10
STAT3
STAT6
EGFR
IL4
VEGFA
HIF-1A
MMP1
MMP2
MMP3
MMP9
PPARγ
PPARd
TME
MBME
MBME-TME

Figure 6A

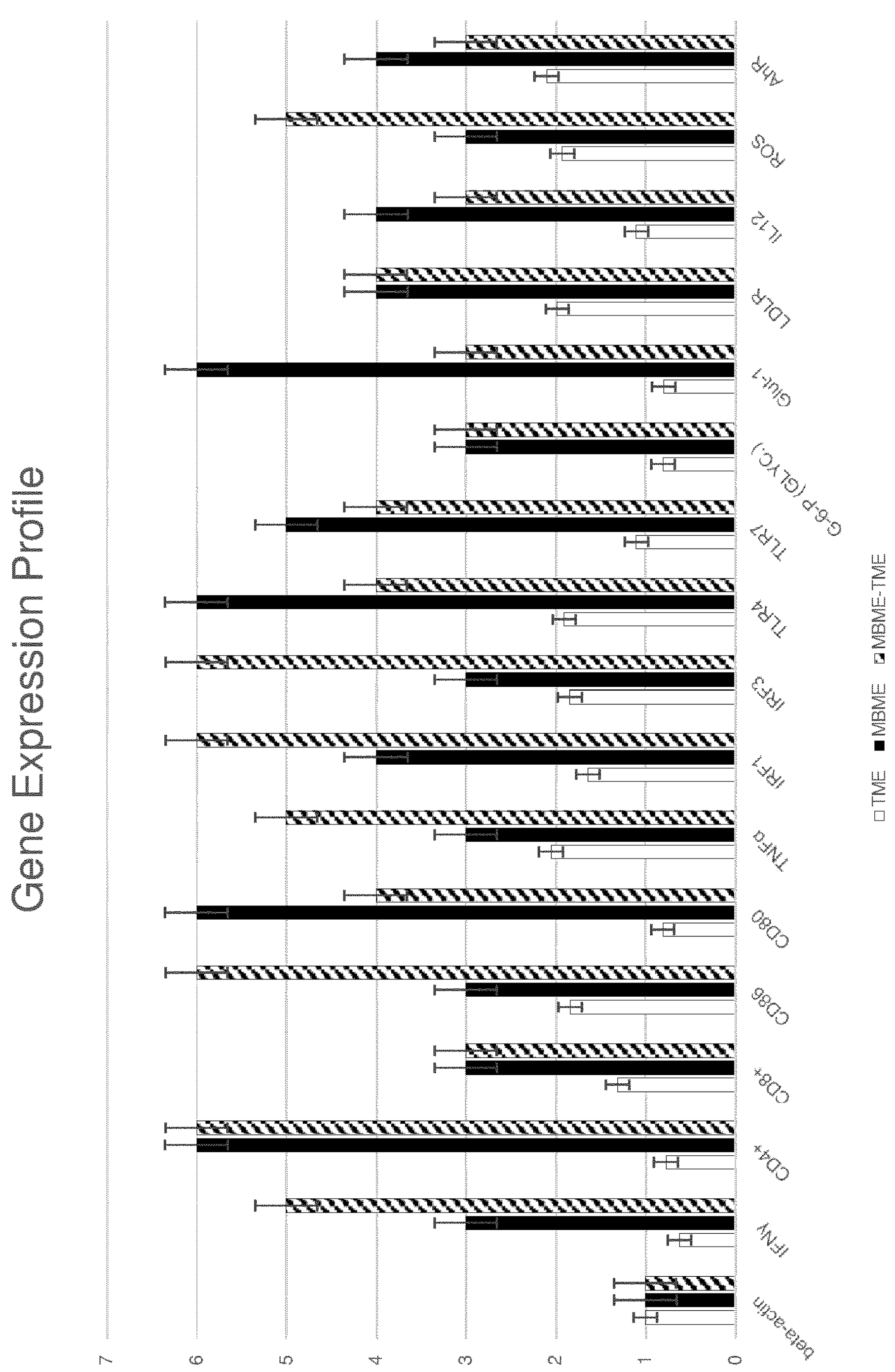
Gene Expression Profile
0
1
2
3
4
5
6
7
beta-actin
IFNγ
CD4+
CD8+
CD86
CD80
TNFα
IRF-1
IRF3
TLR4
TLR7
G-6-P (GLYC.)
Glut-1
LDLR
IL12
ROS
AhR
□ TME ■ MBME ▨ MBME-TME

Figure 6B

Figure 6C

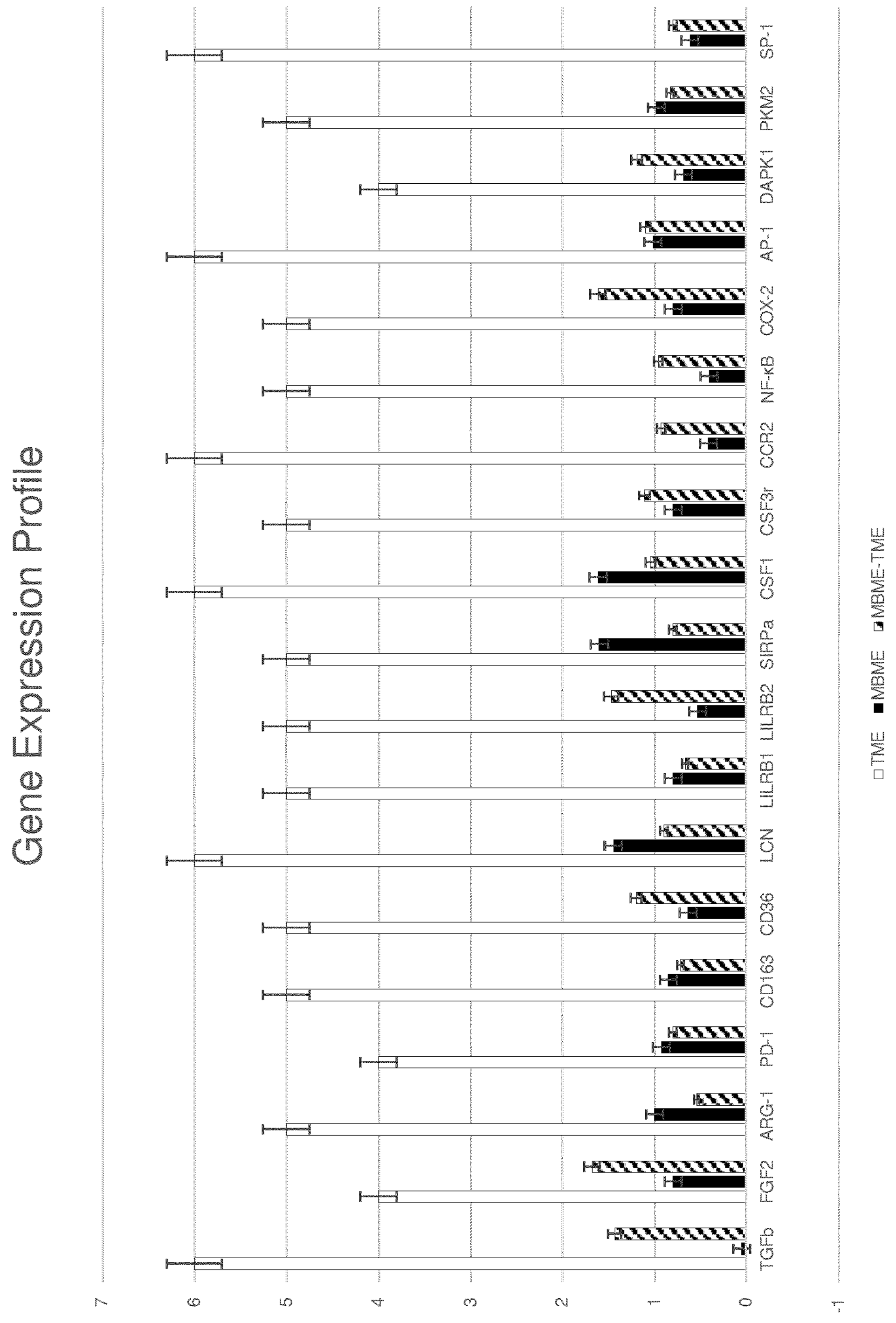
Gene Expression Profile
7
6
5
4
3
2
1
0
-1
TGFb
FGF2
ARG-1
PD-1
CD163
CD36
LCN
LILRB1
LILRB2
SIRPa
CSF1
CSF3r
CCR2
NF-κB
COX-2
AP-1
DAPK1
PKM2
SP-1
TME
MBME
MBME-TME

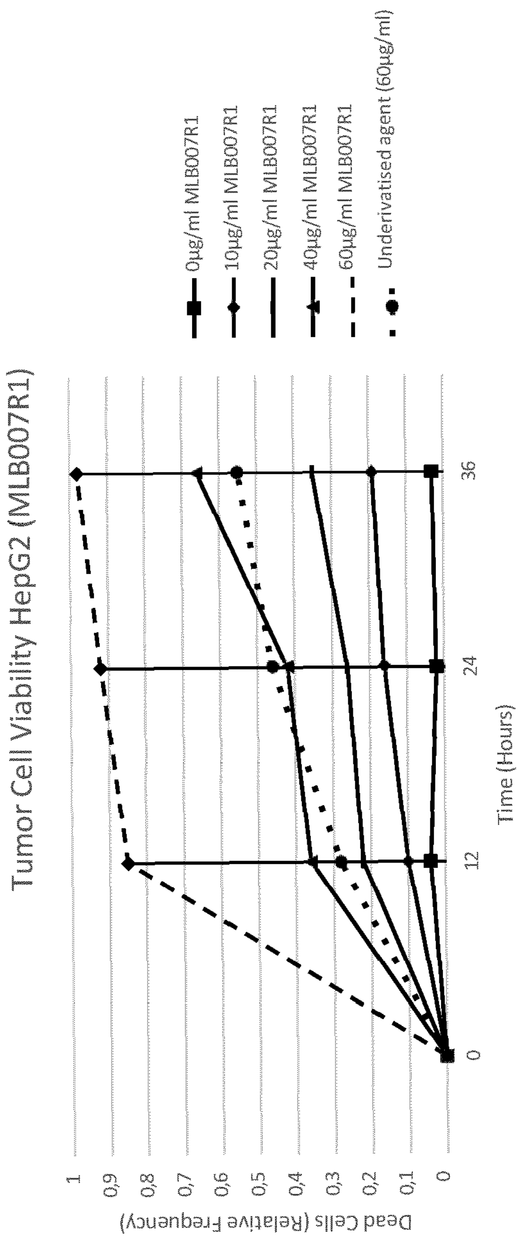
Tumor Cell Viability HepG2 (MLB007R1)
Dead Cells (Relative Frequency)
1
0,9
0,8
0,7
0,6
0,5
0,4
0,3
0,2
0,1
0
0
12
24
36
Time (Hours)
0µg/ml MLB007R1
10µg/ml MLB007R1
20µg/ml MLB007R1
40µg/ml MLB007R1
60µg/ml MLB007R1
Underivatised agent (60µg/ml)

Figure 7A

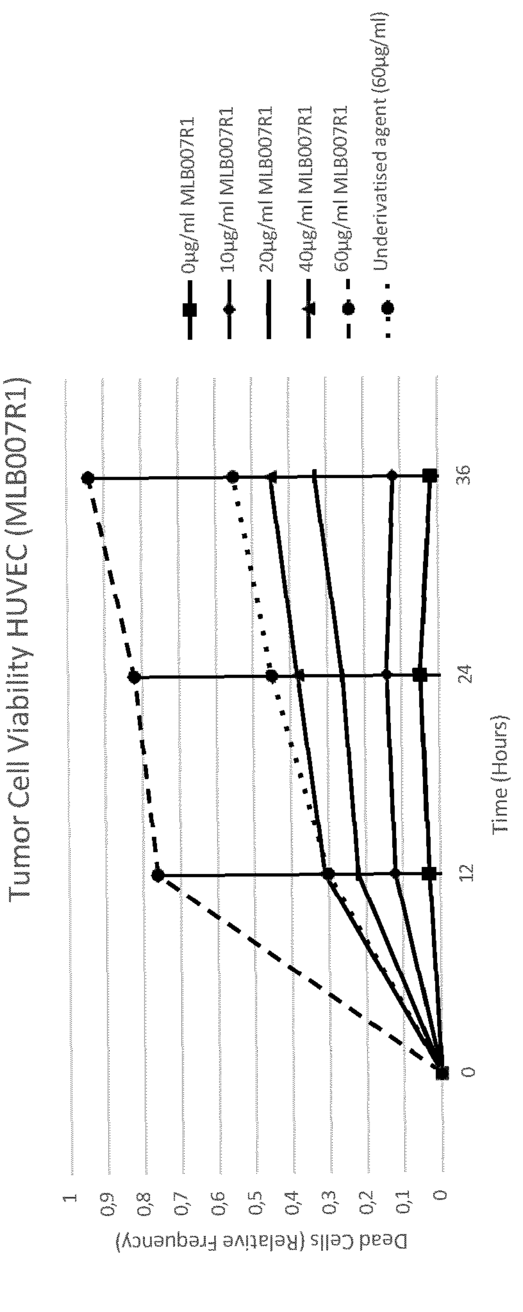
Tumor Cell Viability HUVEC (MLB007R1)
Dead Cells (Relative Frequency)
1
0,9
0,8
0,7
0,6
0,5
0,4
0,3
0,2
0,1
0
Time (Hours)
0
12
24
36
0µg/ml MLB007R1
10µg/ml MLB007R1
20µg/ml MLB007R1
40µg/ml MLB007R1
60µg/ml MLB007R1
Underivatised agent (60µg/ml)

Figure 7B

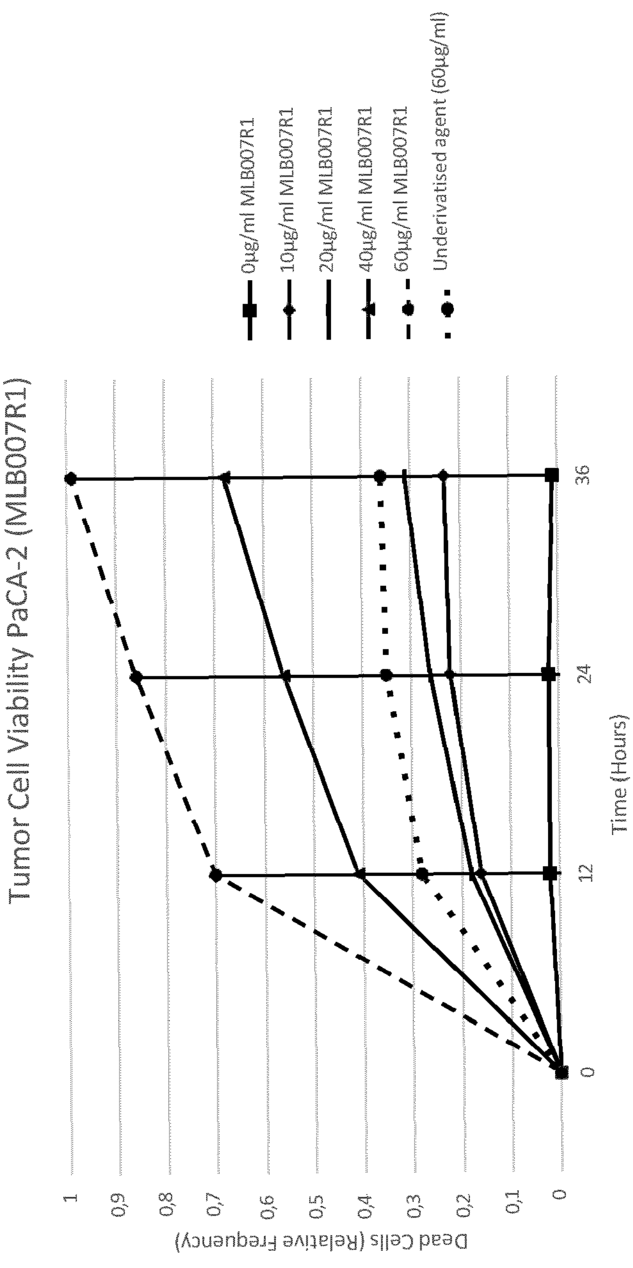
Tumor Cell Viability PaCA-2 (MLB007R1)
Dead Cells (Relative Frequency)
1
0,9
0,8
0,7
0,6
0,5
0,4
0,3
0,2
0,1
0
0
12
24
36
Time (Hours)
0µg/ml MLB007R1
10µg/ml MLB007R1
20µg/ml MLB007R1
40µg/ml MLB007R1
60µg/ml MLB007R1
Underivatised agent (60µg/ml)

Figure 7C

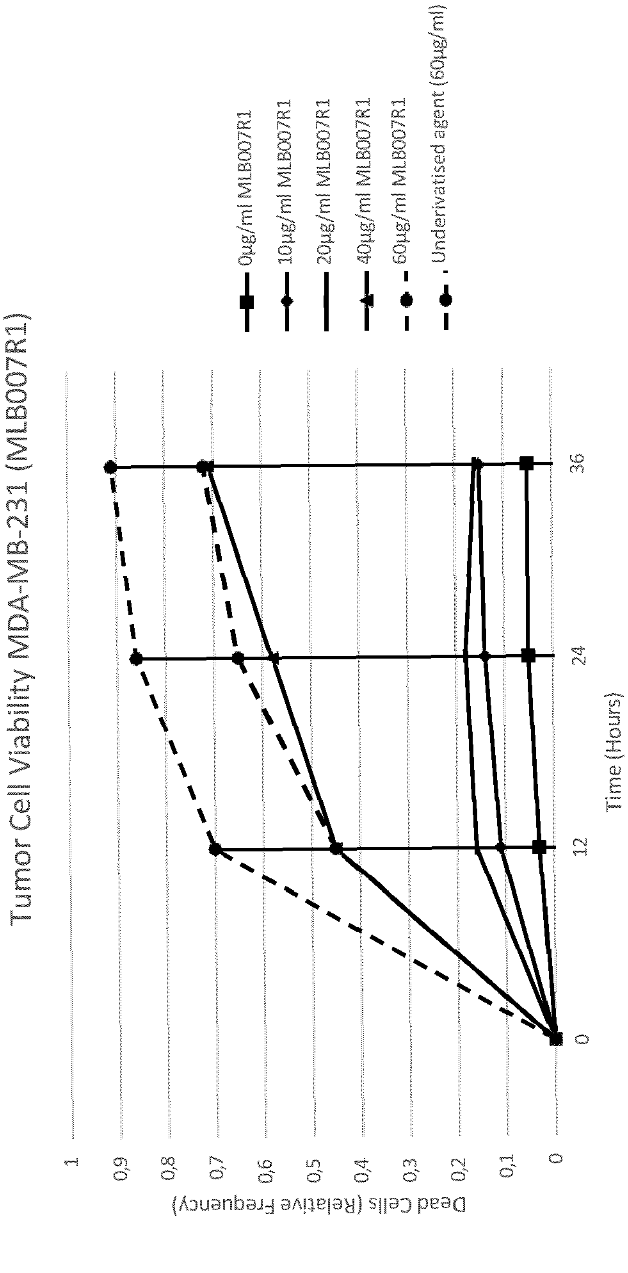
Tumor Cell Viability MDA-MB-231 (MLB007R1)
Dead Cells (Relative Frequency)
1
0,9
0,8
0,7
0,6
0,5
0,4
0,3
0,2
0,1
0
Time (Hours)
0
12
24
36
0µg/ml MLB007R1
10µg/ml MLB007R1
20µg/ml MLB007R1
40µg/ml MLB007R1
60µg/ml MLB007R1
Underivatised agent (60µg/ml)

Figure 7D

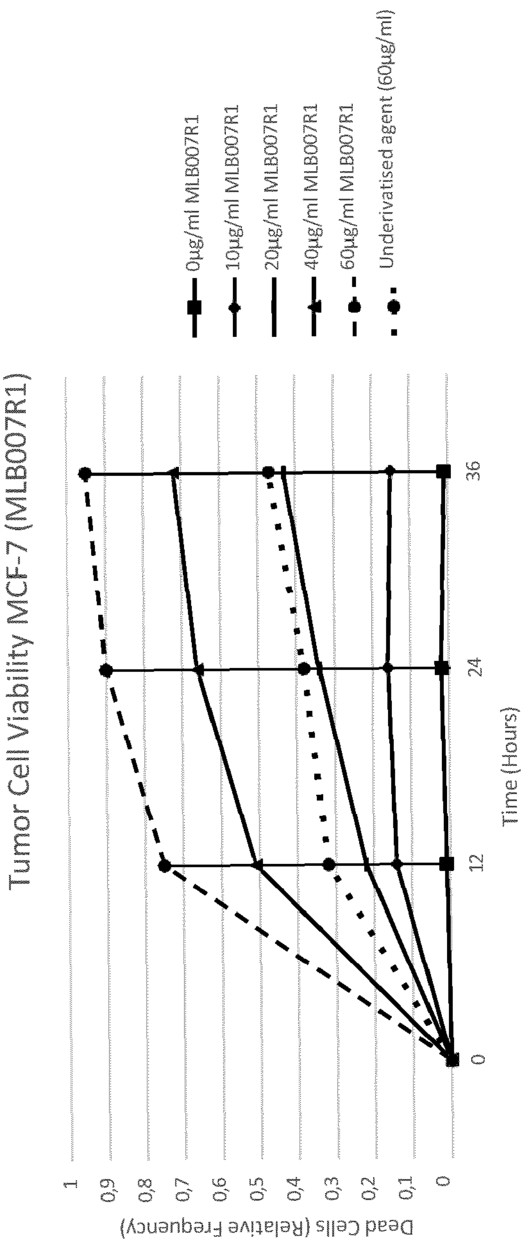
Tumor Cell Viability MCF-7 (MLB007R1)
Dead Cells (Relative Frequency)
1
0,9
0,8
0,7
0,6
0,5
0,4
0,3
0,2
0,1
0
0
12
24
36
Time (Hours)
0µg/ml MLB007R1
10µg/ml MLB007R1
20µg/ml MLB007R1
40µg/ml MLB007R1
60µg/ml MLB007R1
Underivatised agent (60µg/ml)

Figure 7E

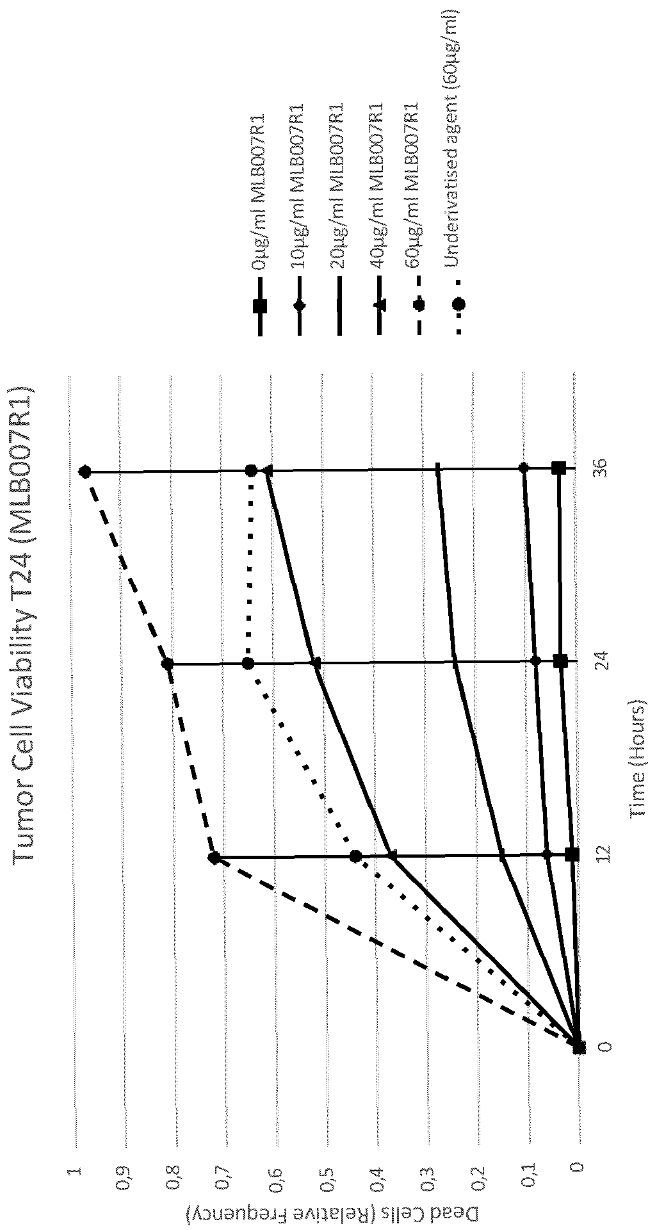
Tumor Cell Viability T24 (MLB007R1)
Dead Cells (Relative Frequency)
0
0,1
0,2
0,3
0,4
0,5
0,6
0,7
0,8
0,9
1
0
12
24
36
Time (Hours)
0µg/ml MLB007R1
10µg/ml MLB007R1
20µg/ml MLB007R1
40µg/ml MLB007R1
60µg/ml MLB007R1
Underivatised agent (60µg/ml)

Figure 7F

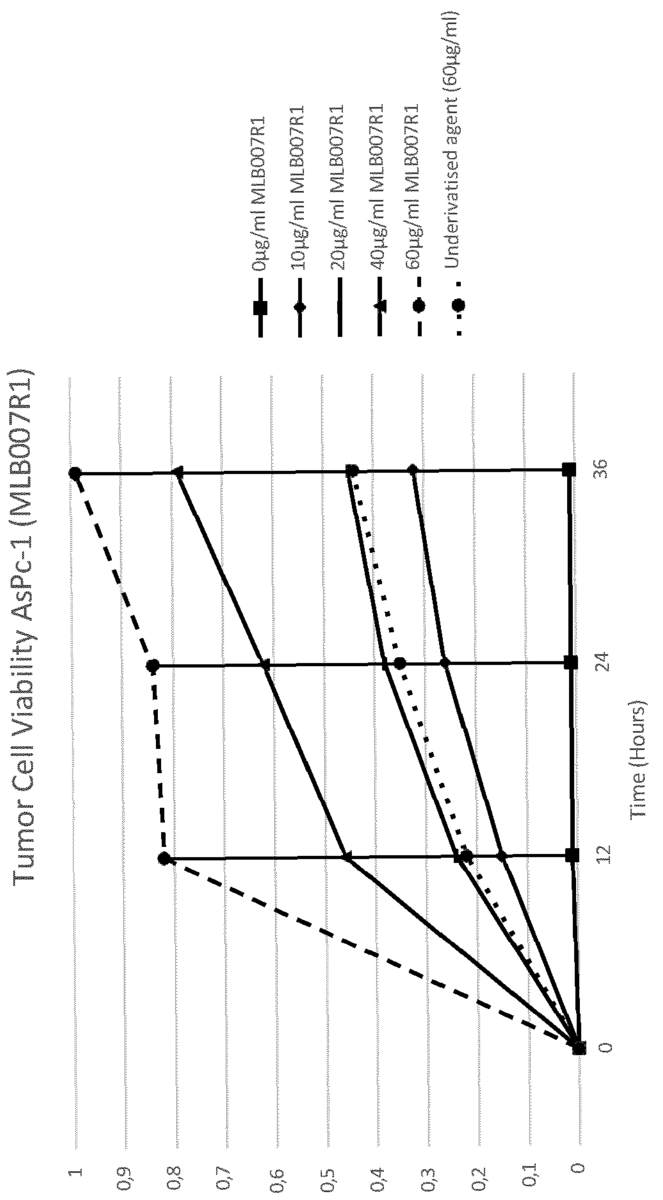
Tumor Cell Viability AsPc-1 (MLB007R1)
Dead Cells (Relative Frequency)
1
0,9
0,8
0,7
0,6
0,5
0,4
0,3
0,2
0,1
0
0
12
24
36
Time (Hours)
0µg/ml MLB007R1
10µg/ml MLB007R1
20µg/ml MLB007R1
40µg/ml MLB007R1
60µg/ml MLB007R1
Underivatised agent (60µg/ml)

Figure 7G

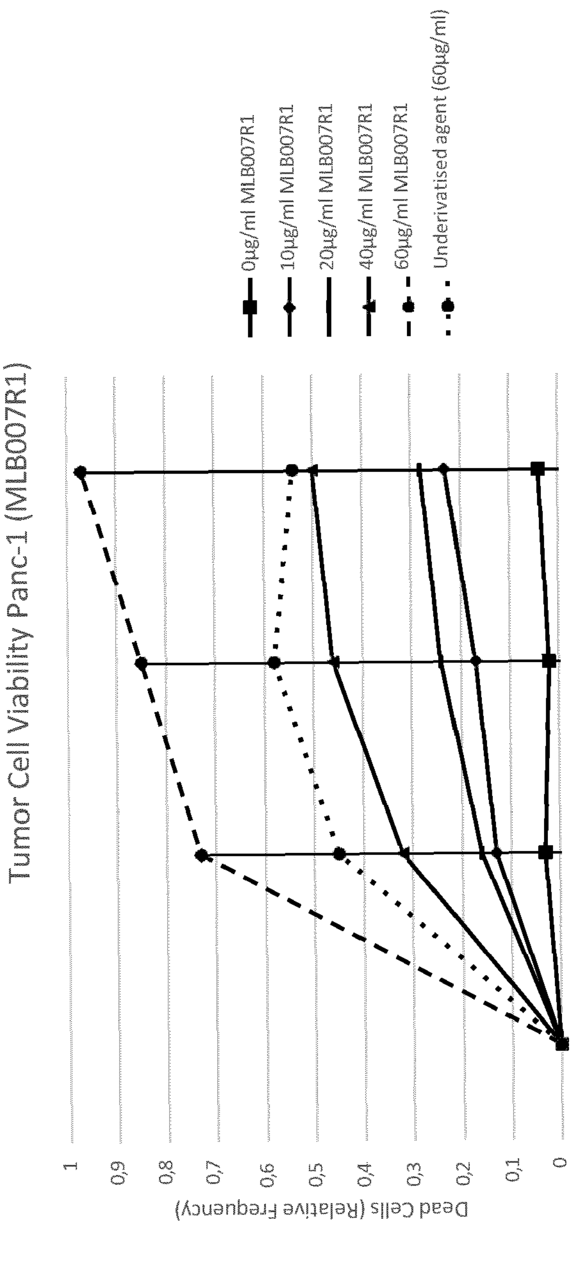
Tumor Cell Viability Panc-1 (MLB007R1)
Dead Cells (Relative Frequency)
1
0,9
0,8
0,7
0,6
0,5
0,4
0,3
0,2
0,1
0
0
12
24
36
Time (Hours)
0µg/ml MLB007R1
10µg/ml MLB007R1
20µg/ml MLB007R1
40µg/ml MLB007R1
60µg/ml MLB007R1
Underivatised agent (60µg/ml)

Figure 7H

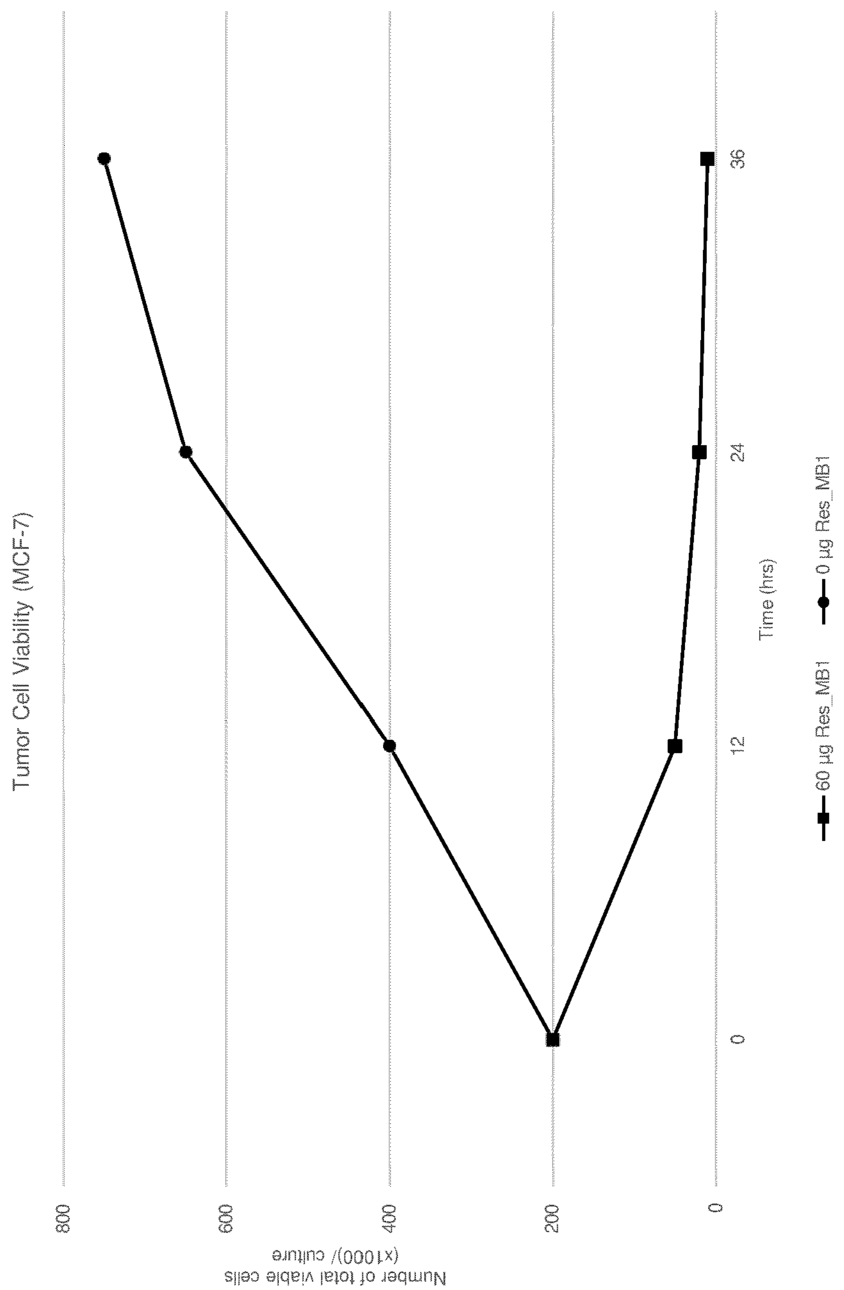
Tumor Cell Viability (MCF-7)
Number of total viable cells (x1000)/ culture
0
200
400
600
800
0
12
24
36
Time (hrs)
60 µg Res_MB1
0 µg Res_MB1

Figure 8A

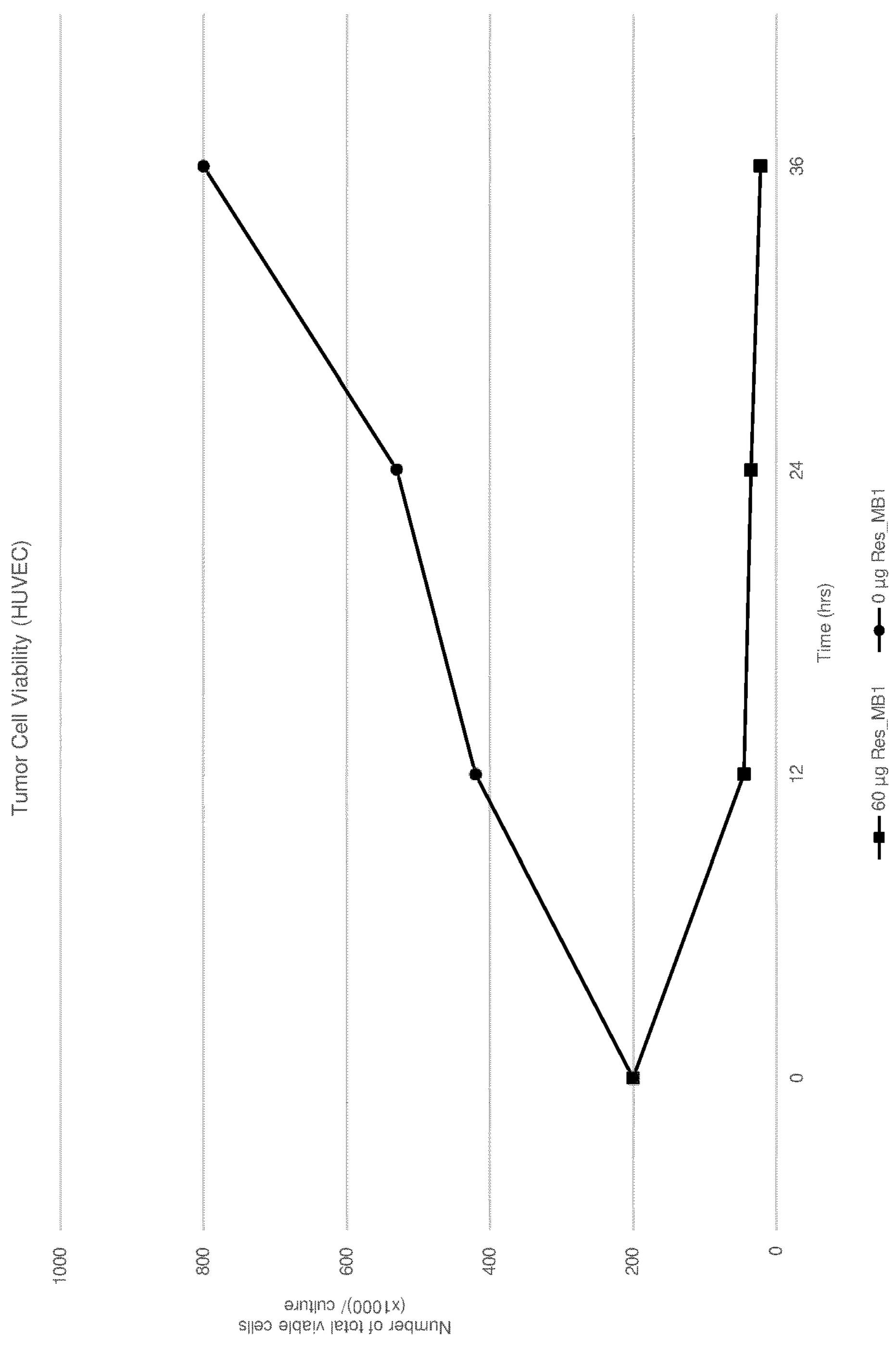
Tumor Cell Viability (HUVEC)
Number of total viable cells (x1000)/ culture
0
200
400
600
800
1000
0
12
24
36
Time (hrs)
60 µg Res_MB1
0 µg Res_MB1

Figure 8B

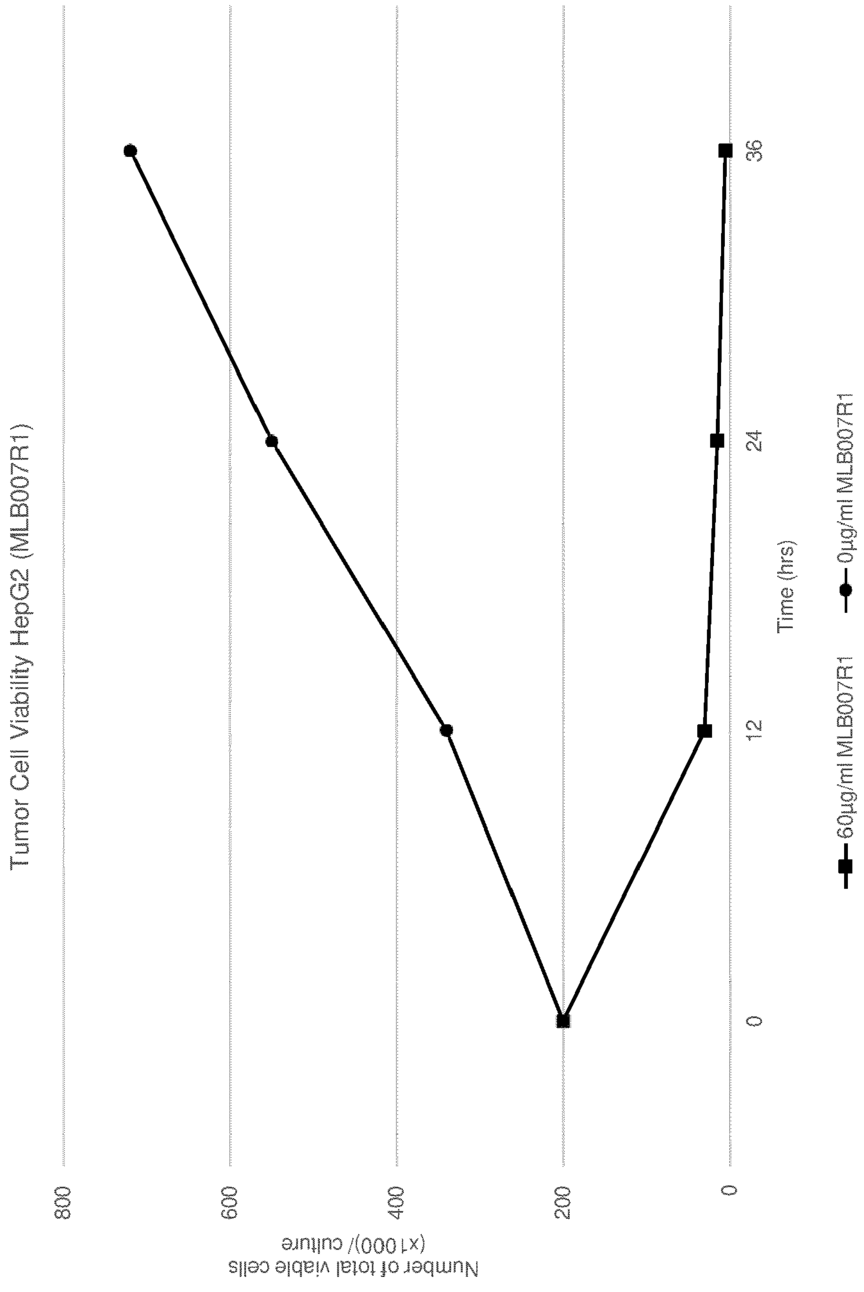
Tumor Cell Viability HepG2 (MLB007R1)
Number of total viable cells (x1000)/ culture
800
600
400
200
0
0
12
24
36
Time (hrs)
60µg/ml MLB007R1
0µg/ml MLB007R1

Figure 8C

Figure 9

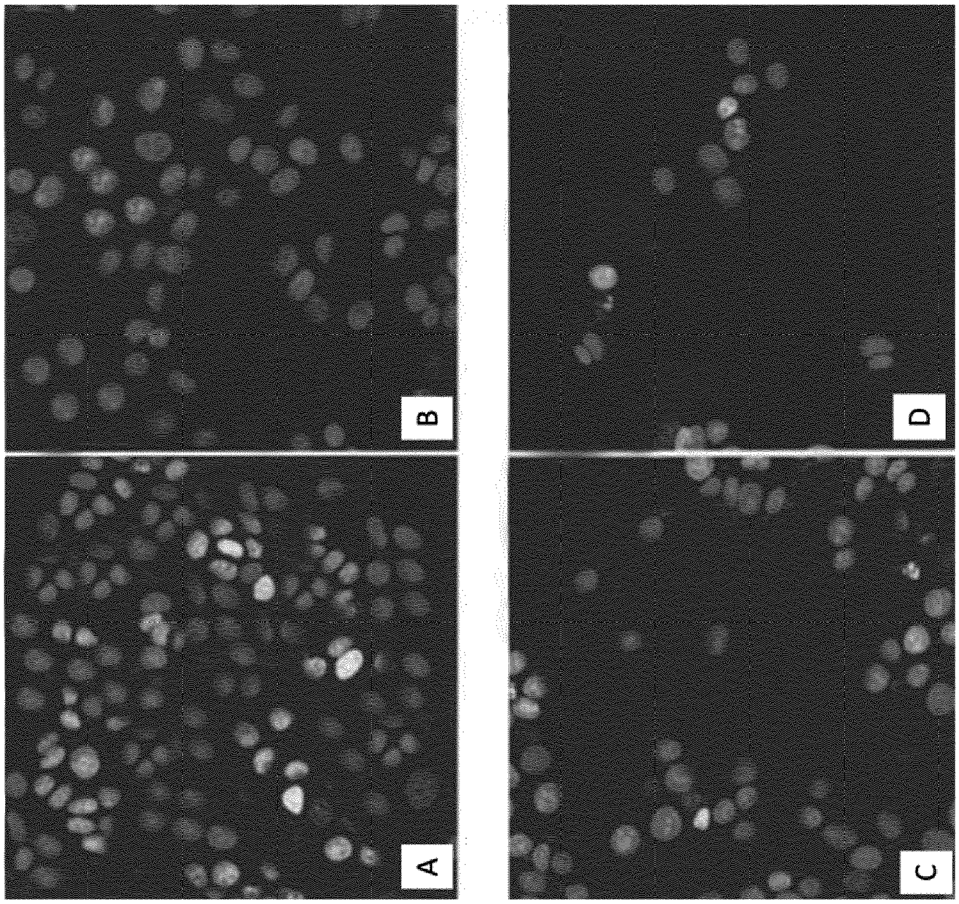

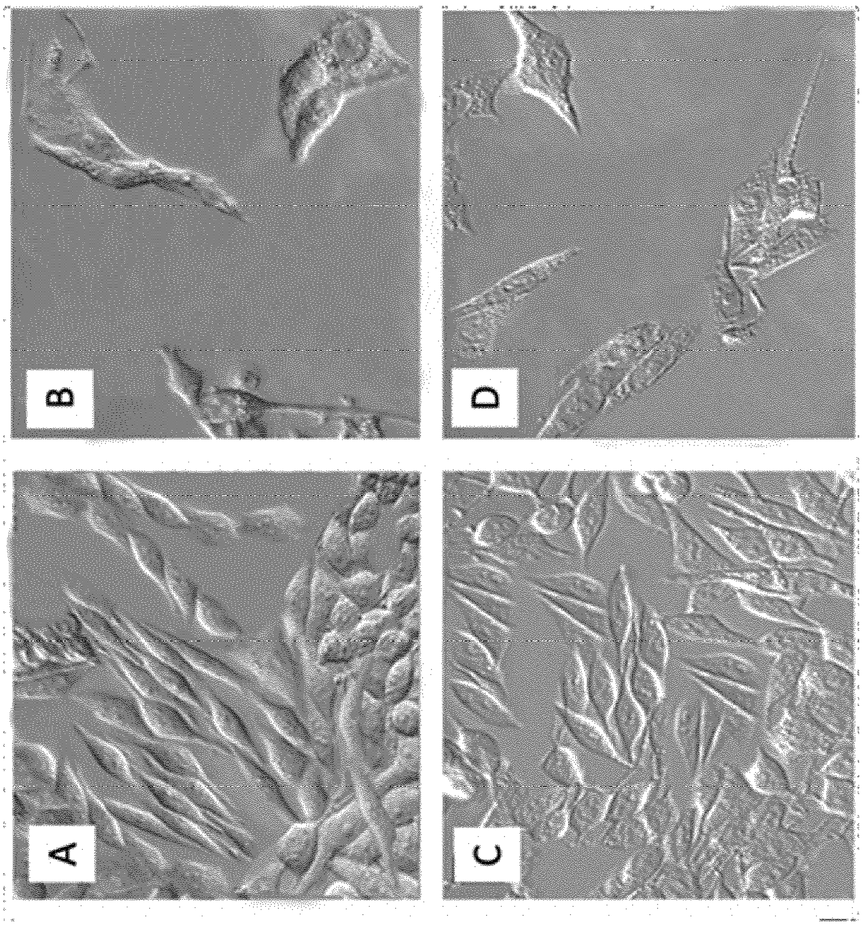


Figure 10

Europäisches Patentamt
European Patent Office
Office européen des brevets

# EUROPEAN SEARCH REPORT

Application Number
EP 23 18 2027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VILLALOBOS-AYALA KRYSTAL ET AL: "Apigenin Increases SHIP-1 Expression, Promotes Tumoricidal Macrophages and Anti-Tumor Immune Responses in Murine Pancreatic Cancer", CANCERS, vol. 12, no. 12, 4 December 2020 (2020-12-04), page 3631, XP093096844, DOI: 10.3390/cancers12123631 | 1,2,11 | INV. A61K31/045 A61K31/05 A61K31/12 A61K31/352 A61K31/404 A61K31/4412 A61K45/06 A61P35/00 A61K31/09 A61K31/167 A61K31/194 A61K31/215 A61K31/216 A61K31/235 A61K31/351 A61K31/4025 A61K31/4045 |
| Y | * Simple summary, Conclusion and sections 2.1 and 2.3 * | 5 | |
| | ----- | | |
| X | WO 2015/035365 A1 (NOHNS HOPKINS UNIVERSITY [US]) 12 March 2015 (2015-03-12) | 1,2,10, 11 | |
| Y | * page 16, line 26 - line 29 * * figures 12A, 12B; example 10 * * page 12, line 15 - line 18 * * page 13, line 10 - line 15 * * page 15, line 24 - line 30 * * page 35, line 12 - line 13 * | 5 | TECHNICAL FIELDS SEARCHED (IPC) |
| | ----- | | A61K A61P G01N |
| X | WO 2016/185026 A1 (INSERM [FR]; UNIVERSITÉ PARIS SUD [FR] ET AL.) 24 November 2016 (2016-11-24) | 1,2,11 | |
| Y | * page 36, line 20 - line 26; claims 12,13; figure 1 * | 5 | |
| | ----- | | |
| X | WO 2007/073518 A2 (NAT JEWISH MED & RES CENTER [US]; DAY BRIAN J [US] ET AL.) 28 June 2007 (2007-06-28) * example 14; tables 4,5 * | 1,2,11 | |
| | ----- | | |
| X | CN 103 705 502 B (INST BOTANY JIANGSU PROVINCE & CAS; UNIV NANJING) 24 May 2017 (2017-05-24) | 1-3 | |
| Y | * examples 2,3 * | 5 | |
| | ----- | | |
| | -/-- | | |

~~The present search report has been drawn up for all claims~~

3

| Place of search | Date of completion of the search | Examiner |
|---|---|---|
| The Hague | 19 January 2024 | Rodrigues Neibecker |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 18 2027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HANÁKOVÁ ZUZANA ET AL: "C -Geranylated Flavanones from Paulownia tomentosa Fruits as Potential Anti-inflammatory Compounds Acting via Inhibition of TNF-[alpha] Production", JOURNAL OF NATURAL PRODUCTS, vol. 78, no. 4, 24 April 2015 (2015-04-24), pages 850-863, XP055895991, US ISSN: 0163-3864, DOI: 10.1021/acs.jnatprod.5b00005 Retrieved from the Internet: URL:https://pubs.acs.org/doi/pdf/10.1021/acs.jnatprod.5b00005> | 1-3 | A61K31/405 A61K31/415 A61K31/4152 A61K31/4184 A61K31/4433 A61K31/453 A61K31/496 A61K31/505 A61K31/513 G01N33/00 |
| Y | * chart 1; figure 1; compound 12 * ----- | 5 | |
| X | KIMURA YOSHIYUKI ET AL: "Resveratrol Prevents Tumor Growth and Metastasis by Inhibiting Lymphangiogenesis and M2 Macrophage Activation and Differentiation in Tumor-associated Macrophages", NUTRITION AND CANCER, vol. 68, no. 4, 4 May 2016 (2016-05-04), pages 667-678, XP093118825, US ISSN: 0163-5581, DOI: 10.1080/01635581.2016.1158295 Retrieved from the Internet: URL:https://dx.doi.org/10.1080/01635581.2016.1158295> * Title * * abstract * * page 669, right-hand column * ----- | 1,2,11 | TECHNICAL FIELDS SEARCHED (IPC) |
| | -/-- | | |

~~The present search report has been drawn up for all claims~~

3

| Place of search | Date of completion of the search | Examiner |
|---|---|---|
| The Hague | 19 January 2024 | Rodrigues Neibecker |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 18 2027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HAN XU ET AL: "Resveratrol attenuates TNBC lung metastasis by down-regulating PD-1 expression on pulmonary T cells and converting macrophages to M1 phenotype in a murine tumor model", CELLULAR IMMUNOLOGY, ACADEMIC PRESS, SAN DIEGO, CA, US, vol. 368, 11 August 2021 (2021-08-11), XP086760896, ISSN: 0008-8749, DOI: 10.1016/J.CELLIMM.2021.104423 [retrieved on 2021-08-11] * title * * abstract * * section 3.6 * * section 2 * ----- | 1,2,11 | |
| X | KIMURA YOSHIYUKI ET AL: "Dihydroxystilbenes prevent azoxymethane/dextran sulfate sodium-induced colon cancer by inhibiting colon cytokines, a chemokine, and programmed cell death-1 in C57BL/6J mice", EUROPEAN JOURNAL OF PHARMACOLOGY, ELSEVIER SCIENCE, NL, vol. 886, 3 August 2020 (2020-08-03), XP086288704, ISSN: 0014-2999, DOI: 10.1016/J.EJPHAR.2020.173445 [retrieved on 2020-08-03] * figures 1, 8 * * page 13, right-hand column * * abstract * * page 1, right-hand column * ----- | 1,2,11 | TECHNICAL FIELDS SEARCHED (IPC) |
| | -/-- | | |

~~The present search report has been drawn up for all claims~~

3

| Place of search | Date of completion of the search | Examiner |
|---|---|---|
| The Hague | 19 January 2024 | Rodrigues Neibecker |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 18 2027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CASEY STEPHANIE C ET AL: "Cancer prevention and therapy through the modulation of the tumor microenvironment", SEMINARS IN CANCER BIOLOGY, SAUNDERS SCIENTIFIC PUBLICATIONS, PHILADELPHIA, PA, US, vol. 35, 10 April 2015 (2015-04-10), XP029303919, ISSN: 1044-579X, DOI: 10.1016/J.SEMCANCER.2015.02.007 * sections 3.2 and 3.8; figure 2 * ----- | 1,2,11 | |
| X | KIVIMÄKI KONSTA ET AL: "Pinosylvin Shifts Macrophage Polarization to Support Resolution of Inflammation", MOLECULES, vol. 26, no. 9, 8 May 2021 (2021-05-08), pages 1-14, XP093119132, CH ISSN: 1420-3049, DOI: 10.3390/molecules26092772 * abstract * ----- | 1,2 | |
| | -/-- | | TECHNICAL FIELDS SEARCHED (IPC) |

~~The present search report has been drawn up for all claims~~

3

| Place of search | Date of completion of the search | Examiner |
|---|---|---|
| The Hague | 19 January 2024 | Rodrigues Neibecker |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 18 2027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MAK KA-KIT ET AL: "Pterostilbene, a bioactive component of blueberries, suppresses the generation of breast cancer stem cells within tumor microenvironment and metastasis via modulating NF-[kappa]B/microRNA 448 circuit", MOLECULAR NUTRITION & FOOD RESEARCH, vol. 57, no. 7, 15 March 2013 (2013-03-15), pages 1123-1134, XP055805935, DE ISSN: 1613-4125, DOI: 10.1002/mnfr.201200549 Retrieved from the Internet: URL:https://api.wiley.com/onlinelibrary/tdm/v1/articles/10.1002%2Fmnfr.201200549> * abstract * * page 1132, right-hand column * * section 2.11 * ----- | 1,2,11 | |
| X | Spatafora Carmela ET AL: "Antiangiogenic Resveratrol Analogues by Mild m-CPBA Aromatic Hydroxylation of 3,5-Dimethoxystilbenes", Natural product communications, 1 February 2009 (2009-02-01), pages 239-246, XP093118981, Los Angeles, CA DOI: 10.1177/1934578X0900400215 Retrieved from the Internet: URL:https://pubmed.ncbi.nlm.nih.gov/19370931/ [retrieved on 2024-01-12] * abstract; compounds 1-12 * * scheme 1 * ----- -/-- | 1,2,7,11 | TECHNICAL FIELDS SEARCHED (IPC) |

~~The present search report has been drawn up for all claims~~

3

| Place of search | Date of completion of the search | Examiner |
|---|---|---|
| The Hague | 19 January 2024 | Rodrigues Neibecker |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 18 2027

| Category | Citation of document with indication, where appropriate, of relevant passages | Relevant to claim | CLASSIFICATION OF THE APPLICATION (IPC) |
|---|---|---|---|
| X | TAN YU-JIA ET AL: "Galloyl esters of trans-stilbenes are inhibitors of FASN with anticancer activity on non-small cell lung cancer cells", EUROPEAN JOURNAL OF MEDICINAL CHEMISTRY, ELSEVIER, AMSTERDAM, NL, vol. 182, 6 August 2019 (2019-08-06), XP085874892, ISSN: 0223-5234, DOI: 10.1016/J.EJMECH.2019.111597 [retrieved on 2019-08-06] * abstract; figure 2; table 1 * ----- | 1,2,7,11 | |
| X | Kimura Yoshiyuki ET AL: "Antitumor and Antimetastatic Activity of Synthetic Hydroxystilbenes Through Inhibition of Lymphangiogenesis and M2 Macrophage Differentiation of Tumor-associated Macrophages", Anticancer research, 31 December 2015 (2015-12-31), pages 137-148, XP093118988, Greece Retrieved from the Internet: URL:https://pubmed.ncbi.nlm.nih.gov/26722037/ [retrieved on 2024-01-12] * abstract; figure 1; table 1 * ----- | 1,2,11 | TECHNICAL FIELDS SEARCHED (IPC) |

-/--

~~The present search report has been drawn up for all claims~~

| Place of search | Date of completion of the search | Examiner |
|---|---|---|
| The Hague | 19 January 2024 | Rodrigues Neibecker |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

3

Europäisches Patentamt
European Patent Office
Office européen des brevets

# EUROPEAN SEARCH REPORT

Application Number
EP 23 18 2027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LEE JUWON ET AL: "Phytochemicals in Cancer Immune Checkpoint Inhibitor Therapy", BIOMOLECULES, vol. 11, no. 8, 27 July 2021 (2021-07-27), page 1107, XP093119150, CH ISSN: 2218-273X, DOI: 10.3390/biom11081107 * abstract * * section 4 * * table 1 * ----- | 1,2,10, 11 | |
| X | GUO KERONG ET AL: "Recent advances in combretastatin A-4 codrugs for cancer therapy", EUROPEAN JOURNAL OF MEDICINAL CHEMISTRY, ELSEVIER, AMSTERDAM, NL, vol. 241, 9 August 2022 (2022-08-09), XP087165785, ISSN: 0223-5234, DOI: 10.1016/J.EJMECH.2022.114660 [retrieved on 2022-08-09] * abstract * * figures 1,10,15 * ----- | 1,2,11 | TECHNICAL FIELDS SEARCHED (IPC) |

~~The present search report has been drawn up for all claims~~

3

| Place of search | Date of completion of the search | Examiner |
|---|---|---|
| The Hague | 19 January 2024 | Rodrigues Neibecker |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 23 18 2027

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

**3, 5, 7(completely); 1, 2, 10, 11(partially)**

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 23 18 2027

The Search Division considers that the present European patent application does not comply with the requirements of unity of invention and relates to several inventions or groups of inventions, namely:

1. claims: 3, 5(completely); 1, 2, 10, 11(partially)

   -A pharmaceutical composition comprising at least one of apigenin derivative or quercetin derivative.
   -A pharmaceutical composition comprising at least one of apigenin derivative or quercetin derivative for use in the treatment or prevention of cancer.

---

2. claims: 12-15(partially)

   -An in-vitro method for polarization or differentiation of monocytes or peripheral blood mononuclear cells (PBMCs) into macrophages with a M1-like phenotype with anti-tumor properties, comprising the step of incubation with an apigenin derivative or quercetin derivate.
   -A method of preparation of repolarized macrophages with M1-like phenotype with anti-tumor properties, comprising the step o of incubation with an apigenin derivative or quercetin derivate.

---

3. claims: 9(completely); 1, 2, 10-15(partially)

   -A pharmaceutical composition comprising an indol-3-carbinol derivative.
   -A pharmaceutical composition comprising an indol-3-carbinol derivative for use in the treatment or prevention of cancer.
   -An in-vitro method for polarization or differentiation of monocytes or peripheral blood mononuclear cells (PBMCs) into macrophages with a M1-like phenotype with anti-tumor properties, comprising the step of incubation with an indol-3-carbinol derivative.
   -A method of preparation of repolarized macrophages with M1-like phenotype with anti-tumor properties, comprising the step o of incubation with an indol-3-carbinol derivative.

---

4. claims: 8(completely); 1, 2, 10-15(partially)

   -A pharmaceutical composition comprising a lupeol derivative.
   -A pharmaceutical composition comprising a lupeol derivative for use in the treatment or prevention of cancer.
   -An in-vitro method for polarization or differentiation of monocytes or peripheral blood mononuclear cells (PBMCs) into macrophages with a M1-like phenotype with anti-tumor properties, comprising the step of incubation with a lupeol derivative.
   A method of preparation of repolarized macrophages with

Europäisches Patentamt
European Patent Office
Office européen des brevets

# LACK OF UNITY OF INVENTION SHEET B

Application Number
EP 23 18 2027

The Search Division considers that the present European patent application does not comply with the requirements of unity of invention and relates to several inventions or groups of inventions, namely:

M1-like phenotype with anti-tumor properties, comprising the step o of incubation with a lupeol derivative.

---

5. claims: 4(completely); 1, 2, 10-15(partially)

-A pharmaceutical composition comprising a piperlongumin derivative.
-A pharmaceutical composition comprising a piperlongumin derivative for use in the treatment or prevention of cancer.
-An in-vitro method for polarization or differentiation of monocytes or peripheral blood mononuclear cells (PBMCs) into macrophages with a M1-like phenotype with anti-tumor properties, comprising the step of incubation with a piperlongumin derivative.
-A method of preparation of repolarized macrophages with M1-like phenotype with anti-tumor properties, comprising the step o of incubation with a piperlongumin derivative.

---

6. claims: 6(completely); 1, 2, 10-15(partially)

-A pharmaceutical composition comprising a curcumin derivative.
-A pharmaceutical composition comprising a curcumin derivative for use in the treatment or prevention of cancer.
-An in-vitro method for polarization or differentiation of monocytes or peripheral blood mononuclear cells (PBMCs) into macrophages with a M1-like phenotype with anti-tumor properties, comprising the step of incubation with a curcumin derivative.
-A method of preparation of repolarized macrophages with M1-like phenotype with anti-tumor properties, comprising the step o of incubation with a curcumin derivative.

---

7. claims: 7(completely); 1, 2, 10, 11(partially)

-A pharmaceutical composition comprising a resveratrol derivative.
-A pharmaceutical composition comprising a resveratrol derivative for use in the treatment or prevention of cancer.

---

8. claims: 12-15(partially)

-An in-vitro method for polarization or differentiation of monocytes or peripheral blood mononuclear cells (PBMCs) into macrophages with a M1-like phenotype with anti-tumor properties, comprising the step of incubation with a resveratrol derivative.

Europäisches Patentamt
European Patent Office
Office européen des brevets

# LACK OF UNITY OF INVENTION SHEET B

Application Number
EP 23 18 2027

The Search Division considers that the present European patent application does not comply with the requirements of unity of invention and relates to several inventions or groups of inventions, namely:

**-A method of preparation of repolarized macrophages with M1-like phenotype with anti-tumor properties, comprising the step o of incubation with a resveratrol derivative.**

---

page 3 of 3

## ANNEX TO THE EUROPEAN SEARCH REPORT ON EUROPEAN PATENT APPLICATION NO.

EP 23 18 2027

This annex lists the patent family members relating to the patent documents cited in the above-mentioned European search report. The members are as contained in the European Patent Office EDP file on
The European Patent Office is in no way liable for these particulars which are merely given for the purpose of information.

19-01-2024

| Patent document cited in search report | Publication date | Patent family member(s) | Publication date |
|---|---|---|---|
| WO 2015035365 A1 | 12-03-2015 | US 2016220692 A1 | 04-08-2016 |
| | | US 2018264130 A1 | 20-09-2018 |
| | | US 2021138082 A1 | 13-05-2021 |
| | | WO 2015035365 A1 | 12-03-2015 |
| WO 2016185026 A1 | 24-11-2016 | EP 3297615 A1 | 28-03-2018 |
| | | EP 3685830 A1 | 29-07-2020 |
| | | US 2018125876 A1 | 10-05-2018 |
| | | US 2020297749 A1 | 24-09-2020 |
| | | WO 2016185026 A1 | 24-11-2016 |
| WO 2007073518 A2 | 28-06-2007 | AU 2006327105 A1 | 28-06-2007 |
| | | CA 2669503 A1 | 28-06-2007 |
| | | EP 1954681 A2 | 13-08-2008 |
| | | US 2006135585 A1 | 22-06-2006 |
| | | US 2011104305 A1 | 05-05-2011 |
| | | WO 2007073518 A2 | 28-06-2007 |
| CN 103705502 B | 24-05-2017 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017019848 A1
- US 11103507 B2


### Non-patent literature cited in the description


- **BISWAS, S. K.** ; **ALLAVENA, P.** ; **MANTOVANI, A.** Tumor-associated macrophages: functional diversity, clinical significance, and open questions. *Seminars in Immunopathology*, 2013, vol. 35 (5), 585-600
- **CASSOL, E.** ; **CASSETTA, L.** ; **RIZZI, C.** ; **ALFANO, M.** ; **POLI, G.** M1 and M2a Polarization of Human Monocyte-Derived Macrophages Inhibits HIV-1 Replication by Distinct Mechanisms.. *The Journal of Immunology*, 2009, vol. 182 (10), 6237-6246
- **COOK, J.** ; **HAGEMANN, T.** Tumour-associated macrophages and cancer. *Current Opinion in Pharmacology*, 2013, vol. 13 (4), 595-601
- **D'ARCY, MS.** ; **PIKE CVS.** ; **COUSSONS PJ.** A novel combined resveratrol/berberine phytochemotherapeutic using the HePG2 cell line as a model for the treatment of hepatocarcinoma. *Cell Biol Int.*, December 2021, vol. 45 (12), 2499-2509
- **HOLZAPFEL, B.** ; **WICKERT, L.** Die quantitative Real-Time-PCR (qRT-PCR). Methoden und Anwendungsgebiete.. *Biologie in Unserer Zeit*, 2007, vol. 37 (2), 120-126
- Praktikum der Molekulargenetik. **KÜCK**. PCR-Analytik. Springer, 2005, 221-245
- **LEE, P. Y.** ; **COSTUMBRADO, J.** ; **HSU, C. Y.** ; **KIM, Y. H.** Agarose gel electrophoresis for the separation of DNA fragments. *Journal of visualized experiments*, 2012, vol. 62, 3923
- **LEWIS, C. E.** ; **POLLARD, J. W.** Distinct Role of Macrophages in Different Tumor Microenvironments. *Cancer Research*, 2006, vol. 66 (2), 605-612
- **LI, Y.** ; **ZHU, W.** ; **LI, J.** ; **LIU, M.** ; **WEI, M.** Resveratrol suppresses the STAT3 signaling pathway and inhibits proliferation of high glucose-exposed HepG2 cells partly through SIRT1. *Oncol Rep.*, December 2013, vol. 30 (6), 2820-8
- **LOEGL, J.** ; **HIDEN, U.** ; **NUSSBAUMER, E.** ; **SCHLIEFSTEINER, C.** ; **CVITIC, S.** ; **LANG, I.** ; **DESOYE, G.** Hofbauer cells of M2a, M2b and M2c polarization may regulate feto-placental angiogenesis. *Reproduction*, 2016, vol. 152 (5), 447-455
- **MARTINEZ, F. O.** ; **GORDON, S.** The M1 and M2 paradigm of macrophage activation: time for reassessment. *F1000Prime Reports*, 2014, 6
- **QUATROMONI, JON G** ; **EVGENIY ERUSLANOV**. Tumor-Associated Macrophages: Function, Phenotype, and Link to Prognosis in Human Lung Cancer.. *American Journal of Translational, Research*, 2012, vol. 4 (4), 376-89
- **SICA, A.** ; **MANTOVANI, A.** Macrophage plasticity and polarization: in vivo veritas. *Journal of Clinical Investigation*, 2012, vol. 122 (3), 787-795
- **SICA, A.** ; **SACCANI, A.** ; **BOTTAZZI, B.** ; **POLENTARUTTI, N.** ; **VECCHI, A.** ; **DAMME, J. V.** ; **MANTOVANI, A.** Autocrine Production of IL-10 Mediates Defective IL-12 Production and NF- B Activation in Tumor-Associated Macrophages. *The Journal of Immunology*, 2000, vol. 164 (2), 762-767
- **SOLINAS, G** ; **G GERMANO** ; **A MANTOVANI** ; **P ALLAVENA**. Tumor-Associated Macrophages (TAM) as Major Players of the Cancer-Related Inflammation.. *Journal of Leukocyte Biology*, 2009, vol. 86 (5), 1065-73
- **WYNN, T. A.** ; **CHAWLA, A.** ; **POLLARD, J. W.** Macrophage biology in development, homeostasis and disease. *Nature*, 2013, vol. 496 (7446), 445-455